# EUROPEAN PATENT APPLICATION

(11) **EP 4 559 487 A2**
(43) Date of publication of application: **28.05.2025**
(21) Application number: 25168662.2
(22) Date of filing: 12.12.2019
(51) Int. Cl.: A61L 27/24

(54) **SYNTHETIC FOOD COMPOSITIONS**

(30) Priority: 12.12.2018 US 201862778751 P
(62) Divisional of application: 19896078.3
(71) Applicant: Wild Type, Inc., San Francisco, California 94107 (US)
(72) Inventor: ELFENBEIN, Arye, San Francisco, 94107 (US); KOLBECK, Justin Lee, San Francisco, 94107 (US); LARSON, Benjamin, San Francisco, 94107 (US)
(74) Representative: De Clercq & Partners

(57) **Abstract**

Systems and methods for producing food products including cultured food products. The cultured food products include sushi-grade fish meat, fish surimi, foie gras, and other food types. Various cell types are utilized to produce the food products and can include muscle, fat, and/or liver cells. The cultured food products are grown in pathogen-free culture conditions without exposure to toxins and other undesirable chemicals. The food products can be processed to provide a desired shape, texture and consistency.

## Description

### CROSS-REFERENCE

This application claims the benefit of U.S. Provisional Application No. 62/778,751, filed December 12, 2018, which is incorporated herein by reference in its entirety.

### BACKGROUND OF THE INVENTION

Traditional meat production is a resource-intensive process that generates a significant environmental footprint. Domesticated animals are raised in agricultural settings requiring substantial quantities of fresh water, feed, land, and other resources. Similarly, consumption of fish is vulnerable to a number of problems including overfishing and by-catch as well as pollution caused by fisheries.

### SUMMARY OF THE INVENTION

Disclosed herein are systems, methods, and compositions for producing synthetic or processed food products. Various ingredients for making these food products are described herein and are consistent with the present disclosure. In some instances, spinning techniques are utilized to create food products having at least one of a desired consistency, texture, composition, taste, and appearance. Examples of spinning techniques are electrospinning and solution blow spinning.

Described herein are various approaches that provide an innovative approach that allows fine control over the composition of individual ingredients as well as how the ingredients are combined to generate the finished food product. In some embodiments, solution blow spinning is used to process and/or create the food product. One advantage of the present disclosure is that the processing or generation of the food product does not require the application of heat or high voltage. Another advantage is the ability to form fibers having a small diameter range such as, for example, ranges small enough to accommodate cells. Another advantage is the relatively high speed of the rate of formation using solution blow spinning compared to alternative methods.

Disclosed herein is an edible composition for human consumption, comprising: a) a population of cells; and b) a plurality of polymeric fibers. Various aspects are consistent with the present disclosure. In some cases, the polymeric fibers comprise an edible ingredient. Sometimes, the edible ingredient comprises a texturizer, bulking agent or thickener, preservative, flavor enhancer, antimicrobial agent, pH modulator, desiccant, sweetener, curing or pickling agent, coloring agent, or any combination thereof. The edible ingredient often comprises a protein, a carbohydrate, a fat, or any combination thereof. In certain instances, the protein comprises gelatin, collagen, elastin, silk, soy derivatives, zein, gliadin, hordein, casein, wheat derivatives, gluten, whey, millet derivatives, quinoa derivatives, algae proteins, marine source proteins, pea proteins, or any combination thereof. In some cases, the carbohydrate comprises chitosan, carrageenan, starch, agar, tapioca starch, alginate, cellulose and cellulose derivatives, pullulan, dextran, glucomannan, cyclodextrin, agarose, konjac, pectin, carrageenan, alginate, xanthan, guar, locust bean gum, tara gum, gum tragacanth, gum arabic, fenugreek gum, modified starch, potato starch, pea starch, Hylon 7 corn starch, hemicellulose, altanan, or any combination thereof. The fat comprises soybean oil, sunflower oil, linseed oil, rapeseed oil, corn oil, olive oil, palm oil, palm kernel oil, butter, cheese, or any combination thereof, in various aspects. Sometimes, the polymeric fiber comprises a supplement. The supplement often comprises a vitamin, a mineral, or both. In various cases, the population of cells comprises a cultured population of cells. In certain instances, the population of cells comprises myocytes. The plurality of fibers sometimes comprises a layer of fibers incorporating the myocytes and aligned to mimic muscle fibers. The population of cells further comprises adipocytes, in some aspects. Sometimes, the plurality of fibers comprises a layer of fibers incorporating the adipocytes and configured to mimic adipose banding patterns. In some cases, the population of cells is deposited onto and within the polymeric fibers. The population of cells is integrated into the polymeric fibers in some instances. Sometimes, the population of cells comprises a non-cultured population of cells. The non-cultured population of cells is often derived or obtained from an animal, fish, or bird. In certain cases, the polymeric fibers are generated using a fiber spinning technique. In many instances, the fiber spinning technique is electrospinning or solution blow spinning. The polymeric fibers and the population of cells are arranged as alternating layers of fibers and cells, in certain cases.

Disclosed herein is a method of producing an edible composition, comprising: a) forming a plurality of polymer fibers using a solution blow spinning process, wherein said polymer fibers are formed from a composition comprising an edible ingredient dissolved in a volatile solvent; and b) depositing said plurality of blow spun polymer fibers onto a surface to form a layer of polymer fibers. Various aspects are consistent with the present disclosure. Sometimes, the edible composition comprises between about 1% and about 95% w/v protein, carbohydrate, fat, or a combination thereof. In certain instances, the volatile solvent comprises ethanol, ethyl acetate, acetic acid, acetone, dimethylformamide (DMF), or any combination thereof. The volatile solvent is acetone or ethyl acetate, in some cases. The edible ingredient often comprises a texturizer, bulking agent or thickener, preservative, flavor enhancer, antimicrobial agent, pH modulator, desiccant, sweetener, curing or pickling agent, coloring agent, or any combination thereof. Sometimes, the edible ingredient comprises a protein, a carbohydrate, a fat, or any combination thereof. The protein comprises gelatin, collagen, elastin, silk, soy derivatives, zein, gliadin, hordein, casein, wheat derivatives, gluten, whey, millet derivatives, quinoa derivatives, algae proteins, marine source proteins, pea proteins, or any combination thereof, in various aspects. In many instances, the carbohydrate comprises chitosan, carrageenan, starch, agar, tapioca starch, alginate, cellulose and cellulose derivatives, pullulan, dextran, glucomannan, cyclodextrin, agarose, konjac, pectin, carrageenan, alginate, xanthan, guar, locust bean gum, tara gum, gum tragacanth, gum arabic, fenugreek gum, modified starch, potato starch, pea starch, Hylon 7 corn starch, hemicellulose, altanan, or any combination thereof. Sometimes, the fat comprises soybean oil, sunflower oil, linseed oil, rapeseed oil, corn oil, olive oil, palm oil, palm kernel oil, butter, cheese, or any combination thereof. In certain aspects, the composition comprises a supplement. The supplement often comprises a vitamin, a mineral, or both. Sometimes, the method further comprises depositing a population of cells onto the layer of polymeric fibers. In some cases, the population of cells comprises a cultured population of cells. Oftentimes, the population of cells comprises myocytes. The layer of polymeric fibers incorporates the myocytes and comprises fibers that are aligned to mimic muscle fibers, in certain aspects. Sometimes, the population of cells further comprises adipocytes. In various cases, the layer of polymeric fibers incorporates the adipocytes and is configured to mimic adipose banding patterns. Sometimes, the population of cells comprises a non-cultured population of cells. In some cases, the non-cultured population of cells is derived or obtained from an animal, fish, or bird. The layer of polymeric fibers and the population of cells are arranged as alternating layers of fibers and cells, in some instances. The method sometimes further comprises collecting the layer of polymeric fibers on a collector. In some instances, the collector is a rotating drum. The solution blow spinning process is configured to achieve a desired polymeric fiber diameter or diameter range, in various aspects. In some cases, the polymer fibers are configured to degrade over time.

Described herein is a method of producing cultured tissue for human consumption, the method comprising: a) obtaining a population of self-renewing cells; b) culturing the population of self-renewing cells; c) inducing differentiation in the population of self-renewing cells to form cultured tissue; and d) processing the cultured tissue for human consumption. Various aspects are consistent with the present disclosure. Sometimes, the population of self-renewing cells comprises at least one cell that has been modified to undergo inducible differentiation. In certain instances, the at least one cell has been modified to incorporate at least one genetic construct comprising: a) an open reading frame (ORF) of at least one pluripotency gene; and b) an open reading frame (ORF) of a regulatory factor configured to inactivate the ORF of the at least one pluripotency gene. Sometimes, the at least one genetic construct further comprises: a) an open reading frame (ORF) of at least one differentiation gene; and b) a promoter controlling expression of the open reading frame (ORF) of the regulatory factor. In some instances, a) the regulatory factor is a transposase; and b) the open reading frame (ORF) of the at least one pluripotency gene is flanked by transposon flanking sequences recognized by the transposase such that expression of the transposase catalyzes excision of the open reading frame (ORF) of at least one pluripotency gene. Sometimes, inducing differentiation comprises exposing the at least one cell to an induction agent to induce expression of the ORF of the regulatory factor, wherein expression of transposase results in excision of the ORF of the at least one pluripotency gene. In some cases, inducing differentiation comprises exposing the at least one cell to an induction agent to induce expression of the ORF of the regulatory factor, wherein expression of transposase results in excision of the at least one differentiation gene. Oftentimes, the method further comprises inducing expression of the ORF of the transposase, wherein expression of transposase results in excision of the at least one genetic construct. In certain aspects, expression of the ORF of the transposase is controlled by a marker of cell differentiation. The marker of cell differentiation is MyoD, myosin heavy chain (MHC), alpha-actinin, Myh1, Myh4, Myh7, titin, nebulin, or myosin light polypeptide 2 (mylz2), and expression of the ORF of the transposase is controlled by a constitutively active promoter, in some aspects. Sometimes, excision of the at least one genetic construct leaves no genetic footprint of the at least one genetic construct in the at least one cell. In some instances, the at least one differentiation gene comprises an ORF of at least one hepatocyte differentiation factor selected from Hepatocyte Nuclear Factor 1 Alpha (HNF1A), Forkhead Box A2 (FOXA2), and Hepatocyte Nuclear Factor 4 Alpha (HNF4A). In various instances, the at least one differentiation gene comprises a myogenic factor selected from Myogenin (MyoG), Myogenic Differentiation 1 (MyoD), Myogenic Factor 6 (MRF4), Myogenic Factor 5 (MYF5), PAX3, PAX7, and MEF2. In some aspects, the at least one differentiation gene comprises at least one adipogenic factor selected from Fatty Acid Binding Protein 4 (FABP4), Insulin-Responsive Glucose Transporter Type 4 (GLUT4), Adiponectin, C1Q And Collagen Domain Containing (ADIPOQ), 1-Acylglycerol-3-Phosphate O-Acyltransferase 2 (AGPAT2), Perilipin 1 (PLIN1), Leptin (LEP), Lipoprotein Lipase (LPL), CEBPalpha, CEBPbeta, PPARgamma, and ZFP423. Sometimes, cells in the population of self-renewing cells are maintained in a self-renewing state using microRNAs targeting at least one differentiation gene. Inducing differentiation comprises removing microRNAs targeting at least one differentiation gene, in certain cases. Sometimes, inducing differentiation comprises exposing the population of self-renewing cells to microRNAs targeting at least one pluripotency gene. In certain instances, inducing differentiation comprises exposing the population of self-renewing cells to episomal DNA constructs targeting at least one pluripotency gene. In certain aspects, inducing differentiation comprises using exosomes to target at least one pluripotency gene in the population of self-renewing cells. In certain instances, inducing differentiation comprises using exosomes to deliver nucleic acids, small molecules, or growth factors targeting at least one pluripotency gene in the population of self-renewing cells. The at least one pluripotency gene comprises at least one of an anti-differentiation gene, an anti-proliferation gene, and a combination thereof, in some cases. In some embodiments, microRNAs are delivered into the population of self-renewing cells through lipofection, nucleofection, transfection, or transduction. In some aspects, inducing differentiation comprises incubating the population of self-renewing cells with scaffolds embedded with DNA vectors containing microRNAs, wherein cells in the population internalize the DNA vectors and subsequently express the microRNAs. Sometimes, cells in the population of self-renewing cells are maintained in a self-renewing state through exposure to synthetic RNA coding for at least one pluripotency gene. In certain cases, inducing differentiation comprises exposing the population of self-renewing cells to synthetic RNA coding for at least one differentiation gene. The population of self-renewing cells is sometimes maintained in a self-renewing state through modulation of DNA methylation. Inducing differentiation comprises modulating DNA methylation in the population of self-renewing cells, in some instances.

Described herein is a method for producing cultured fish roe for human consumption, the method comprising: a) obtaining a population of fish cells capable of self-renewal; b) inducing formation of primordial germ cells in the population of fish cells; and c) culturing the primordial germ cells to maturate into fish roe. Various aspects are consistent with the present disclosure. Sometimes, the population of fish cells comprises cells selected from embryonic stem cells or induced pluripotent stem cells. In some instances, inducing formation of primordial germ cells comprises incubating the population of fish cells in a media formulation comprising BMP, retinoic acid, EGF, or any combination thereof. In various aspects, the media formulation further comprises luteinizing hormone, DHP, or both. Sometimes, inducing formation of primordial germ cells further comprises increasing expression of VASA, deadend, DAZL, CXCR4b, Buckyball, or any combination thereof.

Described herein is a method of making a cultured food product, comprising: a) forming polymer fibers using solution blow spinning; b) seeding cultured cells onto the polymer fibers to form synthetic muscle tissue; and c) processing the cultured cells and polymer fibers to produce the cultured food product. Various aspects are consistent with the present disclosure. Sometimes, the synthetic muscle tissue comprises a first configuration comprising cultured muscle cells and nanofibers arranged in parallel alignment to simulate muscle fibers. In certain cases, the synthetic muscle tissue comprises a second configuration comprising cultured adipose cells and nanofibers arranged to simulate adipose banding patterns. In some instances, the first configuration and second configuration are combined to form the cultured food product configured to simulate a taste and texture of non-synthetic muscle tissue. The step of forming the polymer fibers in (a) occurs concurrently with the step of seeding cultured cells onto the polymer fibers in (b), in some aspects. Oftentimes, the polymer fibers are made of monomers selected from proteins, polysaccharides, or both. In various cases, the monomers comprise proteins selected from gelatin, collagen, elastin, silk, soy, zein, gliadin, hordein, amaranth, casein, wheat, whey, avenin, secalin, corn protein meal, ovomucin, ovotransferrin, ovalbumin, 8S mung bean protein, and algae and marine source proteins, or any combination thereof. In some cases, the monomers comprise polysaccharides selected from chitosan, carrageenan, starch, agar, tapioca starch, alginate, cellulose and cellulose derivatives, pullulan, dextran, glucomannan, cyclodextrin, agarose, konjac, pectin, carrageenan, alginate, xanthan, guar, locust bean gum, tara gum, gum tragacanth, gum arabic, fenugreek gum, modified starch, potato starch, pea starch, Hylon 7 corn starch, hemicellulose, altanan, or any combination thereof. Sometimes, the step of forming polymer fibers in (a) comprises dissolving monomers in a solvent to form a pre-polymer solution, ejecting the solution into a gas or air stream to generate the polymer fibers as the solvent evaporates. In some cases, the polymer fibers are configured to degrade over time.

Described herein is a method of producing a food composition having a fat phase, comprising: a) forming a plurality of polymer fibers using a solution blow spinning process, wherein said polymer fibers are formed from a composition comprising an edible ingredient dissolved in a volatile solvent; and b) dispersing said plurality of blow spun polymer fibers in a fat phase. Various aspects are consistent with the present disclosure. In some cases, the polymer fibers comprise a lipophilic material selected from prolamins and lipophilic cellulose derivatives. Sometimes, the method further comprises homogenizing a mixture of the plurality of blow spun polymer fibers and the fat phase. In certain instances, the method further comprises fragmenting the mixture into particles. The fat phase often comprises a vegetable oil, a dairy oil, a fish oil, an algae oil, or any combination thereof. The food composition comprises up to 95% by weight of an aqueous phase, in various aspects. Sometimes, the food composition comprises an emulsion comprising 1 to 99% by weight within the fat phase. In many instances, the polymer fibers make up between 1% and 50% (w/w) of the food composition.

Disclosed herein are methods of producing cultured fish meat for human consumption. Some such methods comprise: a) obtaining a population of self-renewing cells derived from fish; b) culturing the population of self-renewing cells in culture media comprising micro-scaffolds; c) inducing differentiation in the population of cells to form at least one of myocytes and adipocytes; and d) processing the population of cells into fish meat for human consumption. Various aspects incorporate at least one of the following elements. The fish meat is often sushi. In some instances, the fish meat is surimi. Sometimes, the fish meat is suitable for raw consumption. In certain cases, the fish meat is cooked. The fish meat is usually salmon meat. In certain aspects, the fish meat is sushi-grade salmon meat. Alternatively, the fish meat is sometimes tuna meat. Sometimes, the fish meat is sushi-grade tuna meat. In some cases, inducing differentiation in (c) causes the population of cells to form myocytes and adipocytes. In some cases, differentiation comprises transdifferentiation of cells into a different cell type. Oftentimes, the fish meat is composed of at least 50% high glycolytic and anaerobic muscle fibers. The population of cells is frequently derived from sea bass, tuna, mackerel, blue marlin, swordfish, yellowtail, salmon, or trout. Processing in (d) usually comprises combining the population of cells with a second population of cells composed of myocytes or adipocytes. In various aspects, the population of cells is isolated as embryonic stem cells. Sometimes, the population of cells has been modified to induce pluripotency. The population of cells is isolated as multipotent adult stem cells, in certain embodiments. Culturing typically comprises growing and expanding the population of cells in cell culture. Inducing differentiation often comprises exposing the population of cells to culture conditions that stimulate differentiation. Sometimes, inducing differentiation comprises exposing the population of cells to at least one growth factor that stimulates differentiation. In certain instances, culturing comprises growing the population of cells on a two dimensional surface. Alternatively, culturing comprises growing the population of cells on a three-dimensional scaffold. Culturing often comprises growing the population of cells on micro-scaffolds within a bioreactor, wherein the micro-scaffolds enable cell adhesion. Sometimes, the population of cells forms non-textured tissue after differentiation. In various aspects, culturing comprises growing the population of cells in a media formulation comprising at least one nutritional supplement. The at least one nutritional supplement usually comprises an omega-3 fatty acid. The at least one nutritional supplement comprises a polyunsaturated fatty acid, sometimes. In certain instances, the at least one nutritional supplement comprises a monounsaturated fatty acid. Sometimes, the population of cells is cultured using a non-serum media formulation. In many cases, the population of cells is cultured using a mushroom-based media formulation.

In some aspects, disclosed herein are methods for producing cultured fish tissue, the methods comprising: a) culturing a population of fish pre-adipocytes and a population of fish satellite cells; b) inducing differentiation in the population of fish pre-adipocytes to form adipocytes; c) inducing differentiation in the population of fish satellite cells to produce myocytes; d) co-culturing the adipocytes and myocytes; and e) processing the adipocytes and myocytes into fish tissue for human consumption. Various aspects include at least one of the following elements. Sometimes, the fish tissue comprises fast twitch muscle fibers. Oftentimes, the fish tissue is salmon tissue. In certain cases, the fish tissue is tuna tissue. The fish tissue is occasionally trout tissue. In many instances, the fish tissue is surimi. The fish tissue is often sushi. The fish tissue is made for raw human consumption, in some cases. The fish tissue is sometimes cooked for human consumption. In various aspects, the adipocytes and myocytes are co-cultured in a media formulation comprising at least one nutritional supplement. The at least one nutritional supplement usually comprises an omega-3 fatty acid. Sometimes, the at least one nutritional supplement comprises a polyunsaturated fatty acid. The at least one nutritional supplement comprises a monounsaturated fatty acid, on occasion. Oftentimes, a non-serum media formulation is used for cell culturing. In certain cases, a mushroom-based media formulation is used for cell culturing.

In some aspects, disclosed herein are methods for producing cultured fish tissue, the methods comprising: a) culturing a population of fish pre-adipocytes and a population of fish satellite cells, said populations adapted for suspension culture; b) inducing differentiation in the population of fish pre-adipocytes to form adipocytes; c) inducing differentiation in the population of fish satellite cells to form myocytes; d) co-culturing the adipocytes and myocytes; and e) processing the adipocytes and myocytes into fish tissue for human consumption.

In some aspects, disclosed herein are edible compositions comprising fish tissue produced from co-cultured myocytes and adipocytes.

In some aspects, disclosed herein are edible compositions comprising fish tissue produced from pre-adipocytes and satellite cells.

In some aspects, disclosed herein are methods of producing cultured fish meat for human consumption, the methods comprising: a) obtaining a population of pre-adipocytes and a population of satellite cells; b) adapting the population of pre-adipocytes and the population of satellite cells to suspension culture; c) inducing differentiation in the population of pre-adipocytes and the population of satellite cells; d) co-culturing the populations in suspension culture; and e) processing the populations into fish meat for human consumption. In some cases, differentiation comprises transdifferentiation of cells into a different cell type. Various aspects include at least one of the following elements. Sometimes, the fish meat is sushi. The fish meat is often surimi. In certain instances, the fish meat is suitable for raw consumption. Oftentimes, the fish meat is cooked. In various aspects, the fish meat is salmon meat. The fish meat is sushi-grade salmon meat, in certain cases. The fish meat is often tuna meat. Sometimes, the fish meat is sushi-grade tuna meat. Occasionally, the fish meat is trout meat. In many instances, the fish meat is composed of at least 50% high glycolytic and anaerobic muscle fibers. The population of pre-adipocytes is usually derived from sea bass, tuna, mackerel, blue marlin, swordfish, yellowtail, salmon, or trout. The population of satellite cells is often derived from sea bass, tuna, mackerel, blue marlin, swordfish, yellowtail, salmon, or trout. Co-culturing typically comprises growing and expanding the populations in cell culture. In certain cases, inducing differentiation comprises exposing the population of pre-adipocytes to at least one growth factor that stimulates differentiation into adipocytes. Sometimes, inducing differentiation comprises exposing the population of satellite cells to at least one growth factor that stimulates differentiation into myocytes. Culturing often comprises growing the population of cells within a bioreactor. In many cases, the myocytes and adipocytes form non-textured tissue after differentiation. The myocytes and adipocytes are often cultured in a media formulation comprising at least one nutritional supplement. The at least one nutritional supplement usually comprises an omega-3 fatty acid. In many instances, the at least one nutritional supplement comprises a polyunsaturated fatty acid. Occasionally, the at least one nutritional supplement comprises a monounsaturated fatty acid. Oftentimes, a non-serum media formulation is used for cell culturing. In certain cases, a mushroom-based media formulation is used for cell culturing.

In some aspects, disclosed herein are fish products suitable for human consumption comprising fish surimi produced from cultured myocytes and adipocytes.

In some aspects, disclosed herein are synthetic food products suitable for human consumption comprising fish meat derived from cultured satellite cells and pre-adipocytes.

In some aspects, disclosed herein are fish products suitable for human consumption comprising fish meat produced from myocytes and adipocytes grown in suspension culture.

In some aspects, disclosed herein are methods of producing cultured fish meat for human consumption, the methods comprising: a) obtaining a population fish pre-adipocytes capable of growing in suspension culture; b) obtaining a population of fish satellite cells capable of growing in suspension culture; c) inducing differentiation in the population of fish pre-adipocytes and the population of fish satellite cells to form adipocytes and myocytes; d) co-culturing the adipocytes and myocytes in suspension culture comprising at least one nutritional supplement; and e) processing the population of cells into fish meat for human consumption. Various aspects include at least one of the following elements. Sometimes, the fish meat is sushi. Oftentimes, the fish meat is surimi. In many cases, the fish meat is suitable for raw consumption. The fish meat is occasionally cooked. The fish meat is sometimes salmon meat. In certain instances, the fish meat is sushi-grade salmon meat. Sometimes, the fish meat is tuna meat. The fish meat is often sushi-grade tuna meat. The fish meat is composed of at least 50% high glycolytic and anaerobic muscle fibers, in various aspects. Typically, the population of cells is derived from sea bass, tuna, mackerel, blue marlin, swordfish, yellowtail, salmon, or trout. Oftentimes, inducing differentiation in (c) comprises exposing the population of pre-adipocytes and the population of satellite cells to culture conditions that stimulate differentiation. inducing differentiation in (c) usually comprises exposing the population of pre-adipocytes to at least one growth factor that stimulates differentiation. In certain instances, inducing differentiation in (c) comprises exposing the population of satellite cells to at least one growth factor that stimulate differentiation. The adipocytes and myocytes usually form non-textured tissue. Sometimes, the at least one nutritional supplement comprises an omega-3 fatty acid. In many cases, the at least one nutritional supplement comprises a polyunsaturated fatty acid. Sometimes, the at least one nutritional supplement comprises a monounsaturated fatty acid. Oftentimes, a non-serum media formulation is used for cell culturing. In certain cases, a mushroom-based media formulation is used for cell culturing. In some cases, the population of cells are transdifferentiated into at least one cell type. In some cases, the population of cells are transdifferentiated into at least one of hepatocytes, myocytes, and adipocytes.

In some aspects, disclosed herein are methods of producing cultured tissue for human consumption, the method comprising: a) obtaining a population of self-renewing cells; b) culturing the population of self-renewing cells; c) inducing differentiation in the population of self-renewing cells to form cultured tissue; and d) processing the cultured tissue for human consumption. Various aspects include at least one of the following elements. In some cases, obtaining the population of self-renewing cells comprises transitioning a population of cells from 2-dimensional adherent culture into 3-dimensional culture in a bioreactor. Sometimes, the population of self-renewing cells comprises differentiated cells that have become immortalized. Inducing differentiation in the population of self-renewing cells often comprises inducing transdifferentiation of cells in the population into myocytes, adipocytes, or a combination thereof. In some cases, the population of cells are transdifferentiated into at least one cell type. In some cases, the population of cells are transdifferentiated into at least one of hepatocytes, myocytes, and adipocytes. In certain instances, culturing comprises seeding the population of self-renewing cells on 3-dimensional micro-scaffolds. In some cases, the 3-dimensional micro-scaffolds promote cell growth, adhesion, differentiation, or a combination thereof. The 3-dimensional micro-scaffolds are conjugated to at least one factor promoting cell growth, adhesion, differentiation, or a combination thereof, in various aspects. Sometimes, the micro-scaffolds comprise at least one of hydrogel, chitosan, polyethylene terephthalate, collagen, elastin, heparan sulfate, chondroitin sulfate, keratan sulfate, hyaluronic acid, laminin, fibronectin, cellulose, hemicellulose, pectin, lignin, alginate, glucomannan, polycaprolactone (PCL), textured vegetable protein (TVP), textured soy protein (TSP), and acrylates. In certain instances, the population of self-renewing cells comprises at least one cell that has been modified to undergo inducible differentiation. In some instances, the at least one cell has been modified to incorporate: a) a first genetic construct comprising an open reading frame (ORF) of at least one pluripotency gene; and b) a second genetic construct comprising an open reading frame (ORF) of a regulatory factor configured to inactivate the at least one pluripotency gene. Often, the population of self-renewing cells comprises at least one cell that undergoes at least 50 cell divisions during culturing. In some cases, the regulatory factor is a recombinase, and the open reading frame (ORF) of at least one pluripotency gene is flanked by recombination sequences recognized by the recombinase such that expression of the recombinase catalyzes excision of the open reading frame (ORF) of at least one pluripotency gene. The second genetic construct comprises an ORF of at least one hepatocyte differentiation factor selected from Hepatocyte Nuclear Factor 1 Alpha (HNF1A), Forkhead Box A2 (FOXA2), and Hepatocyte Nuclear Factor 4 Alpha (HNF4A), in some instances. In various aspects, the second genetic construct comprises at least one myogenic factor selected from Myogenin (MyoG), Myogenic Differentiation 1 (MyoD), Myogenic Factor 6 (MRF4), and Myogenic Factor 5 (MYF5). The second genetic construct often comprises at least one adipogenic factor selected from Fatty Acid Binding Protein 4 (FABP4), Insulin-Responsive Glucose Transporter Type 4 (GLUT4), Adiponectin, C1Q And Collagen Domain Containing (ADIPOQ), 1-Acylglycerol-3-Phosphate O-Acyltransferase 2 (AGPAT2), Perilipin 1 (PLIN1), Leptin (LEP), and Lipoprotein Lipase (LPL). Sometimes, the second genetic construct further comprises: a) an open reading frame (ORF) of at least one differentiation gene; and b) an inducible promoter controlling expression of: i) the open reading frame (ORF) of the at least one differentiation gene; and ii) the open reading frame (ORF) of the regulatory factor. In certain cases, inducing differentiation comprises exposing the at least one cell to an induction agent to induce expression of the ORF of at least one cell lineage gene and the ORF of the regulatory factor. The method typically comprises removing the induction agent after the population of self-renewing cells has been treated with the induction agent and before being processed for human consumption in step d). Inducing differentiation comprises generating myotubes within the population of self-renewing cells, in certain cases. In many instances, inducing differentiation further comprises generating adipocytes within the population of self-renewing cells. Sometimes, the population of self-renewing cells comprises multipotent cells that are induced to differentiate into myocytes and adipocytes during step c). The multipotent cells often comprise a first subpopulation of myosatellite cells and a second subpopulation of pre-adipocytes. In some instances, inducing differentiation comprises generating hepatocytes within the population of self-renewing cells. The population of self-renewing cells is derived from an avian species selected from duck, goose, chicken, and turkey, in some aspects. The method often further comprises inducing steatosis within at least one of the hepatocytes. In certain instances, the population of self-renewing cells comprises at least one cell modified to express at least one gene for enhancing steatosis upon treatment with an induction agent. Sometimes, the at least one cell is stably transformed using a construct comprising an open reading frame (ORF) encoding ATF4, ZFP423, LPIN1, PPAR, APOC3, APOE, ORL1, PEMT, MTTP, SREBP, STAT3, or KLF6. 433. In various aspects, inducing steatosis comprises incubating the hepatocytes in a culture medium comprising at least nutritional supplement. The at least one nutritional supplement often comprises a polyunsaturated fatty acid, a monounsaturated fatty acid, or a combination thereof. In some cases, the at least one nutritional supplement comprises palmitic acid, oleic acid, docosahexaenoic acid, stearic acid, linoleic acid, linolenic acid, arachidonic acid, eicosapentaenoic acid, or a combination thereof. The cultured tissue comprises octopus, squid, or cuttlefish muscle cells, in certain aspects. Sometimes, the cultured tissue comprises fish muscle tissue. The population of self-renewing cells can be derived from sea bass, tuna, mackerel, blue marlin, swordfish, yellowtail, salmon, or trout. In many cases, the fish muscle tissue is combined with separately cultured fish fat tissue during step d). In some instances, the population of cells is cultured using a non-serum media formulation. The non-serum media formulation comprises a mushroom extract or soybean hydrolysate, in some embodiments.

Disclosed herein are methods of producing cultured meat for human consumption. Some such methods comprise: a) obtaining a population of self-renewing cells, said cells capable of growing in suspension culture; b) culturing the population of self-renewing cells in suspension; c) inducing differentiation in the population of cells to form at least one of myocytes and adipocytes; and d) processing the population of cells into meat for human consumption. Various aspects incorporate at least one of the following elements. Sometimes, the meat is fish meat. The fish meat is usually sushi. In some embodiments, the fish meat is surimi. Oftentimes, the fish meat is suitable for raw consumption. In certain cases, the fish meat is cooked. In certain cases, the fish meat is salmon meat. In certain aspects, the fish meat is sushi-grade salmon meat. In some cases, the fish meat is tuna meat. Sometimes, the fish meat is sushi-grade tuna meat. Oftentimes, inducing differentiation in c) causes the population of cells to form myocytes and adipocytes. The fish meat is usually composed of at least 50% high glycolytic and anaerobic muscle fibers. The population of cells is usually derived from sea bass, tuna, mackerel, blue marlin, swordfish, yellowtail, salmon, or trout. Processing in d) often comprises combining the population of cells with a second population of cells composed of myocytes or adipocytes. In certain cases, the population of cells is isolated as embryonic stem cells. Sometimes, the population of cells has been modified to induce pluripotency. Certain populations of cells are isolated as multipotent adult stem cells. Sometimes, the population of self-renewing cells are immortalized cells. Culturing typically comprises growing and expanding the population of cells in cell culture. Oftentimes, inducing differentiation comprises exposing the population of cells to culture conditions that stimulate differentiation. In some cases, differentiation comprises transdifferentiation of cells into a different cell type. Inducing differentiation comprises exposing the population of cells to at least one growth factor that stimulates differentiation, in some instances. Culturing sometimes comprises growing the population of cells on a two dimensional surface. Certain populations of cells form non-textured tissue after differentiation. In certain aspects, culturing comprises growing the population of cells in a media formulation comprising at least one nutritional supplement. Sometimes, the at least one nutritional supplement comprises an omega-3 fatty acid. In other cases, the at least one nutritional supplement comprises a polyunsaturated fatty acid. Occasionally, the at least one nutritional supplement comprises a monounsaturated fatty acid. Sometimes, the population of cells is cultured using a non-serum media formulation. In many cases, the population of cells is cultured using a mushroom-based media formulation.

Disclosed herein are methods of producing cultured cells having high lipid accumulation for human consumption. Some methods comprise: a) culturing a population of cells; b) inducing differentiation within the population of cells; c) inducing high lipid accumulation within the population of cells; and d) processing the population of cells for human consumption. Various aspects incorporate at least one of the following elements. In certain cases, the population of cells following differentiation comprises hepatocytes. Oftentimes, processing comprises using the population of cells as an ingredient in foie gras. The population of cells is derived from duck or goose, in some cases. The population of cells is sometimes derived from at least one of poultry and livestock. In certain instances, inducing high lipid accumulation comprises inducing steatosis. In some embodiments, high lipid accumulation is characterized by excess accumulation of cytoplasmic lipid droplets. Inducing high lipid accumulation often comprises exposing the population of cells to an exogenous compound that modulates at least one lipid metabolic pathway. In certain cases, inducing high lipid accumulation comprises exposing the population of cells to at least one of a toxin and a high lipid concentration. Sometimes, inducing high lipid accumulation comprises modulating at least one lipid metabolic pathway to enhance lipid retention within the population of cells. In some instances, inducing high lipid accumulation comprises altering at least one gene in within the population of cells to modulate lipid metabolism. Oftentimes, the population of cells following differentiation comprises liver, heart, kidney, stomach, intestine, lung, diaphragm, esophagus, thymus, pancreas, or tongue cells. Processing the population of cells for human consumption comprises blending the population of cells with cells having low lipid accumulation, in various aspects. The population of cells is sometimes isolated as embryonic stem cells. In certain cases, the population of cells has been modified to induce pluripotency. In some instances, the population of cells is isolated as multipotent adult stem cells. Culturing typically comprises growing and expanding the population of cells in cell culture. In certain aspects, inducing differentiation comprises exposing the population of cells to culture conditions that stimulate differentiation. In some embodiments, inducing differentiation comprises exposing the population of cells to at least one growth factor that stimulates differentiation. In some cases, differentiation comprises transdifferentiation of cells into a different cell type. Culturing sometimes comprises growing the population of cells on a two dimensional surface. In many cases, culturing comprises growing the population of cells on a three-dimensional scaffold. In certain instances, culturing comprises growing the population of cells on micro-scaffolds within a bioreactor, wherein the micro-scaffolds enable cell adhesion. In some embodiments, the population of cells does not require an adherence substrate for survival and proliferation. Sometimes, the population of cells is adapted to suspension culture. The population of cells often forms non-textured tissue after differentiation. The population of cells forms non-muscle tissue after differentiation, in some aspects. In various cases, culturing comprises growing the population of cells in a media formulation comprising at least one nutritional supplement. In some aspects, the at least one nutritional supplement comprises an omega-3 fatty acid. The at least one nutritional supplement frequently comprises at least one of a polyunsaturated fatty acid. Sometimes, the population of cells is cultured using a non-serum media formulation. In many cases, the population of cells is cultured using a mushroom-based media formulation.

Disclosed herein are methods of producing cultured non-textured tissue having high lipid content. Some such methods comprise: a) obtaining a population of differentiated cells capable of self-renewal; b) culturing the population of differentiated cells; c) manipulating at least one lipid metabolic pathway to induce steatosis in the population of differentiated cells such that the cells accumulate high lipid content; and d) processing the population of differentiated cells into non-textured tissue. Various aspects incorporate at least one of the following elements. In some cases, obtaining the population of differentiated cells capable of self-renewal comprises transforming differentiated cells into immortalized cells. Oftentimes, obtaining the population of differentiated cells capable of self-renewal comprises culturing differentiated cells until spontaneous mutations give rise to immortalized cells. In some cases, differentiation comprises transdifferentiation of cells into a different cell type. Sometimes, the population of differentiated cells comprises fibroblasts. In some instances, the population of differentiated cells are transdifferentiated into myocytes, adipocytes, or a combination thereof. The population of differentiated cells are derived from fish such as salmon or trout, in some aspects. The population of differentiated cells comprises hepatocytes, in certain instances. In many cases, processing comprises using the population of differentiated cells as an ingredient in foie gras. In certain embodiments, the population of differentiated cells is derived from duck or goose. The population of differentiated cells is oftentimes derived from at least one of poultry and livestock. Typically, steatosis is characterized by excess accumulation of cytoplasmic lipid droplets. In certain embodiments, manipulating the at least one lipid metabolic pathway comprises exposing the population of cells to an exogenous compound. Manipulating the at least one lipid metabolic pathway comprises exposing the population of differentiated cells to at least one of a toxin and a high lipid concentration, in some aspects. Alternatively, or in combination, manipulating the at least one lipid metabolic pathway comprises altering at least one gene in within the population of cells to modulate lipid metabolism. In many cases, the population of differentiated cells comprises liver, heart, kidney, stomach, intestine, lung, diaphragm, esophagus, thymus, pancreas, or tongue cells. Sometimes, processing the population of differentiated cells comprises blending the population of cells with cells having low lipid accumulation. In many aspects, culturing comprises growing and expanding the population of cells in cell culture. Culturing sometimes comprises growing the population of cells on a two dimensional surface. In certain aspects, culturing comprises growing the population of cells on a three-dimensional scaffold. In some cases, culturing comprises growing the population of cells on micro-scaffolds within a bioreactor, wherein the micro-scaffolds enable cell adhesion. The population of cells does not require an adherence substrate for survival and proliferation, in certain instances. Sometimes, the population of cells is adapted to suspension culture. Oftentimes, the population of differentiated cells forms non-textured tissue. In some cases, the population of cells forms non-muscle tissue. Culturing comprises growing the population of cells in a media formulation comprising at least one nutritional supplement, in many aspects. In certain instances, the at least one nutritional supplement comprises an omega-3 fatty acid. Sometimes, the at least one nutritional supplement comprises a polyunsaturated fatty acid. Sometimes, the at least one nutritional supplement comprises a monounsaturated fatty acid. Sometimes, the population of cells is cultured using a non-serum media formulation. In many cases, the population of cells is cultured using a mushroom-based media formulation.

Disclosed herein are methods of producing cultured non-muscle tissue for human consumption. Some such methods comprise: a) obtaining a population of self-renewing cells; b) culturing the population of self-renewing cells; c) inducing differentiation in the population of cells to form non-muscle tissue; and d) processing the cultured non-muscle tissue for human consumption. Various aspects incorporate at least one of the following elements. In some cases, differentiation comprises transdifferentiation of cells into a different cell type.

In some aspects, disclosed herein are methods for producing cultured tissue for human consumption, the methods comprising: obtaining a population of self-renewing cells; adapting the population of self-renewing cells to suspension culture; culturing the population of self-renewing cells; inducing differentiation in the population of cells to form cultured tissue; and processing the cultured tissue for human consumption. In some cases, differentiation comprises transdifferentiation of cells into a different cell type.

Disclosed herein are methods of producing cultured non-textured muscle tissue for human consumption. Some such methods comprise: a) obtaining a population of self-renewing cells; b) culturing the population of self-renewing cells; c) inducing differentiation in the population of cells to form non-textured muscle tissue; and d) processing the cultured non-textured muscle tissue for human consumption. Various aspects incorporate at least one of the following elements. In some cases, the non-textured muscle tissue is octopus, squid, or cuttlefish muscle. Sometimes, the non-textured muscle tissue is fish muscle tissue. In certain instances, the fish muscle tissue comprises high glycolytic and anaerobic muscle fibers. The high glycolytic and anaerobic muscle fibers often make up at least 80% of the fish muscle tissue. In some cases, the population of cells is derived from sea bass, tuna, mackerel, blue marlin, swordfish, yellowtail, salmon, or trout. In various aspects, the non-textured muscle tissue is combined with fat tissue. Sometimes, the muscle tissue and fat tissue are combined to create a surimi product. In certain occasions, the fish muscle and fat tissue is sushi-grade. The population of cells is isolated as embryonic stem cells, in some embodiments. In certain aspects, the population of cells has been modified to induce pluripotency. In many cases, the population of cells is isolated as multipotent adult stem cells. Culturing comprises growing and expanding the population of cells in cell culture, in various instances. Oftentimes, inducing differentiation comprises exposing the population of cells to culture conditions that stimulate differentiation. In some cases, differentiation comprises transdifferentiation of cells into a different cell type. Sometimes, inducing differentiation comprises exposing the population of cells to at least one growth factor that stimulates differentiation. In various aspects, culturing comprises growing the population of cells on a two dimensional surface. Oftentimes, culturing comprises growing the population of cells on a three-dimensional scaffold. In certain instances, culturing comprises growing the population of cells on micro-scaffolds within a bioreactor, wherein the micro-scaffolds enable cell adhesion. In some scenarios, the population of cells does not require an adherence substrate for survival and proliferation. Sometimes, the population of cells is adapted to suspension culture. In certain embodiments, the population of cells forms non-textured tissue after differentiation. The population of cells sometimes forms non-muscle tissue after differentiation. In certain cases, culturing comprises growing the population of cells in a media formulation comprising at least one nutritional supplement. In some instances, the at least one nutritional supplement comprises an omega-3 fatty acid. Typically, the at least one nutritional supplement comprises a polyunsaturated fatty acid. Sometimes, the at least one nutritional supplement comprises a monounsaturated fatty acid. Sometimes, the population of cells is cultured using a non-serum media formulation. In many cases, the population of cells is cultured using a mushroom-based media formulation.

Disclosed herein are methods of preparing foie gras, comprising cultured avian liver tissue. Some such methods comprise: a) obtaining a population of avian derived cells capable of self-renewal; b) differentiating the population of avian derived cells into hepatocytes; and c) inducing steatosis in the hepatocytes to generate cultured avian liver tissue having high lipid content; and d) preparing the cultured avian liver tissue as foie gras. Various aspects incorporate at least one of the following elements. Sometimes, the cells are duck cells. In certain aspects, the cells are goose cells. In some cases, differentiation comprises transdifferentiation of cells into a different cell type.

Disclosed herein are culinary foie gras compositions comprising tissue cultured hepatocytes having high lipid content and processed for human consumption. Various aspects incorporate at least one of the following elements. In some cases, the composition has been processed into a plurality of slices. In certain instances, each slice weighs no more than about 5 ounces. Each slice is oftentimes individually packaged. Sometimes, the foie gras composition weighs at least about 1.5 pounds, is round and firm, and has no blemish. In various aspects, the foie gras composition has a package label indicating an A grade rating. In certain embodiments, the foie gras composition weighs between about 0.75 to about 1.5 pounds. In some instances, the foie gras composition has a package label indicating a B grade rating. The foie gras composition weighs less than about 1 pound and has no more than three blemishes, in some cases. In some cases, the foie gras composition has a package label indicating a C grade rating. In certain embodiments, the tissue cultured hepatocytes are steatotic. In many instances, the tissue cultured hepatocytes are characterized by excess accumulation of cytoplasmic lipid droplets. The high lipid content is obtained by exposure to an exogenous compound that modulates at least one lipid metabolic pathway, in certain aspects. The high lipid content is often obtained by exposure to at least one of a toxin and a high lipid concentration. Sometimes, the high lipid content is obtained by modulation of at least one lipid metabolic pathway to enhance lipid retention within the population of cells. In certain instances, the high lipid content is obtained by alteration of at least one gene in the tissue cultured hepatocytes. Oftentimes, the foie gras composition further comprises cells having low lipid accumulation. In various aspects, the tissue cultured hepatocytes are differentiated from isolated embryonic stem cells. The tissue cultured hepatocytes are differentiated from induced pluripotent stem cells, in certain cases. The tissue cultured hepatocytes are differentiated from isolated multipotent adult stem cells, in certain instances. In some instances, the tissue cultured hepatocytes are generated by differentiation in a population of cells capable of self-renewal. Sometimes, differentiation comprises exposing the population of cells to culture conditions that stimulate differentiation. In some cases, differentiation comprises transdifferentiation of cells into a different cell type. In various cases, differentiation comprises exposing the population of cells to at least one growth factor that stimulates differentiation. Oftentimes, the tissue cultured hepatocytes are grown on a two dimensional surface. The tissue cultured hepatocytes are grown on a three-dimensional scaffold, in some instances. In various aspects, the tissue cultured hepatocytes are grown on micro-scaffolds within a bioreactor, wherein the micro-scaffolds enable cell adhesion. In certain embodiments, the tissue cultured hepatocytes do not require an adherence substrate for survival and proliferation. Sometimes, the tissue cultured hepatocytes are adapted to suspension culture. Sometimes, the tissue cultured hepatocytes form non-textured tissue. In various instances, the tissue cultured hepatocytes form non-muscle tissue. The tissue cultured hepatocytes are cultured in a media formulation comprising at least one nutritional supplement, in many cases. Sometimes, the at least one nutritional supplement comprises an omega-3 fatty acid. In certain embodiments, the at least one nutritional supplement comprises a polyunsaturated fatty acid. Sometimes, the at least one nutritional supplement comprises a monounsaturated fatty acid. Sometimes, the tissue cultured hepatocytes are cultured using a non-serum media formulation. In many cases, the tissue cultured hepatocytes are cultured using a mushroom-based media formulation.

Disclosed herein are compositions comprising cultured organ cells processed into a non-textured non-muscle food product for human ingestion. Various aspects incorporate at least one of the following elements. In some cases, the cultured organ cells comprise hepatocytes. In certain aspects, the cultured organ cells comprise avian cells. Oftentimes, the food product is processed into a plurality of slices. Sometimes, each slice weighs no more than about 5 ounces. Each slice is usually individually packaged. In many aspects, the food product is foie gras. The foie gras usually weighs at least about 1.5 pounds, is round and firm, and has no blemish. In certain instances, the foie gras has a package label indicating an A grade rating. In various aspects, the foie gras weighs between about 0.75 to about 1.5 pounds. The foie gras has a package label indicating a B grade rating, in certain embodiments. Sometimes, the foie gras weighs less than about 1 pound and has no more than three blemishes. In various instances, the foie gras has a package label indicating a C grade rating. Oftentimes, the tissue cultured hepatocytes are steatotic. In certain cases, the foie gras is characterized by high lipid content. The high lipid content is obtained by exposure to an exogenous compound that modulates at least one lipid metabolic pathway, in some aspects. The high lipid content is often obtained by exposure to at least one of a toxin and a high lipid concentration. In certain cases, the high lipid content is obtained by modulation of at least one lipid metabolic pathway to enhance lipid retention within the population of cells. Sometimes, the high lipid content is obtained by alteration of at least one gene in the tissue cultured hepatocytes. In some aspects, the foie gras composition further comprises cells having low lipid accumulation. In certain instances, the cultured organ cells are grown on a two dimensional surface. Oftentimes, the cultured organ cells are grown on a three-dimensional scaffold. The cultured organ cells are grown on micro-scaffolds within a bioreactor, wherein the micro-scaffolds enable cell adhesion, in various embodiments. The cultured organ cells sometimes do not require an adherence substrate for survival and proliferation. Sometimes, the cultured organ cells are adapted to suspension culture. In various aspects, the cultured organ cells form non-textured tissue. Sometimes, the cultured organ cells form non-muscle tissue. In various cases, the cultured organ cells are cultured in a media formulation comprising at least one nutritional supplement. Sometimes, the at least one nutritional supplement comprises an omega-3 fatty acid. The at least one nutritional supplement comprises a polyunsaturated fatty acid, in many instances. Sometimes, the at least one nutritional supplement comprises a monounsaturated fatty acid. Sometimes, the cultured organ cells are cultured using a non-serum media formulation. In many cases, the cultured organ cells are cultured using a mushroom-based media formulation.

Disclosed herein are edible foie gras compositions comprising cultured steatotic avian liver cells and seasoning. In some cases, the seasoning includes at least one of salt, pepper, and sugar.

Disclosed herein are foie gras compositions comprising cultured liver cells having high lipid content and liver cells having low lipid content. Various aspects incorporate at least one of the following elements. In some cases, the cultured liver cells having high lipid content and the liver cells having low lipid content are blended together. In certain instances, the foie gras composition is suitable as an ingredient for preparing one of a mousse, a parfait, and a pâté. Typically, the liver cells having low lipid content are cultured cells. In some embodiments, the liver cells having low lipid content are un-cultured cells.

Disclosed herein are edible compositions comprising avian liver cells grown in cell culture and processed for human consumption.

Disclosed herein are packaged foie gras compositions comprising cultured liver cells and packaging having a label indicating the foie gras composition was not produced by forced feeding.

Disclosed herein are packaged foie gras compositions comprising cultured liver cells processed into foie gras and packaging having a label indicating the foie gras was produced in a pathogen-free environment. In certain cases, the label indicates the composition was produced without exposure to avian bird flu virus.

Disclosed herein are packaged edible compositions comprising cultured cells processed into a food product and packaging having a label indicating the composition was produced without exposure to a toxin. In certain cases, the toxin is one of an insecticide, herbicide, and fungicide.

Disclosed herein are methods of producing cultured cells for human consumption without using antibiotics. Some such methods comprise: a) culturing a population of cells without using antibiotics; b) inducing differentiation within the population of cells; c) inducing high lipid accumulation within the population of cells; and d) processing the population of cells for human consumption. In some cases, differentiation comprises transdifferentiation of cells into a different cell type.

Disclosed herein are methods of producing cultured cells for human consumption without exposure to pathogens. Some such methods comprise: a) culturing a population of cells in a pathogen-free culture environment; b) inducing differentiation within the population of cells; c) inducing high lipid accumulation within the population of cells; and d) processing the population of cells for human consumption. In some cases, differentiation comprises transdifferentiation of cells into a different cell type.

Disclosed herein are methods of producing cultured cells for human consumption without exposure to toxins. Some such methods comprise: a) culturing a population of cells in a toxin-free culture environment; b) inducing differentiation within the population of cells; c) inducing high lipid accumulation within the population of cells; and d) processing the population of cells for human consumption. In some cases, differentiation comprises transdifferentiation of cells into a different cell type.

Disclosed herein are methods of producing cultured non-textured tissue having high lipid content and no vasculature. Some such methods comprise: a) culturing a population of cells; b) inducing differentiation in the population of cells; c) manipulating lipid metabolic pathways to induce steatosis in the population of cells such that the cells accumulate high lipid content; and d) processing the population of cells into non-textured tissue having no vasculature. In some cases, differentiation comprises transdifferentiation of cells into a different cell type.

Disclosed herein are methods of producing cultured tissue having increased nutritional content for human consumption. Some such methods comprise: a) culturing a population of cells in a culture medium having at least one nutritional supplement; b) manipulating lipid metabolic pathways to induce steatosis in the population of differentiated cells such that the cells accumulate high lipid content; and c) processing the population of differentiated cells into non-textured tissue having no vasculature for human consumption. Various aspects incorporate at least one of the following elements. In some cases, the at least one nutritional supplement comprises an omega-3 fatty acid. Oftentimes, the at least one nutritional supplement comprises a polyunsaturated fatty acid. Sometimes, the at least one nutritional supplement comprises a monounsaturated fatty acid.

Disclosed herein are methods of producing cultured organ tissue for human consumption. Some such methods comprise: a) culturing a population of cells capable of self-renewal; b) inducing differentiation in the population of cells to generate organ tissue; and c) processing the organ tissue for human consumption. Various aspects incorporate at least one of the following elements. In some cases, the organ tissue is liver, heart, kidney, stomach, intestine, lung, diaphragm, esophagus, thymus, pancreas, or tongue tissue. In some cases, differentiation comprises transdifferentiation of cells into a different cell type. In various embodiments, the organ tissue is liver tissue. Sometimes, processing comprises blending the organ tissue with additional cellular tissues. The additional cellular tissues comprise non-steatotic liver cells, in many instances.

Disclosed herein are methods of producing cultured fish tissue having enhanced nutritional content for human consumption. Some such methods comprise: a) culturing a population of fish myocytes in a culture media having at least one nutritional supplement; b) expanding the population of myocytes; and c) processing the population of myocytes into fish tissue for human consumption. Various aspects incorporate at least one of the following elements. In some cases, the fish tissue comprises fast twitch muscle fibers. In certain embodiments, the method further comprises combining the population of myocytes with a population of adipocytes. The fish myocytes are often salmon myocytes. The fish myocytes are sometimes tuna myocytes. In some cases, the fish myocytes are trout myocytes.

Disclosed herein are edible compositions comprising fish tissue produced from cultured myocytes and adipocytes according to any of the methods described herein.

Disclosed herein are bioreactor systems for producing cultured tissues suitable for human consumption comprising: a) a reactor chamber comprising a plurality of micro-scaffolds that provide adhesion surfaces for cellular attachment; b) a population of self-renewing cells cultivated within bioreactor; c) a first source providing at least one maintenance media comprising components for maintaining the population of self-renewing cells without spontaneous differentiation; and d) a second source providing at least one differentiation media comprising components for differentiating the population of self-renewing cells into a specific lineage; wherein the reactor chamber receives maintenance media from the first source to cultivate the population of cells and receives differentiation media from the second source to differentiate the population of cells, wherein the population of cells generated in a single batch comprises cultured tissues suitable for human consumption and having a dry weight of at least 1 kg. Various aspects incorporate at least one of the following elements. In some cases, the system further comprises at least one sensor for monitoring the reactor chamber. In certain embodiments, the at least one sensor is a biosensor, a chemosensor, or an optical sensor. Oftentimes, the at least one sensor is configured to monitor at least one of pH, temperature, oxygen, carbon dioxide, glucose, lactate, ammonia, hypoxanthine, amino acid(s), dopamine, and lipid(s). Sometimes, the system further comprises at least one additional reactor chamber. The single batch often has a dry weight of at least 5 kg. Sometimes, the bioreactor system further comprises a plurality of micro-scaffolds. Alternatively, the bioreactor system further comprises at least one 3D scaffold. The bioreactor system frequently comprises a third source providing at least one steatotic media comprising components for inducing steatosis or lipid accumulation in the population of cells. In various cases, the population of cells is cultured in media comprising at least one nutritional supplement. Sometimes, the population of cells is cultured using a non-serum media formulation. In many cases, the population of cells is cultured using a mushroom-based media formulation.

Disclosed herein, in some aspects, are cultured food products for human consumption, comprising the cultured tissue produced according to the methods of any one of the foregoing methods. Sometimes, the cultured food product comprises packaging having a label indicating the cultured tissue was produced in a pathogen-free environment, a toxin-free environment, without force-feeding an animal, or any combination thereof. In certain instances, the cultured tissue is processed into a plurality of slices and packaged to form the cultured food product.

Disclosed herein is an edible composition for human consumption, comprising: a) a population of cells; and b) a plurality of polymeric fibers or scaffolds. In some cases, the polymeric fibers or scaffolds comprise at least one edible ingredient. In some cases, the edible ingredient comprises a texturizer, bulking agent or thickener, preservative, flavor enhancer, antimicrobial agent, pH modulator, desiccant, sweetener, curing or pickling agent, coloring agent, or any combination thereof. In some cases, the edible ingredient comprises a protein, a carbohydrate, a fat, or any combination thereof. In some cases, the protein comprises collagen, elastin, silk, soy derivatives, zein, gliadin, hordein, casein, wheat derivatives, gluten, whey, yeast extracts, kafirin, millet derivatives, quinoa derivatives, bean derivatives, textured vegetable proteins, egg white proteins, sea vegetable derivatives, algae proteins, transglutaminase, glutamic acid derivatives, gelatin, fish source proteins, pea proteins, or any hydrolysates thereof, any isolates thereof, or any combination thereof. In some cases, the carbohydrate comprises chitosan, carrageenan, agar. tapioca starch, alginate, cellulose and cellulose derivatives, pullulan, dextran, glucomannan, cyclodextrin, agarose, konjac, pectin, sea vegetable derivatives, alginate, xanthan, guar gum, locust bean gum, tara gum, gum tragacanth, gum arabic, fenugreek gum, modified starch, potato starch, pea starch, Hylon 7 corn starch, hemicellulose, altanan, or any combination thereof. In some cases, the fat comprises soybean oil, sunflower oil, linseed oil, rapeseed oil, corn oil, algal oil, lecithin, olive oil, palm oil, palm kernel oil, butter, cheese, or any combination thereof. In some cases, the polymeric fiber or scaffold comprises a supplement. In some cases, the supplement comprises a vitamin, a mineral, or both. In some cases, the population of cells comprises a cultured population of cells. In some cases, the population of cells comprises myocytes. In some cases, the plurality of fibers comprises a layer of fibers incorporating the myocytes and aligned to mimic muscle fibers. In some cases, the population of cells further comprises adipocytes. In some cases, the plurality of fibers comprises a layer of fibers incorporating the adipocytes and configured to mimic adipose banding patterns. In some cases, the population of cells is deposited onto and within the polymeric fibers. In some cases, the population of cells is integrated into the polymeric fibers. In some cases, the population of cells comprises a non-cultured population of cells. In some cases, the non-cultured population of cells is derived or obtained from an animal, fish, or bird. In some cases, the polymeric fibers or scaffolds are generated using a fiber spinning technique. In some cases, the fiber spinning technique is electrospinning or solution blow spinning. In some cases, the polymeric fibers or scaffolds are generated using unidirectional freeze-drying, electro-spinning, solution blow spinning, wet spinning, casting, micromolding, 3D printing or extrusion, or any combination thereof. In some cases, the composition comprises polymeric fibers generated using a fiber spinning technique and scaffolds generated using unidirectional freeze-drying, electro-spinning, solution blow spinning, wet spinning, casting, micromolding, 3D printing or extrusion, or any combination thereof. In some cases, the polymeric fibers or scaffolds and the population of cells are arranged as alternating layers of fibers and cells.

Disclosed herein is a method of producing an edible composition, comprising: a) forming a plurality of polymer fibers using a solution blow spinning process, wherein said polymer fibers are formed from a composition comprising an edible ingredient dissolved in a solvent; and b) depositing said plurality of blow spun polymer fibers onto a surface to form a layer of polymer fibers. In some cases, said composition comprises between about 1% and about 95% w/v protein, carbohydrate, fat, other polymer or a combination thereof. In some cases, the solvent comprises a non-volatile solvent. In some cases, the non-volatile solvent comprises water, salt water, an oil, or any combination thereof. In some cases, the salt water comprises at least 1%, at least 2%, at least 5%, at least 10%, at least 20%, at least 30%, at least 40%, at least 50%, at least 60%, at least 70%, at least 80%, or at least 90% salt by weight. In some cases, the oil comprises linear, branched, saturated, or unsaturated fatty acids or fatty esters, or any combination or derivative thereof. In some cases, the oil comprises a C3-C50 fatty acid or derivative thereof. In some cases, the oil is wheat germ oil, corn oil, sunflower oil, karite butter, castor oil, sweet almond oil, macadamia oil, apricot oil, soybean oil, rapeseed oil, cottonseed oil, alfalfa oil, poppy oil, pumpkin oil, sesame seed oil, marrow oil, avocado oil, hazelnut oil, grape seed oil, blackcurrant seed oil, evening primrose oil, millet oil, barley oil, quinoa oil, olive oil, rye oil, safflower oil, candlenut oil, passion flower oil or musk rose oil; or caprylic/capric acid triglycerides, synthetic oils, or any combination thereof. In some cases, the solvent comprises a volatile solvent. In some cases, the volatile solvent comprises ethanol, ethyl acetate, methanol, acetic acid, formic acid, phenol, acetone, dimethylformamide (DMF), or any combination thereof. In some cases, the volatile solvent is acetone or ethyl acetate. In some cases, the surface is a stationary or rotating surface. In some cases, the edible ingredient comprises a texturizer, bulking agent or thickener, preservative, flavor enhancer, antimicrobial agent, pH modulator, desiccant, sweetener, curing or pickling agent, coloring agent, or any combination thereof. In some cases, the edible ingredient comprises a protein, a carbohydrate, a fat, or any combination thereof. In some cases, the protein comprises collagen, elastin, silk, soy derivatives, gelatin, zein, gliadin, hordein, casein, wheat derivatives, gluten, whey, yeast extract, kafirin millet derivatives, quinoa derivatives, bean derivatives, textured vegetable proteins, egg white proteins, sea vegetable derivatives, algae proteins, transglutaminase, glutamic acid derivatives, fish source proteins, pea proteins, or any combination, isolate, or derivative thereof. In some cases, the carbohydrate comprises chitosan, carrageenan, agar, tapioca starch, alginate, cellulose and cellulose derivatives, pullulan, dextran, glucomannan, cyclodextrin, agarose, konjac, pectin, sea vegetable derivatives, alginate, xanthan, guar gum, locust bean gum, tara gum, gum tragacanth, gum arabic, fenugreek gum, modified starch, potato starch, pea starch, Hylon 7 corn starch, hemicellulose, altanan, or any combination thereof. In some cases, the fat comprises soybean oil, sunflower oil, linseed oil, rapeseed oil, corn oil, algal oil, lecithin, olive oil, palm oil, palm kernel oil, butter, cheese, or any combination thereof. In some cases, the other polymer comprises a block copolymer, block copolymer intermediate, modified poly(ethylene glycol), modified ethylene glycol monomer, polyester, poly(caprolactone), polylactic acid, poly(lactic-co-glycolic acid), polyethylene oxide, polyethylene glycol, poly(hydroxybutyrate), poly(ester urethane), polyvinyl alcohol, poloxamer, or any combination thereof. In some cases, the composition comprises a supplement. In some cases, the supplement comprises a vitamin, a mineral, or both. In some cases, the method comprises depositing a population of cells onto the layer of polymeric fibers. In some cases, the population of cells comprises a cultured population of cells. In some cases, the population of cells comprises myocytes. In some cases, the layer of polymeric fibers incorporates the myocytes and comprises fibers that are aligned to mimic muscle fibers. In some cases, the population of cells further comprises adipocytes. In some cases, the layer of polymeric fibers incorporates the adipocytes and is configured to mimic adipose banding patterns. In some cases, the population of cells comprises a non-cultured population of cells. In some cases, the non-cultured population of cells is derived or obtained from an animal, fish, or bird. In some cases, the layer of polymeric fibers and the population of cells are arranged as alternating layers of fibers and cells. In some cases, the method further comprises collecting the layer of polymeric fibers on a collector. In some cases, the collector is a rotating drum or stationary surface. In some cases, the solution blow spinning process is configured to achieve a desired polymeric fiber diameter or diameter range.

Disclosed herein is a method of producing cultured tissue for human consumption, the method comprising: a) obtaining a population of self-renewing cells; b) culturing the population of self-renewing cells; c) inducing differentiation in the population of self-renewing cells to form cultured tissue; and d) processing the cultured tissue for human consumption. In some cases, the population of self-renewing cells comprises at least one cell that has been modified to undergo inducible differentiation. In some cases, the at least one cell has been modified to incorporate at least one genetic construct comprising: a) an open reading frame (ORF) of at least one pluripotency gene; and b) an open reading frame (ORF) of a regulatory factor configured to inactivate the ORF of the at least one pluripotency gene. In some cases, the at least one genetic construct further comprises: a) an open reading frame (ORF) of at least one differentiation gene; and b) a promoter controlling expression of the open reading frame (ORF) of the regulatory factor. In some cases: a) the regulatory factor is a transposase; and b) the open reading frame (ORF) of the at least one pluripotency gene is flanked by transposon flanking sequences recognized by the transposase such that expression of the transposase catalyzes excision of the open reading frame (ORF) of at least one pluripotency gene. In some cases, inducing differentiation comprises exposing the at least one cell to an induction agent to induce expression of the ORF of the regulatory factor, wherein expression of transposase results in excision of the ORF of the at least one pluripotency gene. In some cases, inducing differentiation comprises exposing the at least one cell to an induction agent to induce expression of the ORF of the regulatory factor, wherein expression of transposase results in excision of the at least one differentiation gene. In some cases, the method further comprises inducing expression of the ORF of the transposase, wherein expression of transposase results in excision of the at least one genetic construct. In some cases, expression of the ORF of the transposase is controlled by a marker of cell differentiation. In some cases, the marker of cell differentiation is MyoD, myosin heavy chain (MHC), alpha-actinin, Myh1, Myh4, Myh7, titin, nebulin, or myosin light polypeptide 2 (mylz2), and expression of the ORF of the transposase is controlled by a constitutively active promoter. In some cases, excision of the at least one genetic construct leaves no genetic footprint of the at least one genetic construct in the at least one cell. In some cases, the at least one differentiation gene comprises an ORF of at least one hepatocyte differentiation factor selected from Hepatocyte Nuclear Factor 1 Alpha (HNF1A), Forkhead Box A2 (FOXA2), and Hepatocyte Nuclear Factor 4 Alpha (HNF4A). In some cases, the at least one differentiation gene comprises a myogenic factor selected from Myogenin (MyoG), Myogenic Differentiation 1 (MyoD), Myogenic Factor 6 (MRF4), Myogenic Factor 5 (MYF5), PAX3, PAX7, and MEF2. In some cases, the at least one differentiation gene comprises at least one adipogenic factor selected from Fatty Acid Binding Protein 4 (FABP4), Insulin-Responsive Glucose Transporter Type 4 (GLUT4), Adiponectin, C1Q And Collagen Domain Containing (ADIPOQ), 1-Acylglycerol-3-Phosphate O-Acyltransferase 2 (AGPAT2), Perilipin 1 (PLIN1), Leptin (LEP), Lipoprotein Lipase (LPL), CEBPalpha, CEBPbeta, PPARgamma, and ZFP423. In some cases, cells in the population of self-renewing cells are maintained in a self-renewing state using microRNAs targeting at least one differentiation gene. In some cases, inducing differentiation comprises removing microRNAs targeting at least one differentiation gene. In some cases, inducing differentiation comprises exposing the population of self-renewing cells to microRNAs targeting at least one pluripotency gene. In some cases, inducing differentiation comprises exposing the population of self-renewing cells to episomal DNA constructs targeting at least one pluripotency gene. In some cases, inducing differentiation comprises using exosomes to target at least one pluripotency gene in the population of self-renewing cells. In some cases, inducing differentiation comprises using exosomes to deliver nucleic acids, small molecules, or growth factors targeting at least one pluripotency gene in the population of self-renewing cells. In some cases, the at least one pluripotency gene comprises at least one an anti-differentiation gene. In some cases, microRNAs are delivered into the population of self-renewing cells through lipofection, nucleofection, transfection, or transduction. In some cases, inducing differentiation comprises incubating the population of self-renewing cells with scaffolds embedded with DNA vectors containing microRNAs, wherein cells in the population internalize the DNA vectors and subsequently express the microRNAs. In some cases, cells in the population of self-renewing cells are maintained in a self-renewing state through exposure to modified RNA coding for at least one pluripotency gene. In some cases, inducing differentiation comprises exposing the population of self-renewing cells to synthetic RNA coding for at least one differentiation gene. In some cases, the population of self-renewing cells is maintained in a self-renewing state through modulation of DNA methylation. In some cases, inducing differentiation comprises modulating DNA methylation in the population of self-renewing cells.

Disclosed herein is a method of making a cultured food product, comprising: a) forming polymer fibers using solution blow spinning; b) seeding cultured cells onto the polymer fibers to form synthetic muscle tissue; and c) processing the cultured cells and polymer fibers to produce the cultured food product. In some cases, the synthetic muscle tissue comprises a first configuration comprising cultured muscle cells and nanofibers arranged in parallel alignment to simulate muscle fibers. In some cases, the synthetic muscle tissue comprises a second configuration comprising cultured adipose cells and nanofibers arranged to simulate adipose banding patterns. In some cases, the first configuration and second configuration are combined to form the cultured food product configured to simulate a taste and texture of non-synthetic muscle tissue. In some cases, the step of forming the polymer fibers in (a) occurs concurrently with the step of seeding cultured cells onto the polymer fibers in (b). In some cases, the polymer fibers are made of monomers selected from proteins, polysaccharides, or both. In some cases, the monomers comprise proteins selected from comprises collagen, elastin, silk, soy derivatives, zein, gliadin, hordein, casein, wheat derivatives, gluten, whey, yeast extracts, kafirin, millet derivatives, quinoa derivatives, bean derivatives, textured vegetable proteins, egg white proteins, sea vegetable derivatives, algae proteins, transglutaminase, glutamic acid derivatives, gelatin, fish source proteins, and pea proteins, or any hydrolysates thereof, any isolates thereof, or any combination thereof. In some cases, the monomers comprise polysaccharides selected from chitosan, carrageenan, starch, agar. tapioca starch, alginate, cellulose and cellulose derivatives, pullulan, dextran, glucomannan, cyclodextrin, agarose, konjac, pectin, sea vegetable derivatives, alginate, xanthan, guar gum, locust bean gum, tara gum, gum tragacanth, gum arabic, fenugreek gum, modified starch, potato starch, pea starch, Hylon 7 corn starch, hemicellulose, and altanan, or any combination thereof. In some cases, the step of forming polymer fibers in (a) comprises dissolving monomers in a solvent to form a pre-polymer solution, ejecting the solution into a gas or air stream to generate the polymer fibers as the solvent evaporates.

Disclosed herein is a method of producing a food composition having a fat phase, comprising: a) forming a plurality of polymer fibers using a solution blow spinning process, wherein said polymer fibers are formed from a composition comprising an edible ingredient dissolved in a solvent; and b) dispersing said plurality of blow spun polymer fibers in a fat phase. In some cases, the polymer fibers comprise a lipophilic material selected from prolamins and lipophilic cellulose derivatives. In some cases, the method further comprises homogenizing a mixture of the plurality of blow spun polymer fibers and the fat phase. In some cases, the method further comprises fragmenting the mixture into particles. In some cases, the fat phase comprises a vegetable oil, a dairy oil, a fish oil, an algae oil, or any combination thereof. In some cases, the food composition comprises up to 95% by weight of an aqueous phase. In some cases, the food composition comprises an emulsion comprising 1 to 99% by weight within the fat phase. In some cases, the polymer fibers make up between 1% and 50% (w/w) of the food composition.

Disclosed herein is an edible food composition for human consumption, comprising: a scaffold comprising a plurality of polymeric fibers, wherein the edible composition has a textural characteristic comparable to a corresponding conventional food composition. In some cases, the polymeric fibers comprise at least one edible ingredient. In some cases, the edible ingredient comprises a texturizer, bulking agent or thickener, preservative, flavor enhancer, antimicrobial agent, pH modulator, desiccant, sweetener, curing or pickling agent, coloring agent, or any combination thereof. In some cases, the edible ingredient comprises a protein, a carbohydrate, a fat, or any combination thereof. In some cases, the protein comprises collagen, elastin, silk, soy derivatives, zein, gliadin, hordein, casein, wheat derivatives, gluten, whey, yeast extracts, kafirin, millet derivatives, quinoa derivatives, bean derivatives, textured vegetable proteins, egg white proteins, sea vegetable derivatives, algae proteins, transglutaminase, glutamic acid derivatives, gelatin, fish source proteins, pea proteins, or any hydrolysates thereof, any isolates thereof, or any combination thereof. In some cases, the carbohydrate comprises chitosan, carrageenan, agar. tapioca starch, alginate, cellulose and cellulose derivatives, pullulan, dextran, glucomannan, cyclodextrin, agarose, konjac, pectin, sea vegetable derivatives, alginate, xanthan, guar gum, locust bean gum, tara gum, gum tragacanth, gum arabic, fenugreek gum, modified starch, potato starch, pea starch, Hylon 7 corn starch, hemicellulose, altanan, or any combination thereof. In some cases, the fat comprises soybean oil, sunflower oil, linseed oil, rapeseed oil, corn oil, algal oil, lecithin, olive oil, palm oil, palm kernel oil, butter, cheese, or any combination thereof. In some cases, the polymeric fiber comprises a supplement. In some cases, the supplement comprises a vitamin, a mineral, or both. In some cases, the polymeric fibers are generated using a fiber spinning technique. In some cases, the fiber spinning technique is electrospinning or solution blow spinning. In some cases, the polymeric fibers are generated using unidirectional freeze-drying, electro-spinning, solution blow spinning, wet spinning, casting, micromolding, 3D printing or extrusion, or any combination thereof. In some cases, the composition comprises polymeric fibers generated using a fiber spinning technique and scaffolds generated using unidirectional freeze-drying, electro-spinning, solution blow spinning, wet spinning, casting, micromolding, 3D printing or extrusion, or any combination thereof.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

A better understanding of the features and advantages of the present invention will be obtained by reference to the following detailed description that sets forth illustrative embodiments, in which the principles of the invention are utilized, and the accompanying drawings of which:
**FIG. 1** shows a flow chart for a process of producing cultured cells having lipid accumulation for human consumption.
**FIG. 2** shows a flow chart for a process of producing cultured meat comprising myocytes and adipocytes for human consumption.
**FIG. 3** shows an overview of an exemplary process for culturing meat.
**FIG. 4A** shows a diagram illustrating methods of generating steatotic hepatocytes for producing cultured foie gras. **FIG. 4B** shows a diagram illustrating methods of producing cultured fish tissue for consumption.
**FIG. 5A** shows isolated trout myosatellite cells. **FIG. 5B** shows expression of genetic markers in the isolated trout myosatellite cells. **FIG. 5C** shows mature myotubes formed by differentiating the myosatellite cells. **FIG. 5D** shows a sheet of myotubes following differentiation from the myosatellite cells.
**FIG. 6A** shows a co-culture of salmon myosatellite cells (arrowheads) and salmon pre-adipocytes (arrows). The pre-adipocytes can be differentiated into adipocytes, and the myosatellite cells differentiated into myocytes (arrowhead) as shown in **FIG. 6B****.**
**FIG. 7A** shows salmon fibroblasts induced to form spheroids for propagation in a bioreactor. **FIG. 7B** shows confirmation of the viability of these spheroids upon returning them to 2-D culture conditions and observing that the fibroblasts migrated circumferentially to form colonies.
**FIG. 8** shows a successful cell culture of bass myosatellite cells.
**FIG. 9** shows a spheroid formed from duck hepatocytes growing in a hanging drop and a spinner flask into which the spheroid can be transferred for 3-dimensional suspension culture.
**FIG. 10A** shows duck hepatocytes growing in culture following successful differentiation. **FIG. 10B** confirmed successful hepatocyte differentiation by measuring markers of hepatocyte differentiation.
**FIG. 11A** shows self-renewing duck cells generated by culturing primary fibroblasts and harvesting colonies of dividing cells. **FIG. 11B** shows trout self-renewing cells generated by culturing primary fibroblasts and harvesting colonies of dividing cells.
**FIG. 12** shows an exemplary embodiment of a genetic construct that can be introduced into a cell to provide inducible differentiation into a hepatocyte.
**FIG. 13** shows an exemplary embodiment of a construct that can be introduced into a cell to allow inducible expression of one or more genes that predispose the cell to steatosis.
**FIG. 14** shows an exemplary embodiment of a DNA construct system that can be introduced into a cell to allow a proliferation/differentiation switch from a pluripotent phenotype into a differentiated phenotype.
**FIG. 15** shows an exemplary construct that can be introduced into a cell to provide an inducible "off-switch".
**FIG. 16A** shows successful induction of steatosis in duck hepatocytes upon incubation with linoleic acid. **FIG. 16B** shows a dose response curve correlating the percentage of steatotic hepatocytes with the concentration of linoleic acid.
**FIG. 17** shows the hepatocyte population size when cultured in the media having progressively decreasing concentrations of fetal bovine serum (FBS) in the presence of soybean hydrolysate.
**FIG. 18** shows duck fibroblasts that have also been successfully grown in 10% shiitake mushroom extract after successive reduction of fetal bovine serum from the cell culture media.
**FIG. 19A** shows duck fibroblasts grown in serum-free media without additional supplementation; **FIG. 19B** shows a control culture grown in DMEM supplemented with 10% fetal bovine serum.
**FIG. 20** shows a diagram of a bioreactor system for culturing cells for human consumption.
**FIG. 21** shows another diagram of a bioreactor system being used as part of a meat production process.
**FIG. 22A** shows an embryoid body generated using the hanging drop method (left panel) and an exemplary bioreactor that the embryoid bodies are transitioned to for growing in 3-D culture. **FIG. 22B** shows another exemplary bioreactor (left panel) and cells from the spheroids that are propagated in the 3-D culture (right panel).
**FIG. 23A** shows trout myotubes that have successfully differentiated from myosatellite cells attached to glucomannan microscaffolds. **FIG. 23B** shows a negative control of undifferentiated myosatellite cells from the same preparation grown in identical cell culture conditions.
**FIG. 24A** shows duck fibroblasts (arrowheads) successfully grown on glucomannan microscaffolds (arrows). **FIG. 24B** shows a representative glucomannan microscaffold.
**FIG. 25** shows a still image captured from a video of duck muscle tissue demonstrating spontaneous contraction.
**FIG. 26** shows duck liver pâté and foie gras butter made using duck steatotic liver cells.
**FIG. 27** shows salmon pâté and duck meat pâté prototypes made according to the methods described herein.
**FIG. 28A** shows an exemplary embodiment of a method of Cre delivery for the purpose of activating / silencing particular genes. **FIG. 28B** shows different methods of using Cre to induce a "switch" between activated gene sets relevant to meat creation (e.g., proliferation and differentiation).
**FIG. 29** shows an embodiment of a method of transposon-mediated genetic self-excision for purpose of footprint-free gene editing.
**FIG. 30** shows an embodiment of a solution blow spinning process for generating fibers.
**FIG. 31** shows another embodiment of a solution blow spinning process for generating fibers.

### DETAILED DESCRIPTION OF THE INVENTION

Disclosed herein are systems and methods for producing food products using cellular agriculture. Cell cultured food products provide many advantages that obviate or greatly reduce the negative impacts caused by traditional food production. These advantages are particularly felt in the area of meat production, which are typically produced using intensive livestock production or through fishing and fisheries. Instead of raising or catching live animals and fish to be harvested for their meat, cells having self-renewal capacity are isolated or created and grown in cell culture. In some cases, the cells are naturally capable of self-renewal such as embryonic stem cells and pluripotent progenitor cells. Alternatively, or in combination, the cells are manipulated to acquire the ability to self-renew. These cells are cultured and expanded to a desired quantity. The pluripotent cells can be piscine in origin and differentiated into roe as a food product. Oftentimes, the cells are cultured in a scalable manner, for example, using bioreactors that enable large-scale production. Various media formulations are optionally used to enable the maintenance of the capacity for self-renewal such as during expansion of the cell population, or to push the cells down certain differentiation pathways to generate the desired cell type. For example, in some instances, the cultured cells are induced to differentiate into muscle cells, adipose cells, or organ cells. In some cases, differentiation comprises transdifferentiation of cells into a different cell type. For instance, immortalized fibroblasts can be expanded and then transdifferentiated into myocytes, adipocytes, hepatocytes, and/or other desired cell types. Sometimes, the media formulations are modified from conventional media to not require fetal bovine serum or serum alternatives, which remain untested for human consumption. Media formulations can include low-serum or no-serum formulations that are derived from plants to reduce or obviate the use of animal components such as fetal bovine serum. Examples of plant-based formulations include soybean-based and plant hydrolysate-based media formulations. Media formulations often comprise at least one mushroom-based ingredient. In certain cases, the at least one mushroom-derived extract replaces fetal bovine serum in the media formulation. Some media formulations comprise at least one ingredient for enhancing the nutritional content of the cultured cells. Alternatively, co-culture systems are used to provide conditioned media systems that increase efficiency by obviating the need for recombinant protein production and allowing the culture media to be recycled. In addition to alternative media formulations, three-dimensional scaffolding and tissue engineering platforms are used to facilitate large-scale growth, in many cases. Oftentimes, scalable bioreactors provide the requisite growth needed for mass production. In some instances, three-dimensional scaffolding is used to provide structural support and guide the growth of the cultured cells into the desired structure and/or texture analogous with the equivalent food product produced using conventional methods. Alternatively or in combination, micro-scaffolds enable the growth of adherent cells in suspension culture such as in a bioreactor. These micro-scaffolds can be engineered to enhance stem cell proliferation, direct cell differentiation into the relevant lineage, and modulate flavor, texture, and tensile elasticity of the final meat product. Some adherent cells are modified to grow in suspension culture without requiring an adherent surface. Certain food products are produced using a homogeneous population of cells such as, for example, liver cells for making foie gras. Alternatively, some food products are produced using a heterogeneous population of cells such as a combination of muscle and fat cells. In some cases, a population of cells is differentiated into multiple cell types to create a heterogeneous population of differentiated cells. Alternatively, independent cell populations are differentiated into distinct cell types and subsequently combined. These methods using heterogeneous cell populations enable the production of certain tissues such as, for example, salmon meat that is composed of a combination of muscle and fat cells. Oftentimes, the cultured cells are modified to produce a desired cell or tissue phenotype. Cultured calls can be modified with one or more genetic constructs to confer a desired phenotype such as a state of self-renewal, differentiation into a cell or tissue type, or predisposition to steatosis. Cultured cells can also be modified through adjustments to the culture environment. For example, liver cells are optionally cultured in lipid rich media to induce steatosis via excess lipid uptake and storage inside the cytoplasm. In many cases, the steatotic liver cells are harvested and processed as foie gras or a foie gras food product. Harvested cells are typically processed to produce a desired consistency and/or texture. In some cases, the harvested cells are processed to achieve particular tastes, textures and other culinary properties that are indistinguishable from the high quality meats they are intended to reproduce. The systems and methods disclosed herein can enable the processing of ingredients such as, for example, spinning techniques to generate fibers used in producing synthetic food products. The fibers can be seeded with cultured cells or tissues (or non-cultured cells or other tissue) and/or processed to achieve a desired texture, composition, taste, or other food property.

The systems and methods for producing cell cultured food products disclosed herein provide numerous advantages. The cultured meat is not exposed to pathogens such as avian bird flu or various bacterial strains during production. Likewise, the systems and methods disclosed herein can provide for meat production without the use of antibiotics. This has the benefit of not inadvertently exposing humans to antibiotics while also avoiding the increased risk of bacteria developing antibiotic resistance. In addition, cultured food production does not require feed crops and avoids the production of animal waste, which often contains fecal coliform bacteria, ammonia, and phosphorus. For example, a substantial amount of land is devoted to growing feed crops for livestock, which entails the widespread use of fertilizer, pesticides, and herbicides. By contrast, cultured food products are capable of being produced with a relatively small environmental footprint.

Non-textured tissues such as foie gras and certain fish meats such as salmon are produced using various systems and methods described herein. Some methods enable production of cultured non-textured tissue having high lipid content such as steatotic hepatocytes useful for making foie gras. Such methods often comprise: a) obtaining a population of differentiated cells capable of self-renewal; b) culturing the population of differentiated cells; c) manipulating at least one lipid metabolic pathway to induce steatosis in the population of differentiated cells such that the cells accumulate high lipid content; and d) processing the population of differentiated cells into non-textured tissue. In some cases, the differentiated cells capable of self-renewal are obtained through transdifferentiation (e.g., direct cell reprogramming). Sometimes, methods described herein produce cultured organ tissue for human consumption. Such methods comprise: a) culturing a population of cells capable of self-renewal; b) inducing differentiation in the population of cells to generate organ tissue; and c) processing the organ tissue for human consumption.

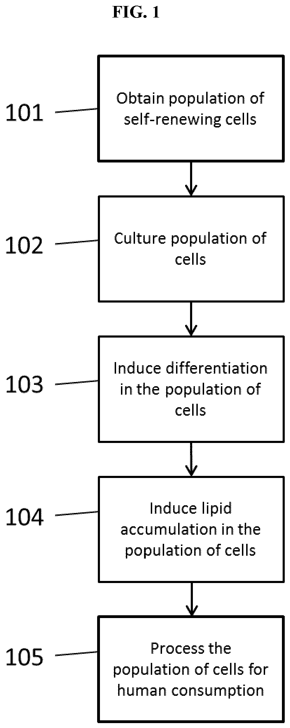

**FIG. 1** illustrates one embodiment of a process for culturing cells for human consumption. In this example, a population of self-renewing cells is obtained **101.** As described herein, self-renewing cells are sometimes embryonic stem cells, induced pluripotent stem cells, embryonic germ cells, immortalized differentiated cells, or nascent adult stem cells. The population of cells is cultured **102,** and typically expanded to the desired population size. Next, differentiation is induced in the population **103.** In some cases, differentiation comprises transdifferentiation of cells into a different cell type. In this example, cells in the population are differentiated into hepatocytes. Oftentimes, lipid accumulation is induced in the population of cells comprising differentiated hepatocytes **104.** Finally, the population of cells is processed for human consumption **105.** For example, hepatocytes are often processed into foie gras or a foie gras food product.

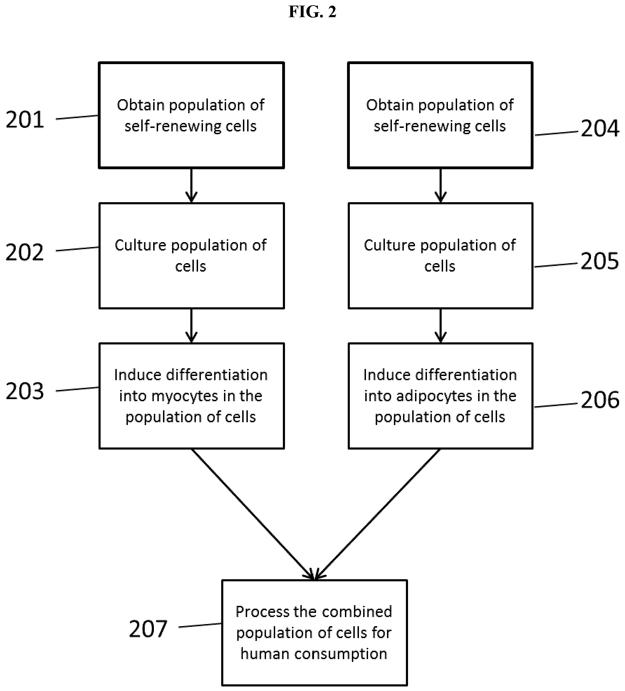

**FIG. 2** illustrates one embodiment of a process for culturing muscle tissue for human consumption. In this example, a first and a second population of self-renewing cells are obtained **201, 204.** The two populations of cells are cultured **202, 205,** and typically expanded to the desired population size. Next, differentiation is induced in the two populations **203, 206.** In this case, the differentiation into myocytes is induced in the first population of cells **203.** In some cases, differentiation comprises transdifferentiation of cells into a different cell type. Differentiation into adipocytes is induced in the second population of cells **206.** Finally, the two populations of cells are processed for human consumption **207.** In this case, the first and second populations are combined and processed into meat comprising both muscle and fat cells for human consumption.

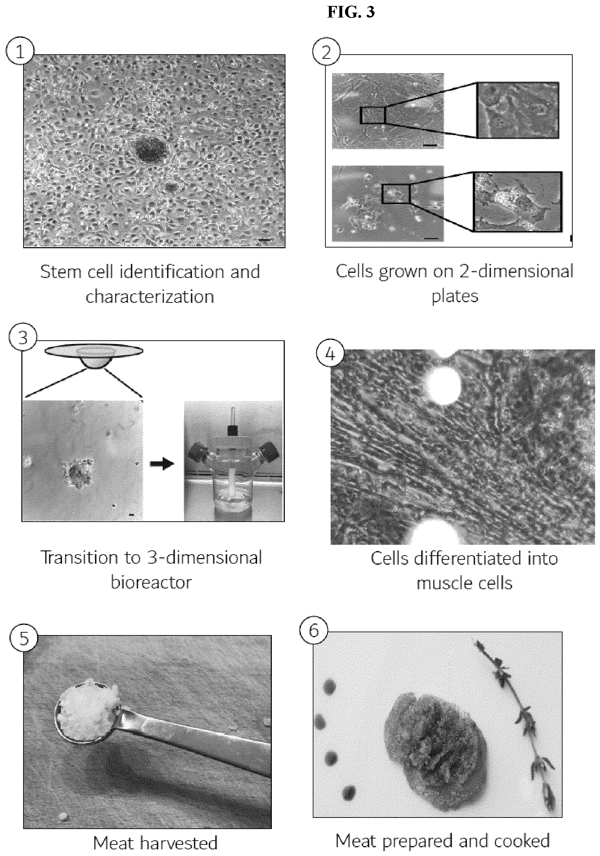

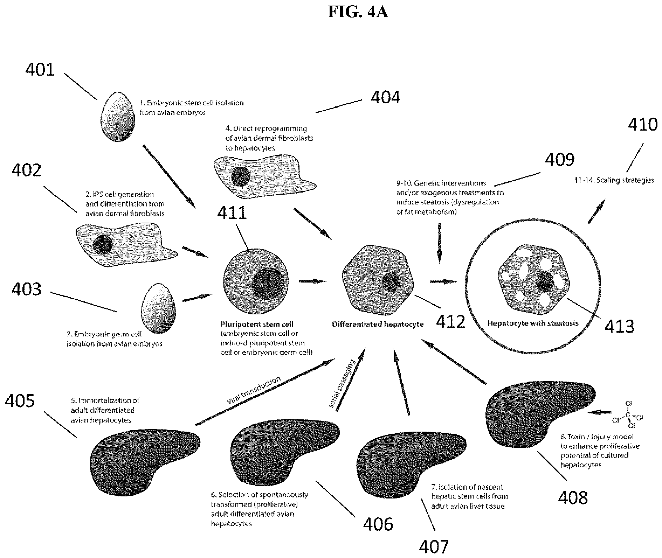

An overview of an exemplary process for preparing cultured meat for consumption is shown in **FIG. 3****.** First, stem cell identification, isolation, and characterization are carried out. These cells are initially grown in two-dimensional culture such as on a feeder cell layer. The cells are eventually transitioned into suspension culture in a bioreactor allowing for larger-scale cell growth. Subsequent to transitioning to suspension culture, the cells are differentiated into muscle cells. In some cases, differentiation comprises transdifferentiation of cells into a different cell type (e.g., from immortalized fibroblasts into muscle and/or fat cells). The meat is then harvested, and finally prepared and cooked. Various approaches can be used to obtain cell lines suitable for preparing cultured food products **(****FIGs. 4A-4B****).**

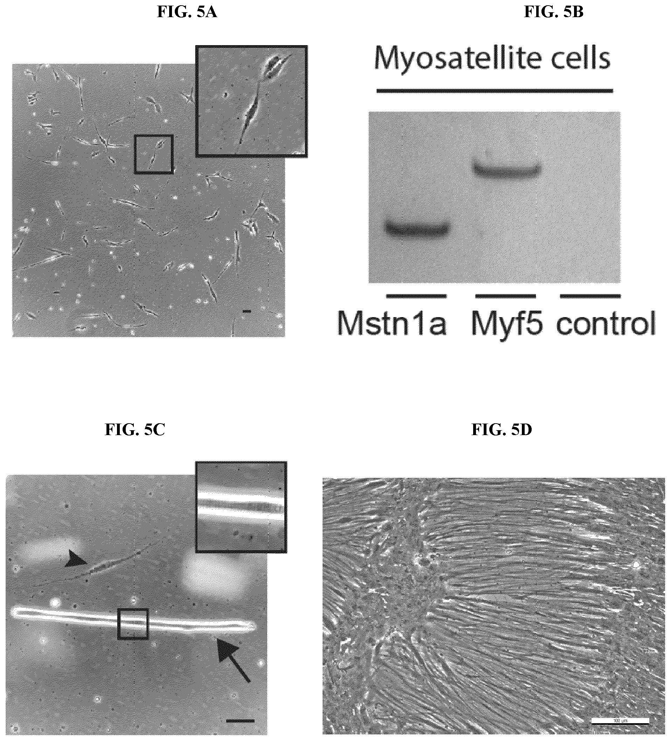

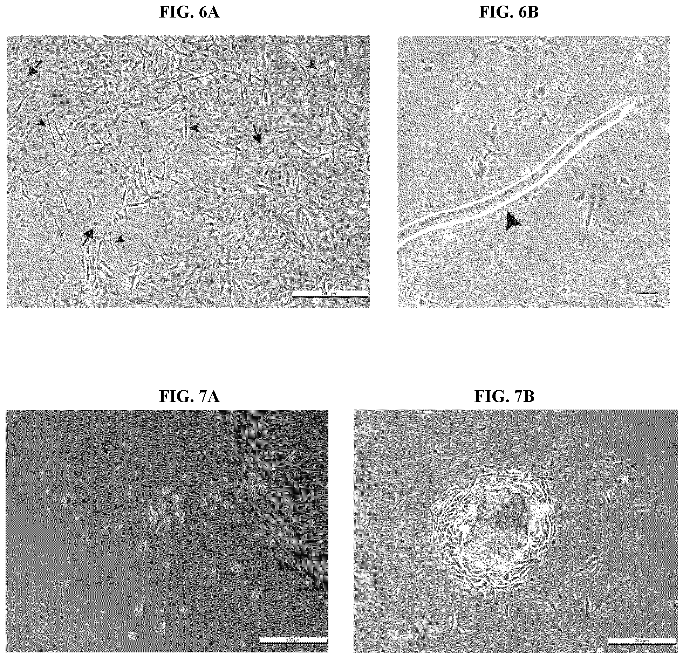

Disclosed herein, in certain aspects, are methods of producing synthetic food products comprising tissue derived from fish. In some embodiments, fish myocytes and adipocytes are utilized for development of fish-related foods based on their intrinsic regenerative capacity during early developmental stages. In an exemplary embodiment of this process, trout pre-adipocytes and myosatellite cells (capable of differentiating into myocytes) were isolated, cultured, and characterized. Trout myosatellite cells were isolated and then characterized as shown in **FIGs. 5A-5D****.** Where present, insets magnify image details, and the scale bar is equal to 10 µm in all micrographs unless otherwise indicated. Substantially pure populations of piscine myosatellite cells were successfully isolated and are shown in **FIG. 5A** with the myosatellite cells making up about 80% of the isolated cells. RT-PCR analysis of these isolated cells revealed expression of the transcription markers Mstn1a and Myf5 which are markers of pluripotency **(****FIG. 5B****).** Next, culture conditions were optimized for these cells. Culture media protocols were used to successfully differentiate myosatellite cells (arrowhead) into mature, differentiated myocytes (arrow) **(****FIG. 5C****).** The resulting sheets of trout myotubes differentiated from the myosatellite cells are shown in **FIG. 5D** (scale bar is 100 µm). In some embodiments, myosatellite cells can be co-cultured with pre-adipocytes. In an exemplary embodiment, salmon myosatellite cells (arrowheads) were co-cultured with salmon pre-adipocytes (arrows) for producing a food product comprising both muscle and fat cells or tissue as shown in **FIG. 6A** (scale bar is 100 µm). The pre-adipocytes were differentiated into adipocytes, and the myosatellite cells differentiated into myocytes (arrowhead) as shown in **FIG. 6B** (scale bar is 10 µm).

In some cases, salmon fibroblasts are used for producing a food product. Salmon fibroblasts can be induced to form spheroids for propagation in a bioreactor **(****FIG. 7A****)** (scale bar is 100 µm). The viability of these spheroids is confirmed by returning them to 2-Dimensional culture conditions and observing that the fibroblasts migrated circumferentially to form colonies **(****FIG. 7B****)** (scale bar is 100 µm). In some cases, the fibroblasts are propagated, and then transdifferentiated into desired cell types such as myocytes, adipocytes, hepatocytes, or any combination thereof.

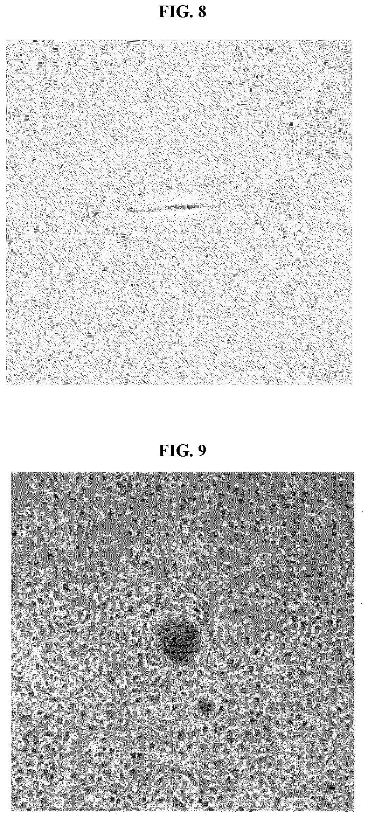

The methods disclosed herein are capable of being applied to various aquatic species. For example, cell cultures of bass myosatellite cells have also been successfully cultivated utilizing standard cell culture protocols **(****FIG. 8****).** In certain embodiments, disclosed herein are meat products comprising cells or tissue derived from one or more types of aquatic organisms. Sometimes, an aquatic organism is selected from the group consisting of sea bass, tuna, mackerel, blue marlin, swordfish, yellowtail, salmon, trout, eel, abalone, squid, clams, ark shell, sweetfish, scallop, sea bream, halfbeak, shrimp, flatfish, cockle, octopus, or crab. In certain cases, an aquatic organism is a type of fish selected from the group consisting of sea bass, tuna, mackerel, blue marlin, swordfish, yellowtail, salmon, trout, or flatfish. In some instances, the aquatic organism can be a round fish or flat fish. A round fish can include bass, catfish, Arctic char, cod, haddock, herring, sardines, tilapia, trout, red snapper, salmon, swordfish, and tuna. A flat fish can include flounder, sole, halibut, and turbot. Varieties of tuna include yellowfin, southern Bluefin, northern Bluefin, Thunnus alalunga, Thunnus atlanticus, and Thunnus obesus. Varieties of salmon include Atlantic salmon, sockeye salmon, Chinook salmon (also called king salmon), Coho salmon, chum salmon, and pink salmon. Varieties of trout include rainbow trout, cutthroat trout, brown trout, red mountain trout, brook trout, and lake trout.

Disclosed herein, in certain aspects, are methods of producing synthetic food products comprising tissue derived from avian species. In some embodiments, the synthetic food product comprises avian hepatocytes and/or liver tissue derived from a duck or goose. In some embodiments, the synthetic food product comprises steatotic liver tissue. In some embodiments, these methods utilize self-renewing cells (e.g. pluripotent or multipotent cells) for development of avian-related foods based on their intrinsic regenerative capacity during early developmental stages. Successfully isolated duck embryonic stem cells growing in culture are shown in **FIG. 9****.**

### Cell Lines

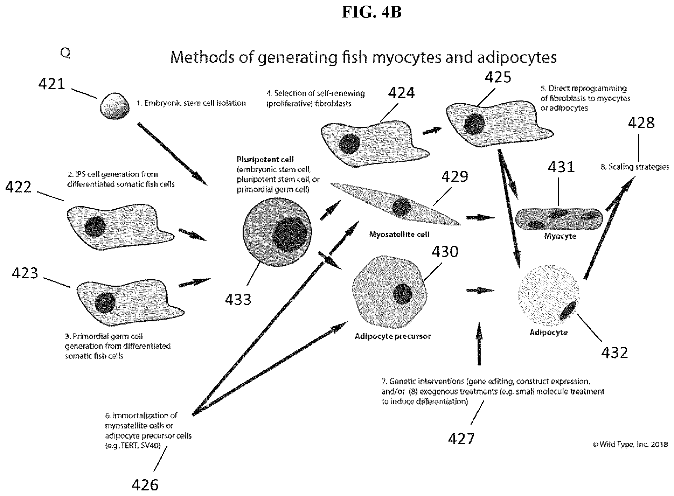

Some systems and methods disclosed herein comprise generation of cell line(s) capable of self-renewal for cultured food production. In one approach, embryonic stem cells are isolated. Embryonic stem cells are pluripotent stem cells generated from early-stage embryos. Typically, the embryonic stem cells are harvested from a blastocyst 4-5 days after fertilization has occurred. The blastocyst has an inner cell mass that is removed and placed in culture. Those cells that remain viable in cell culture conditions are used to establish a cell line capable of self-renewal. For example, **FIG. 4A** illustrates certain approaches for generating steatotic hepatocytes. In some instances, the embryonic stem cells are obtained from avian embryos such as, for example, duck or goose embryos **401.** These duck or goose embryonic stem cells are pluripotent stem cells **411** optionally used for producing cultured foie gras. Sometimes, avian embryonic stem cells are isolated from blastodermal cells in Eyal-Giladi and Kochav Stage 10 (EGK-X) avian embryos. For example, avian embryonic stem cells can be isolated by culturing them on inactivated STO feeders cells in an embryonic stem cell medium (ESA) with certain growth factors such as bFGF, IGF-1, mSCF, IL-6, OSM, LIF, IL-6, and IL-11 as described in Aubel P., Pain B. Chicken embryonic stem cells: establishment and characterization. Methods Mol. Biol. 2013; 1074:137-150. Successfully isolated duck embryonic stem cells growing in culture are shown in **FIG. 9****.** These approaches can also be utilized for generating fish myocytes and/or adipocytes **(****FIG. 4B****).**

Pluripotent stem cells such as embryonic or induced pluripotent stem cells can be used to produce edible fish roe (e.g., caviar or ikura). For example, fish pluripotent stem cells can be grown into roe. The roe may be usable in various food products such as caviar or sushi. The roe can be derived from salmon, smelt or Capelin, flying fish, sturgeon, pollock, herring, or other suitable type of fish. This approach can enable the use of fish pluripotent stem cells for the purpose of primordial germ cell (PGC) production, followed by maturation to yolk-rich eggs for human consumption.

In some cases, fish embryonic stem cells (or fish-derived iPS cells) are cultured in a media formulation containing BMP4, retinoic acid, and EGF, for the purpose of creating primordial germ cells. Expression of several transcription factors (e.g., VASA, deadend, DAZL, CXCR4b, or any combination thereof) are induced in the presence of luteinizing hormone and/or DHP for the purpose of PGC maturation. PGC formation and maturation of eggs can be enhanced with induced expression of additional genes such as Buckyball.

Once isolated, the embryonic stem cells are usually maintained in an undifferentiated state. Sometimes, published protocols are modified to maintain the embryonic stem cells in an undifferentiated state. Modifications to published protocols can include the use of optimized matrix substrates and the use of optimized media formulations to achieve persistent cellular proliferation and maintenance of a de-differentiated state. In some cases, avian embryonic stem cells are maintained in the undifferentiated state using leukemia inhibitory factor (LIF), a member of the interleukin-6 family of cytokines as described in Horiuchi et a., Chicken leukemia inhibitory factor maintains chicken embryonic stem cells in the undifferentiated state. J.Biol.Chem. 2004;279: 24514-24520. Alternatively, avian embryonic stem cells are maintained in an undifferentiated state without requiring the use of LIF in the culture media. In certain instances, the avian embryonic stem cells are maintained in an undifferentiated state in a culture media formulation containing LIF without the use of other cytokines or feeder cells. A media formulation sometimes comprises recombinant LIF. In some cases, recombinant LIF is produced as a fusion protein with an affinity tag for purification. Typically, a fusion protein with an affinity tag for purification uses at least one affinity tag selected from glutathione S-transferase (GST), FLAG tag, S-tag, heavy chain of protein C (HPC), streptavidin binding peptide, streptavidin, streptavidin tag, histidine affinity tag, polyhistidine tag, polycysteine tag, polyaspartate tag, albumin-binding protein (ABP), calmodulin binding peptide, cellulose binding domain, chitin binding domain, and choline binding domain. In some instances, an affinity purified fusion protein is cleaved or digested to remove the affinity tag.

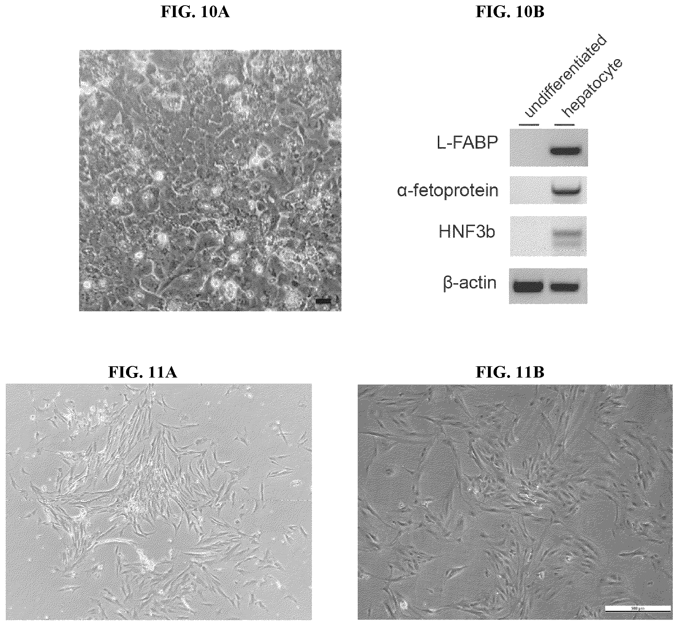

The isolated embryonic stem cells are typically differentiated into a desired cell type. The desired cell type is usually the fully differentiated cell that makes up the food product or a portion of the food product. Sometimes, a differentiated cell is a hepatocyte or liver cell **412.** **FIG. 10A** shows duck hepatocytes growing in culture following successful differentiation. Differentiation was confirmed by measuring markers of hepatocyte differentiation (L-FABP, alpha-fetoprotein, and HNF3b, with beta-actin as a loading control) using RT-PCR **(****FIG. 10B****).** As shown in **FIG. 10B****,** hepatocytes (right lane) generates observable expression of the hepatocyte differentiation markers compared to a lack of expression in the control undifferentiated cells (left lane). Cultured hepatocytes are often used to generate foie gras. In some instances, a differentiated cell is a myocyte or skeletal muscle cell. A differentiated cell is often an adipocyte that is optionally used in combination with other cell types such as myocytes for production of cultured meat products having both fat and muscle tissue. For example, salmon myocytes and adipocytes are sometimes used to produce sushi grade salmon meat for human consumption. Oftentimes, differentiated cells are organ cells such as, for example, striated or skeletal muscle cells, smooth muscle cells, cardiac muscle cells, spleen cells, thymus cells, endothelial cells, blood cells, bladder cells, liver cells, kidney cells, pancreas cells, lung cells, or any combination thereof. Alternatively, the desired cell type is sometimes an intermediate cell type such as an adult stem cell or progenitor cell useful for generating the fully differentiated cell type. Oftentimes, differentiation is achieved by optimization of standard protocols such as described in the website www.abcam.com/protocols/hepatocyte-differentiation-protocol. For example, embryonic stem cells and induced pluripotent stem cells are both capable of being differentiated into hepatocytes by splitting the cells into Matrigel coated plates in mTESR media with ROCK inhibitor Y27632, treating with definitive endoderm (DE) media, followed by hepatic endoderm (HE) media, immature hepatocyte (IMH) media, and finally mature hepatocyte (MH) media. Some media formulations are modified to enhance proliferation, differentiation, or other desired qualities in the cultured cells.

Some methods provide for generation of induced pluripotent stem cells **402** used to produce the food products disclosed herein. Sometimes, an episomal reprogramming strategy is employed to create induced pluripotent stem cells (iPSC) from fibroblasts, using an episomal reprogramming strategy optimized from the approach reviewed in Drozd et al., Generation of human iPSCs from cells of fibroblastic and epithelial origin by means of the oriP/EBNA-1 episomal reprogramming system. Stem Cell Research & Therapy. 2015; 6:122. For example, in some instances, at least one episomal vector expressing a combination of reprogramming factors such as Oct3/4, Sox2, Klf4, L-Myc, C-Myc, Lin28, Nanog, and Lin4 are introduced cells originating from adult avian fibroblasts. Additional factors that are sometimes added include p53 for overcoming reprogramming barriers such as cellular senescence. As an example, an (oriP/EBNA-1)-based episomal vector enables reprogramming while persisting episomally inside of the reprogrammed cells. Episomal reprogramming provides an approach that precludes the generation of a "genetic footprint" because the episomal approach generates iPSC lines without integration of reprogramming vectors that result from classical viral reprogramming strategies. Finally, in some cases, differentiation of iPSCs is achieved using optimized standard protocols as described elsewhere herein for embryonic stem cells.

In some cases, embryonic germ cells are used as a source of pluripotent stem cells capable of self-renewal **403.** Embryonic germ cells are capable of differentiation into the desired cell type such as, for example, mature hepatocytes for the purpose of liver tissue production. Sometimes, embryonic germ cells are isolated using protocols such as described in Guan et al., Derivation and characteristics of pluripotent embryonic germ cells in duck. Poultry Science. 2010; 89(2): 312-317. For example, duck embryonic tissue at stage 28 is obtained and subsequently dissociated using trypsin. The dissociated cells are harvested by centrifugation and then cultured in suspension culture in the presence of stem cell factor (SCF), leukemia inhibitory factor (LIF), and basic fibroblast growth factor (FGF). The embryonic germ cells typically form colonies, which are then reseeded into plates with feeder cells. In some instances, the isolated embryonic germ cells are expanded and optionally differentiated into the desired cell type for food production as described herein.

In some cases, differentiated cells are reprogrammed into the desired cell type without creating an intermediate pluripotent cell type **404.** This process is sometimes referred to as transdifferentiation in which the desired cell type is generated from a non-stem cell. Sometimes, this method is carried out as described in Simeonov KP and Uppal H, Direct reprogramming of human fibroblasts to hepatocyte-like cells by synthetic modified mRNAs. PLOS ONE. 2014; 9(6): e100134. Oftentimes, isolated fibroblasts are reprogrammed into hepatocytes or hepatocyte-like cells by culturing the fibroblasts in an optimized hepatic growth media while expressing at least one factor of FOXA1, FOXA3, HNF1A, and HNF4A. In some cases, HNF1A and at least two of FOXA1, FOXA3, and HNF4A are expressed in the fibroblasts to convert them into hepatocytes. Oftentimes, expression or overexpression of any of the foregoing factors into differentiated cells for reprogramming is obtained by introducing exogenous DNA or RNA into the cells using genetic techniques known in the art. Alternatively, the isolated fibroblasts are reprogrammed into myocytes or adipocytes. In some cases, the reprogramming entails transdifferentiation of the isolated cells such as fibroblasts into a different cell type. For example, reprogrammed salmon myocytes and adipocytes are useful for producing edible salmon meat such as salmon grade sushi.

Sometimes, fully differentiated cells of the desired cell type are immortalized to generate a cell line capable of self-renewal. For example, myocytes, adipocytes, and/or hepatocytes can be immortalized for purposes of food production. Oftentimes, a classically-defined immortalization strategy using transformation is applied to differentiated adult cells to generate cell lines with indefinite proliferative capacity **405.** In various cases, cell immortalization is achieved by artificial expression of key proteins required for immortality. In some examples, differentiated adult cells are immortalized via expression or overexpression of at least one of SV40 Large T Antigen, hTERT, HPV E6/E7, EBV, MycT58A, RasV12, and p53. In some cases, avian hepatocytes are immortalized to generate an adult avian hepatocyte cell line capable of self-renewal. Such a cell line aids in the large-scale production of hepatocyte-based food products such as foie gras. Sometimes, salmon myocytes and/or adipocytes are immortalized to generate adult salmon myocyte and adipocyte cell lines capable of self-renewal. Such immortalized cell lines allow for large-scale production of salmon meat such as sushi grade salmon. In some cases, differentiated cell lines (e.g., fibroblasts) are immortalized and subsequently transdifferentiated into a desired cell type such as myocytes, adipocytes, hepatocytes, or any combination thereof, and can be used to generate various types of food products such as fish meat or avian liver.

In some cases, an immortalized cell line capable of self-renewal is generated without transformation or direct genetic modification **406,** in certain instances. Under this approach, a cell population is typically harvested and sequentially passaged for weeks until most cells undergo senescence, while a few spontaneous mutations arise that lead to the generation of a cell line with indefinite replicative potential. In some cases, the cell population is obtained from embryonic differentiated cells such as, for example, embryonic (differentiated) liver cells. This process is capable of being applied to avian cells to generate immortalized avian hepatocytes such as described in Lee et al., Establishment of an immortal chicken embryo liver-derived cell line. Poultry Science. 2013; 92(6):1604-12. This method obviates the need for integrating viruses in producing an immortalized cell line without the use of exogenous genetic material or genetic manipulation. For example, **FIG. 11A** shows duck self-renewing cells generated by culturing primary fibroblasts and harvesting colonies of dividing cells after 6-8 weeks. **FIG. 11B** shows trout self-renewing cells generated by culturing primary fibroblasts and harvesting colonies of dividing cells after 6-8 weeks. The self-renewing cells were then characterized for morphology, proliferation rate, and proliferative capacity (e.g. number of passages achieved without changes in morphology, proliferative rate, and without genomic instability). In some cases, differentiated cell lines (e.g., fibroblasts) are immortalized and subsequently transdifferentiated into a desired cell type such as myocytes, adipocytes, hepatocytes, or any combination thereof, and can be used to generate various types of food products such as fish meat or avian liver.

In some cases, nascent adult stem cells capable of self-renewal are isolated. For example, the liver is one of the few organs with regenerative capacity in adult mammalian and avian organisms. The existence of stem cells within adult hepatic tissue is reviewed in Navarro-Alvarez et al., Hepatic stem cells and liver development. Methods Mol Biol. 2010;640:181-236. Accordingly, in some instances, nascent hepatic stem cells are isolated, cultivated, and expanded for use in cultured food production **407.** Sometimes, fish pre-adipocytes and satellite cells are isolated and cultured to form cell lines suitable for expansion and differentiation into adipocytes and myocytes, respectively. The differentiated adipocytes and myocytes are usually then co-cultured together at a certain ratio to produce a desired final composition of adipocytes and myocytes in the resulting food product.

In some cases, liver cells are treated with toxic chemical compounds to generate cells with enhanced proliferative capacity **408.** For example, such exposure to toxic compounds has been shown to elicit a proliferative response within the liver parenchyma. Accordingly, these liver cells with enhanced proliferative capacity are cultivated and expanded for use in cultured food production, in various instances.

In some cases, cells obtained using any of the foregoing methods is further modified to generate a cell line that does not require an adherent substrate for growth or survival. This method sometimes involves the creation of hepatocytes that do not require an extracellular matrix for attachment in order to survive and proliferate. Advantages of being able to grow cells in suspension culture include the ability to easily and rapidly scale up growth. Oftentimes, suspension cultures are less labor and/or resource intensive because they are able to culture cells based on volume rather than surface area and allow for passaging of cells without detachment steps such as trypsinization. A cell line that does not require an adherent substrate is often combined with a bioreactor cell culture system to enhance large-scale food production. In certain cases, stem cells are adapted to suspension culture, allowing for expansion before differentiation into a differentiated cell type such as, for example, hepatocytes, myocytes, or adipocytes. Alternatively, in some instances, stem cells are differentiated into hepatocytes and subsequently transitioned to 3-dimensional suspension cultures. In some cases, differentiated cells are transdifferentiated into a desired cell type.

### Genetic modification of cell lines

Disclosed herein are methods for performing one or more modifications on a cell or cell line. In some cases, the modification is a genetic modification carried out by introducing nucleic acids or genetic constructs into a cell or cell line. Cells can be modified to provide the capacity for self-renewal, differentiation into a desired cell type, obtaining a particular cell phenotype (e.g. steatosis), or other desirable changes. In some cases, cells are modified through introduction of exogenous nucleic acids such as one or more DNA construct(s). The introduction of foreign nucleic acids into the cell can be accomplished using various methods including, but not limited to, transfection, transduction, viral transduction, microinjection, lipofection, nucleofection, or transformation. For example, cells of a particular cell type can be transdifferentiated into a desired cell type.

In addition, gene editing systems such as TALENS (transcription activator-like effector nucleases) or CRISPR can be utilized to perform genetic modification in cells. For example, CRISPR can be customized because the active form consists of an invariant Cas9 protein and a programmable guide RNA (gRNA). The Cas9-gRNA complex probes DNA for the protospacer-adjacent motif (PAM) sequence followed by formation of an R-loop. Upon formation of a macromolecular complex comprising Cas9, gRNA, and the target DNA, the Cas9 protein generates two nicks in the target DNA, creating a blunt double-strand break that is predominantly repaired by the non-homologous end joining pathway or template-directed homologous recombination.

Genetic constructs can comprise a promoter and ORF(s) for one or more genes. A genetic construct may be introduced into a cell population followed by selection for a stable cell line that has incorporated the construct. For example, a plasmid comprising a gene of interest and a neo gene providing resistance to G418 can be linearized (e.g. cut once with a restriction endonuclease) and transfected into a duck hepatocyte fibroblast cell line and then selected with G418 to obtain fibroblasts successfully incorporating the linear plasmid vector into the genome. Examples of promoter include cytomegalovirus (CMV), CMV enhancer fused to chicken beta-actin promoter (CAG), human elongation factor 1- α (HEF-1α), telomerase reverse transcriptase (hTERT) promoter, and simian virus (SV40) promoter. In some cases, a promoter having a low or nonexistent basal transcription rate is used to minimize or prevent leaky expression. As an example, expression of a recombinase used in various constructs described herein can cause irreversible changes to a cell (e.g. by exciting genes involved in maintaining pluripotency). Thus, in some embodiments, the construct(s) comprises a promoter allowing no more than 1 transcription event per mitotic cell cycle. In some cases, the promoter allows no more than 1, 2, 3, 4, 5, 6, 7, 8, 9, 10, 20, 30, 40, or 50 transcription events per mitotic cell cycle (on average). In some cases, the promoter allows less than 1 transcription event per mitotic cycle (on average). Sometimes, the promoter allows no transcription events per mitotic cycle (e.g. when the average is less than half a transcription event per mitotic cycle).

The genetic modifications allow generation of cell lines that possess desired properties. For example, modified cell lines may express gene(s) that maintain the cell line in a state of self-renewal and/or proliferation. A state of self-renewal can be a state of proliferation or division while maintaining an undifferentiated state. As an example, induced pluripotent stem cells having the self-renewal property can be generated from differentiated adult cells by expressing one or more of Oct3/4, Sox2, Klf4 and c-Myc. In some cases, a cell can be both differentiated and have capacity for indefinite proliferation (e.g. immortalized fibroblasts). Sometimes, the modified cell lines are responsive to an inducing agent that triggers a switch from one phenotype to another. The switch can be from a state of self-renewal to a differentiated state (e.g. myosatellite cells into myocytes or pre-adipocytes into adipocytes). The methods disclosed herein can comprise one or more constructs for inducible adipogenesis. For example, the method may utilize a first construct comprising one or more pluripotency genes for promoting cell division and/or maintaining pre-adipocytes in an undifferentiated state, and a second construct comprising a TRE, one or more adipogenic genes, and a regulatory factor (e.g. Cre recombinase) for inactivating the pluripotency genes. In some cases, the switch comprises changing from one differentiated cell type into another differentiated cell type (e.g. from adult fibroblasts into hepatocytes). Sometimes, the switch does not entail a change in cell type but instead comprises a change in cell phenotype or a cellular characteristic. As an example, the switch can induce a hepatocyte to undergo steatosis, become predisposed to steatosis (e.g. more likely to undergo steatosis under the appropriate conditions such as incubation with a fatty acid), or results in enhanced steatosis (e.g. increased lipid accumulation compared to a control). Examples of genes implicated in adipogenesis include FABP4, GLUT4, ADIPOQ, AGPAT2, PLIN1, LEP, and LPL. In some instances, the construct comprises FABP4, GLUT4, ADIPOQ, AGPAT2, PLIN1, LEP, LPL, CEBPalpha, CEBPbeta, PPARgamma, or any combination thereof. The construct can comprise at least one, at least two, at least three, at least four, at least five, at least six, or all seven of the ORFs for genes selected from the group consisting of FABP4, GLUT4, ADIPOQ, AGPAT2, PLIN1, LEP, and LPL.

In some instances, cells are modified to express (inducible expression or constitutively active expression) one or more pluripotency genes that promote cell division. In certain cases, the pluripotency gene(s) promote at least about 50, at least about 100, at least about 150, at least about 200, at least about 250, at least about 300, at least about 350, at least about 400, at least about 500, at least about 600, at least about 700, at least about 800, at least about 900, or at least about 1000 cell divisions. In some cases, the number of cell divisions for a given cell line or population of cells is monitored for quality control. For example, in some instances, cell lines or populations that exceed a threshold cell division count are not used for producing cultured food products. In some cases, the threshold cell division count is at least about 100, about 200, about 300, about 400, about 500, about 600, about 700, about 800, about 900, about 1000, about 2000, about 3000, about 4000, about 5000, about 6000, about 7000, about 8000, about 9000, about 10000, about 20000, about 30000, about 40000, or about 50000 or more cell divisions. In some cases, the threshold cell division count is no more than about 100, about 200, about 300, about 400, about 500, about 600, about 700, about 800, about 900, about 1000, about 2000, about 3000, about 4000, about 5000, about 6000, about 7000, about 8000, about 9000, about 10000, about 20000, about 30000, about 40000, or about 50000 or more cell divisions.

Disclosed herein are inducible cells that are modified using constructs that are responsive to an inducing agent. These modified cells can be used for generating cultured food products such as fish meat, avian liver tissue, and other foods. The modified cells can be used to control proliferation, differentiation, cell phenotype (e.g., steatosis/lipid accumulation), or other cellular properties. Exemplary non-limiting examples of such inducible systems are the Tet-on/off systems which utilize tetracycline/doxycycline as the inducing agent. Other inducible systems are also contemplated for carrying out the methods described herein. Examples of non-Tet inducible systems include the coumermycin inducible expression system, the RheoSwitch^{®} Mammalian Inducible Expression system, estrogen receptor inducible systems, cumate-inducible systems, and Cre-Lox recombinase systems. In some cases, cell lines are generated that have stably incorporated the inducible systems or constructs described herein. Alternatively, cells can be modulated to transiently express the inducible systems or constructs described herein (e.g., via transient transfection of at least one construct).

A Tet-on or Tet-off system typically utilizes a tetracycline transactivator protein. TetO sequences are typically positioned upstream of any ORF(s) whose expression is sought to be controlled using the Tet system. A promoter and the TetO sequence(s) can make up a tetracycline response element (TRE). In some cases, the TRE consists of TetO sequence(s) and is placed upstream of a promoter and the ORF(s) for one or more genes of interest. In the Tet-on system, the transactivator protein has a strong binding affinity for TetO operator sequence(s) when it is not bound by tetracycline (or a derivative such as doxycycline). In the absence of tetracycline, the transactivator protein does not bind to the tetracycline response element (TRE). When tetracycline is added, it binds to the transactivator protein and causes the transactivator protein to bind to the TRE to induce expression of downstream ORF(s). In a Tet-off system, the transactivator protein has a strong binding affinity for TetO operator sequence(s) only when it is not bound by tetracycline. In the absence of tetracycline, the transactivator protein binds the TetO sequences and promotes expression of the downstream ORF(s). Added tetracycline binds to the transactivation protein causing a conformational change that results in decreased or loss of binding to the TRE, resulting in reduced expression of the downstream ORF(s).

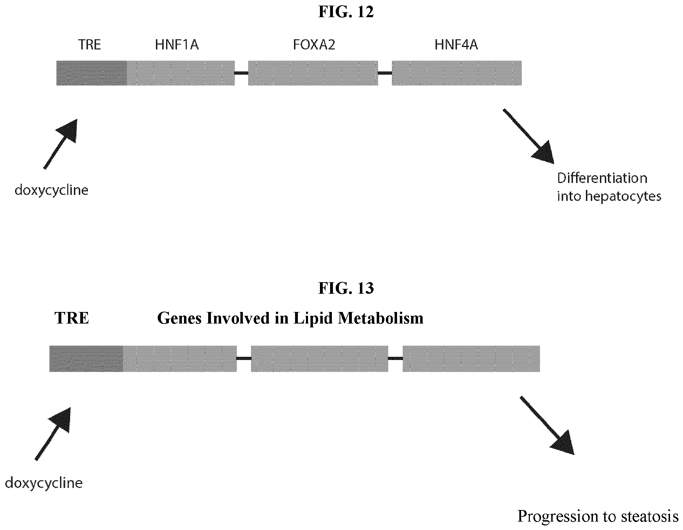

**FIG. 12** shows an exemplary embodiment of a genetic construct that can be introduced into a cell to provide inducible differentiation into a hepatocyte. The construct comprises a tetracycline responsive element (TRE) and ORFs for the hepatocyte reprogramming factors HNF1A, FOXA1, and HNF4A. The construct can be stably transformed into a target cell such as a pluripotent or multipotent cell. In some cases, the construct can be stably transformed into a terminally differentiated cell such as an immortalized fibroblast (obtained according to the techniques described herein). Expression of the ORFs is normally suppressed in the absence of tetracycline. Treatment with tetracycline induces expression of the ORFs, which pushes the cells towards differentiation into a hepatocyte. Thus, a cell line stably incorporating this construct can be induced to differentiate into a hepatocyte via treatment with tetracycline/doxycycline. In certain cases, the construct comprises at least one of HNF1A, FOXA1, and HNF4A. Sometimes, the construct comprises at least two of HNF1A, FOXA1, and HNF4A. In some instances, the construct comprises HNF1A, FOXA1, and HNF4A. The construct can comprise HNF1A, FOXA1, HNF4A, or any combination thereof. In certain instances, the construct comprises HNF1A and FOXA1; HNF1A and HNF4A; or FOXA1 and HNF4A.

**FIG. 13** shows an exemplary embodiment of a construct that can be introduced into a cell to allow inducible expression of one or more proteins that predispose the cell to steatosis. The construct comprises a tetracycline responsive element (TRE) and the ORF for one or more genes involved in lipid metabolism. The construct can be stably transformed into a target cell such as a pluripotent or multipotent cell. In some cases, the construct can be stably transformed into a terminally differentiated cell such as a fibroblast. The TRE suppresses expression of the ORFs but allows the ORFs to be transcribed in the presence of tetracycline or doxycycline. Thus, a cell line stably incorporating this construct can be induced to undergo steatosis or become predisposed to steatosis via treatment with tetracycline/doxycycline. In some cases, the construct comprises the ORF for ZFP423. In some cases, the construct comprises the ORF for ATF4 (activating transcription factor 3) (Kim JY et al., Activating transcription factor 3 is a target molecule linking hepatic steatosis to impaired glucose homeostasis. J Hepatol. 2017 Aug;67(2):349-359). In some cases, the construct comprises the ORF for SREBP-1c (Ferre P et al., Hepatic steatosis: a role for de novo lipogenesis and the transcription factor SREBP-1c. Diabetes Obes Metab. 2010 Oct;12 Suppl 2:83-92). Other genes are contemplated for use in the methods and construct systems described herein and include: LPIN1, PPAR, APOC3, APOE, ORL1, PEMT, MTTP, SREBP, STAT3, KLF6, or any combination thereof. In some cases, the construct comprises at least one, at least two, at least three, at least four, at least five, at least six, at least seven, at least eight, at least nine, at least ten, or at least eleven genes selected from the group consisting of: ATF4, ZFP423, LPIN1, PPAR, APOC3, APOE, ORL1, PEMT, MTTP, SREBP, STAT3, and KLF6.

Exemplary genes utilized in the methods described herein are listed in **Table 1** below. Expression of genes involved in lipid metabolism can be induced or enhanced to facilitate or enhance lipid accumulation and/or steatosis in target cells such as hepatocytes or adipocytes. Hepatocyte reprogramming factor(s) can be used to reprogram a cell type into hepatocytes such as transdifferentiation of fibroblasts into hepatocytes. Similarly, myocyte reprogramming factor(s) can be used to reprogram a cell type such as a fibroblast into a myocyte. Adipocyte reprogramming factor(s) can be used to reprogram a cell type such as a fibroblast into an adipocyte. Likewise, genes involved in myocyte differentiation and adipogenesis can be used to induce differentiation from myosatellite cells into myocytes and pre-adipocytes into adipocytes, respectively. Finally, various genes are listed that can be used to generate induced pluripotent stem cells (iPSCs). Any one gene or combination of genes listed in **Table 1** is contemplated for the stated purposes described herein.

**Table 1 - Gene List**

| **No. / Gene Name / Gene Symbol and Synonyms / HGNC ID** |
|---|
| **Genes Involved in Lipid Metabolism / Steatosis** |
| No. 1 / Activating Transcription Factor 4 / ATF4 / 786 |
| No. 2 / Lipin 1 / LPIN1 / 13345 |
| No. 3 / Peroxisome Proliferator Activated Receptor Gamma / PPARG / 9236 |
| No. 4 / Peroxisome Proliferator Activated Receptor Delta / PPARD / 9235 |
| No. 5 / Peroxisome Proliferator Activated Receptor Alpha / PPARA / 9232 |
| No. 6 / Apolipoprotein C3 / APOC3 / 610 |
| No. 7 / Apolipoprotein E / APOE / 613 |
| No. 8 / Opioid Related Nociceptin Receptor 1 / ORL1 / 8155 |
| No. 9 / Phosphatidylethanolamine N-Methyltransferase / PEMT / 8830 |
| No. 10 / Microsomal Triglyceride Transfer Protein / MTTP / 7467 |
| No. 11 / Sterol Regulatory Element Binding Transcription Factor 1 / SREBPF1 / SREBP1 / 11289 |
| No. 12 / Sterol Regulatory Element Binding Transcription Factor 2 / SREBPF2 / SREBP2 / 11290 |
| No. 13 / Signal Transducer And Activator Of Transcription 3 / STAT3 / APRF / 11364 |
| No. 14 / Kruppel Like Factor 6 / KLF6 / CPBP / ZF9 / 2235 |
| No. 15 / Zinc Finger Protein 423 / ZFP423 / 16762 |

| **Hepatocyte Reprogramming Factors** |
|---|
| No. 16 / Hepatocyte Nuclear Factor 1 Alpha / HNF1A / 11621 |
| No. 17 / Forkhead Box A1 / FOXA1 / 5021 |
| No. 18 / Hepatocyte Nuclear Factor 4 Alpha / HNF4A / 5024 |

| **Myocyte Differentiation Factors** |
|---|
| No. 19 / Myogenin / MYOG / 7612 |
| No. 20 / Myogenic Factor 6 / MRF4 / MYF6 / 7566 |
| No. 21 / Myogenic Factor 5 / MYF5 / 7565 |
| No. 22 / Myogenic Differentiation 1 / MYOD1 / MYOD / 7611 |

| **Genes Involved in Adipogenesis** |
|---|
| No. 23 / Fatty Acid Binding Protein 4 / FABP4 / 3559 |
| No. 24 / Insulin-Responsive Glucose Transporter Type 4 / GLUT4 / SLC2A4 / 11009 |
| No. 25 / Adiponectin, C1Q And Collagen Domain Containing / ADIPOQ / 13633 |
| No. 26 / 1-Acylglycerol-3-Phosphate O-Acyltransferase 2 / AGPAT2 / 325 |
| No. 27 / Perilipin 1 / PLIN / PLIN1 / 9076 |
| No. 28 / Leptin / LEP / LEPD / 6553 |
| No. 29 / Lipoprotein Lipase / LPL / LIPD / 6677 |
| No. 30 / CEBPalpha / CEBPA / 1833 |
| No. 31 / CEBPbeta / CEBPB / 1834 |

| **Genes used to generate iPSCs** |
|---|
| No. 32 / Octamer-Binding Protein 3/4 / OCT4 / OCT3 / 9221 |
| No. 33 / SRY-Box 2 / SOX2/ 11195 |
| No. 34 / Kruppel Like Factor 4 / KLF4 / 6348 |
| No. 35 / MYC Proto-Oncogene, BHLH Transcription Factor / C-MYC / CMYC / 7553 |
| No. 36 / MYCN Proto-Oncogene, BHLH Transcription Factor / N-MYC / NMYC / 7559 |
| No. 37 / MYCL Proto-Oncogene, BHLH Transcription Factor / L-MYC / LMYC / 7555 |
| No. 38 / Nanog Homeobox / NANOG / 20857 |
| No. 39 / Lin-28 Homolog A / LIN28A / 15986 |
| No. 40 / GLIS Family Zinc Finger 1 / GLIS1 / 29525 |

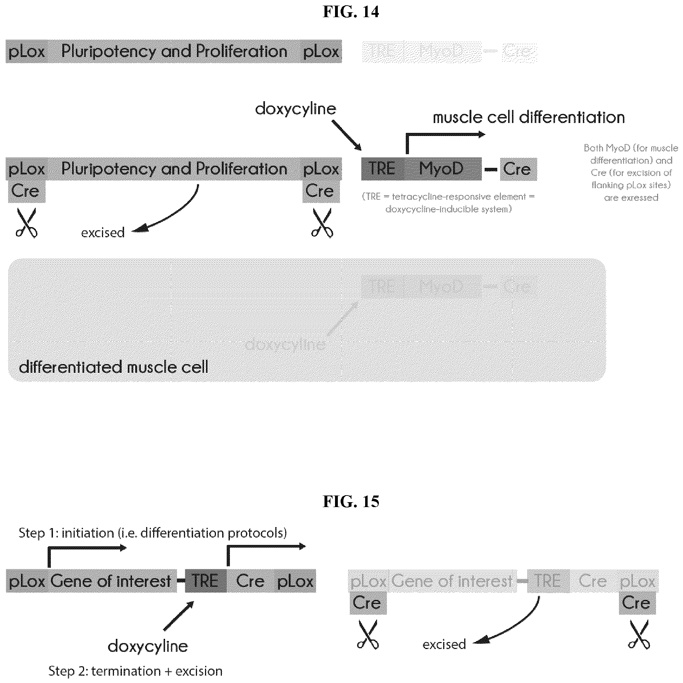

**FIG. 14** shows an exemplary embodiment of a DNA construct system that can be introduced into a cell to allow a proliferation/differentiation switch from a pluripotent phenotype into a differentiated phenotype. This system has a first construct comprising a pluripotency cassette providing constitutive expression of the ORFs for pluripotency factors (e.g. Oct4, Sox2, Klf4, I-Myc). The pluripotency factors of the first construct are flanked by pLox sites. The system has a second construct comprising a differentiation cassette providing tetracycline inducible expression of MyoD and Cre recombinase. The addition of an inducing agent such as tetracycline or doxycycline can induce expression of MyoD and Cre recombinase. MyoD expression can help cause the cell to undergo differentiation into a muscle cell. The Cre recombinase enzyme can catalyze the excision of the pluripotency factors flanked by the pLox sites. Next, the inducing agent can be removed to cease induction of MyoD and Cre recombinase expression. An advantage of this system is the low footprint left by the system following excision of the pluripotency factors and removal of the inducing agent. Other genes can be used for inducing myogenesis and can include Myogenin (MyoG), MRF4, and Myf5. In some cases, MyoD, MyoG, MRF4, Myf5, or any combination thereof are used for inducing myogenesis. Sometimes, the methods and/or construct systems described herein utilize at least one, at least two, at least three, or all four myogenic factors selected from the group consisting of MyoD, MyoG, MRF4, and Myf5.

**FIG. 15** shows an exemplary construct that can be introduced into a cell to provide an inducible "off-switch". This construct comprises ORF(s) for one or more genes of interest and an expression cassette comprising TRE and Cre recombinase, which are flanked by pLox sites. The construct as shown in **FIG. 15** comprises the TRE-Cre expression cassette located downstream of the ORF(s). Alternatively, in construct can have the TRE-Cre expression cassette located upstream of the ORF(s). A promoter is generally positioned upstream of the ORF(s) and is a separate promoter from the TRE such that the ORF(s) are expressed independently of the Cre recombinase. Addition of an inducing agent can cause a cell line stably incorporating this construct to express Cre recombinase for catalyzing the excision of the intervening sequence flanked by the pLox sites. Thus, the one or more genes (e.g., one(s) that promote differentiation) and the TRE and Cre recombinase expression cassette are removed, resulting in footprint-free excision of the genes of interest. Such constructs can be used for inducing transdifferentiation of cells into a different cell type.

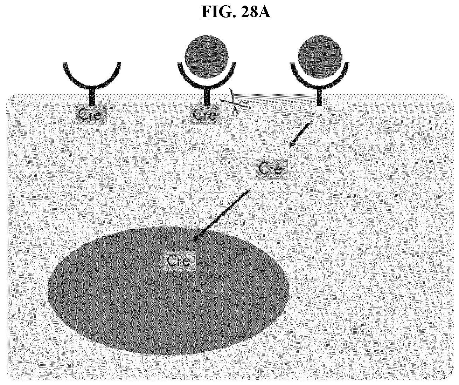

**FIG. 28A** shows one embodiment of a synthetic receptor for modulating gene expression (e.g., activating and/or inactivating target ORFs). This system is modeled after the "synthetic notch receptor" system described in Morsut et al., Engineering Customized Cell Sensing and Response Behaviors Using Synthetic Notch Receptors. Cell. 2016 Feb 11;164(4):780-91. In this system, a receptor is engineered to comprise an extracellular component that is the same as endogenous Notch receptors (which signal by cleaving intracellular domains upon binding a ligand, such as the protein Delta). Accordingly, the intracellular domain can be replaced by an enzyme, such as Cre recombinase. When a ligand is added to the cell, the engineered Notch receptor becomes activated, and the intracellular domain is cleaved, which in this case releases Cre into the cytoplasm. Cre enters the nucleus by passive diffusion (or has a nuclear localization sequence engineered into the protein, facilitating entry into the nucleus). There, it induces recombination of loxP sites, as described herein (e.g., **FIGs. 12-15****).** Such constructs can be used for inducing transdifferentiation of cells into a different cell type.

The model shown in **FIG. 28A** represents an irreversible switch, by which Cre is released from the cell membrane and into the nucleus to induce recombination events. Such events (including the switch from maintenance of pluripotency to differentiation) are described herein. Cre delivery has the potential to affect various cellular functions or properties, such as proliferation, adhesion, differentiation, migration, and other cell properties. In various embodiments, this switch allows the transition from a proliferative state to the activation of genes that induce differentiation (into myocytes, adipocytes, etc). An important benefit of this system is that it does not require activation of genes to enable Cre to function; instead, the enzyme is constitutively expressed and localized as reservoirs at the cell surface. Such constructs can be used for inducing transdifferentiation of cells into a different cell type.

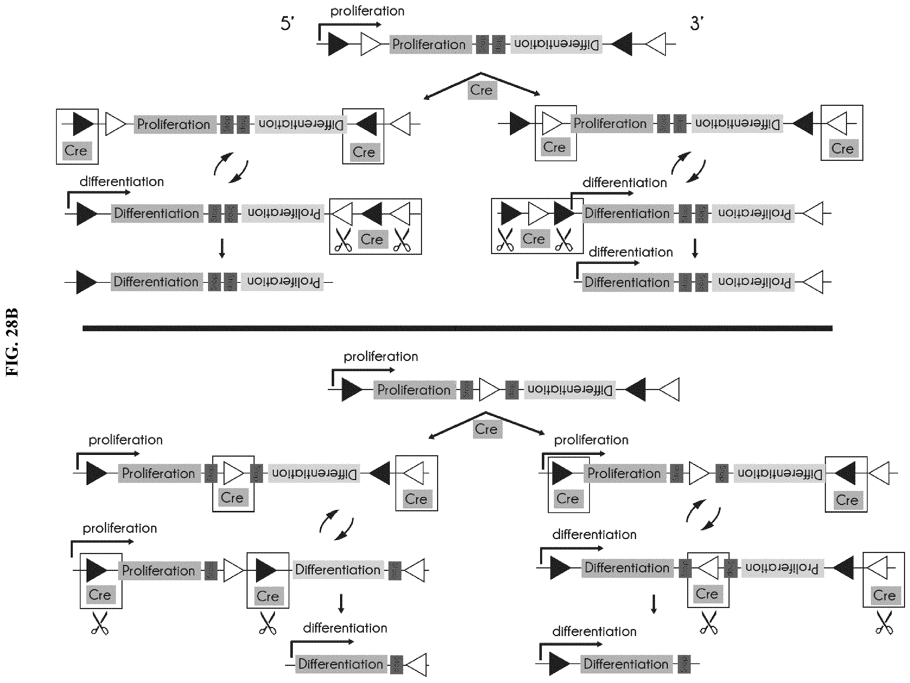

**FIG. 28B** shows additional strategies for switching between gene sets (e.g., inactivating a pluripotency gene set and activating a differentiation gene set). These strategies can implement site-specific recombinase (SSR) systems in combination with inducible gene expression systems (e.g., tetracycline/doxycycline inducible systems). The SSR/inducible combination is described in Zhang et al. Conditional gene manipulation: Cre-ating a new biological era. J Zhejiang Univ-Sci B (Biomed & Biotechnol) 2012 13(7):511-524. In some cases, this SSR/inducible system is used as a switch between pluripotency and differentiation for creating ex vivo meat. Such constructs can be used for inducing transdifferentiation of cells into a different cell type.

As shown in **FIG. 28B****,** the triangles represent lox sequences (black is loxP and white is lox5171, although other sequences may be used for this purpose). When the triangles are aligned, a recombination event (catalyzed by the Cre enzyme) leads to excision / deletion of the sequence between them. When the triangles (representing defined DNA sequences) are facing each other, a recombination event leads to stochastic inversion (flipping back and forth) of the intervening sequence. For the purpose of this illustration, proliferation is described generally (and can encompass genes such as members of the cyclin family, cyclin-dependent kinases, cell cycle inhibitors such as p27kip, TERT, and others). Proliferation can also be substituted with "Pluripotency," genes (such as the Yamanaka factors for generating iPSCs), as detailed in other gene switch mechanisms described herein (e.g., **FIGs. 12-15****).** Likewise, differentiation can encompass various genes (such as MyoD in the case of myocytes). Other genes may be used in the same way, stimulating differentiation to any lineage. Such constructs can be used for inducing transdifferentiation of cells into a different cell type.

Two representative scenarios are shown in **FIG. 28B****.** In the upper panel (1), when Cre is added, it first induces recombination in either the black pair or white pair of triangles. When the initial event involves the black triangles (loxP) as shown in the left scenario, this induces inversion of the intervening DNA sequence, which puts two white triangles (lox5171) in parallel; this permits the Cre enzyme to then excise the intervening sequence. The result is a switch from proliferation genes to differentiation genes. In the right scenario, Cre first acts upon the white triangles (lox5171). This induces an inversion that then places the black triangles (loxP) in parallel, permitting Cre to then excise the intervening sequence. Again, the result is a switch from transcription of proliferation genes to transcription of differentiation genes. Such constructs can be used for inducing transdifferentiation of cells into a different cell type.

In the lower panel (2), a different schematic yields a switch from proliferation to differentiation. The lox sequences are placed such that when Cre first induces recombination between the white triangles (lox5171), an inversion event occurs. The inversion event puts the two black triangles in parallel (loxP), enabling Cre to excise the proliferation genes and activate differentiation. The inverse occurs in the right panel in which Cre first induces recombination between the black triangles. Such constructs can be used for inducing transdifferentiation of cells into a different cell type.

Unlike other systems (which require the activation of one gene program, followed by a second step of gene activation), the processes described herein in **FIG. 28B** are unique in that they induce a complete switch from one gene set to another, using a single input (Cre), with very high efficiency. This approach provides an improvement in the method of generating cultured food products that can simplify the process and/or reduce required inputs, especially at large-scale production. At scale, the processes disclosed herein represent a significant improvement in process simplification, and a reduction in requisite inputs.

The inducible systems described herein are not limited to Tet and/or Cre recombinase based systems. Other embodiments of a system utilizing inducible recombinase expression to excise one or more genes of interest are contemplated. For example, the Flp-FRT system utilizes Flp (flippase) recombinase to excise DNA flanked by FRT (flippase recognition target) sequences. Such systems can be used for inducing transdifferentiation of cells into a different cell type.

Described herein are systems and methods utilizing transposons to genetically engineer or modify cells. Transposons are naturally-occurring genetic sequences that facilitate the transposition of genes (excision and subsequent re-integration of genes into a different region of the genome). This phenomenon was initially characterized in the context of maize genes (colloquially termed "jumping genes") and has since been discovered in other species. Transposon sequences have previously been described for the purpose of integrating transgenes into a host genome; the best-characterized ones to date are Tol2, Piggybac, and Sleeping Beauty. Transposons typically contain genetic sequences that flank the gene, and require an enzyme (transposase) to facilitate both integration and excision. Piggybac transposase has previously been mutated to enhance either its ability to integrate genes within a host genome, or its ability to excise genes. Excision has also been characterized as completely "footprint free," meaning that no residual non-native genetic material remains upon excision.

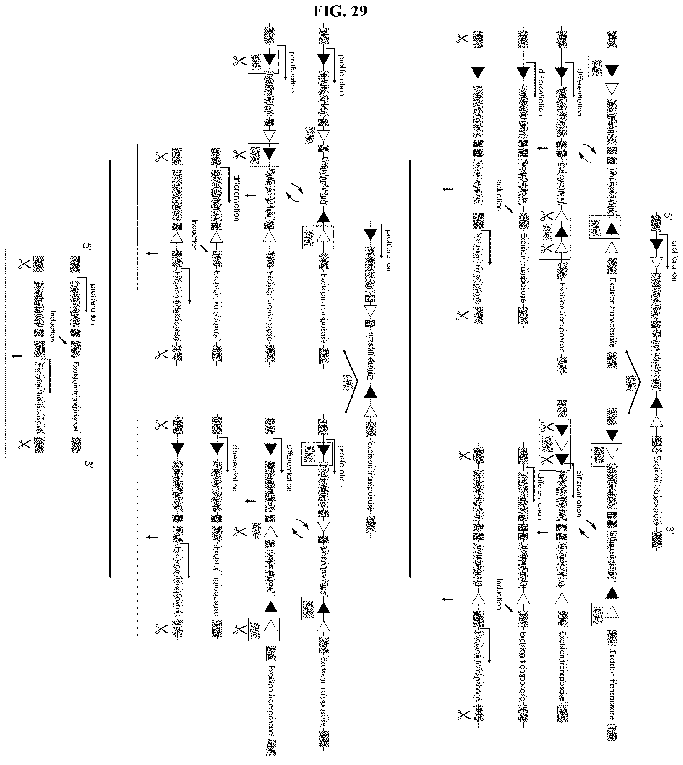

**FIG. 29** shows an embodiment of a method of transposon-mediated genetic self-excision for purpose of footprint-free gene editing. In this example, pLox sequences of the same color in parallel configurations undergo an excision event (removing the sequences between these triangles) in the presence of Cre enzyme. In contrast, pLox sequences in anti-parallel configurations (similarly-colored triangles that face each other) undergo recombination events, whereby the DNA sequences between the triangles flip back and forth in a stochastic manner. These systems are designed such that these recombination flip events occasionally position other Cre sequences (triangles of the color not initially acted upon by Cre) align in a parallel configuration. This alignment permits an excision event (also catalyzed by Cre enzyme) that irreversibly maintains the intervening DNA in a flipped conformation (e.g., differentiation, versus the initial proliferation state). As shown in **FIG. 29****,** the transgenes are flanked by a transposon flanking sequence (TFS in the legend) permitting excision of the entire transgene cassette.

The excision transposase gene is under the control of an inducible promoter (Pro in the figure). This may be a classically-described inducible promoter (such as tetracycline-inducible with a tetracycline-responsive element (TRE], an estrogen receptor, or similar system), or it may be a promoter corresponding to a reporter of a differentiated cell lineage. In the latter case, this reporter may be used to excise the entire transgene cassette once a cell has undergone terminal differentiation and maturation. For example, the excision transposase may be under the control of a MyoD promoter; in this case, a cell undergoing differentiation to skeletal muscle (myocyte) will express MyoD when differentiation is complete. At that time, MyoD will activate the MyoD promoter, thus resulting in expression of the excision transposase and subsequent excision of the entire transgene cassette. In this way, terminal differentiation will result in a penultimate genomic rearrangement, whereby all transgenes are excised in a "footprint-free" manner. The same principle may be applied to adipogenesis, vasculogenesis, and differentiation into any other lineage. In some cases, this system is used in simpler configurations, such as the use of a single gene (proliferation or differentiation), in an excisable transgene cassette **(****FIG. 29** lower panel).

The combination of induced expression and gene excision to maintain cells in an undifferentiated state of self-renewal may face a technical problem of promoter leakiness or a basal expression level. For example, leakiness in the promoter could result in some expression of the recombinase, which then excises the pluripotent stem cell factors before the inducing agent is added. However, a key advantage of utilizing this system for purposes of cultured food production as described herein is that those cells that experience leakiness will likely lose their self-renewal phenotype and become outcompeted by the cells that maintain tighter control of recombinase expression. Thus, when self-renewing (e.g. pluripotent or multipotent) cell cultures are scaled up to produce commercial quantities of cultured food products, most or all of the cultured cells in the population should retain their undifferentiated and self-renewing properties when the basal expression level is sufficiently low. Modified cells may be clonally selected to identify cell lines that have strong repression of recombinase expression in the absence of an inducing agent. In some cases, the inducing agent is added to induce differentiation (and/or other desired properties) shortly before the cells are harvested to produce the meat product for human consumption.

Disclosed herein are systems and methods for modulating cell proliferation and/or differentiation in the context of food production that utilize non-integrating approaches. Some approaches require the stable integration of transgenes into the genome. Alternatively, non-integrating DNA are also consistent with the present disclosure. Examples of non-integrating DNA include episomes (extrachromosomal) or circular DNA sequences (e.g., plasmids) that are capable of independent replication and transcription.

In some cases, microRNA (miRNA) is used to modulate proliferation and differentiation. For example, miRNA that serves to inhibit anti-proliferation genes and/or anti-differentiation genes (1) can be delivered to cells directly (with conventional lipofection / nucleofection methods ), (2) can be included as genes within the excisable transgene cassettes described herein, or (3) utilize DNA plasmids containing miRNA sequences that are embedded within cellular scaffolds for cells to internalize (and subsequently transcribe into miRNA) as they grow within / remodel these scaffolds.

Modified RNA can be used to express proteins that induce proliferation and differentiation. Modified RNA is synthetic RNA using several chemical modifications (e.g., bases are modified, such as pseudo-uridine in place of uridine and 6-methyladenine in place of adenine, and the RNA cap structure is often also modified for enhanced stability). Modified RNA can be synthesized using these chemically modified bases, and subsequently delivered to cells directly using conventional lipofection / nucleofection methods, or it may be embedded within cellular scaffolds for cells to internalize as they grow within / remodel these scaffolds.

In certain approaches, synthetic exosomes are used for the purpose of RNA, small molecule, and/or protein (e.g., growth factor) delivery to induce / enhance cellular proliferation and differentiation.

Modulating cell proliferation and/or differention using transcription factors are also consistent with the present disclosure. In some cases, cell proliferation and/or differention is affected through modulation of DNA methylation to control gene expression. DNA sequences contain varying degrees of accessibility to interactions with proteins, and to resultant transcription / gene expression; this process is largely mediated by DNA methylation. In this way, there are commonly regions of DNA that are found in a "closed" or "open" conformation, corresponding to heterochromatin and euchromatin, respectively. Inaccessible (closed conformation) DNA is often refractory to transcription, even when protein transcription factors are present to initiate transcription and gene expression. For this reason, alteration of the chromatin landscape either using small molecule drugs (e.g., Azacitadine [5-AZA], RG108, valproic acid, tranylcypromine, trichostatin A, sodium butyrate, suberanilohydroxamic acid, and others) or by the use of "pioneer transcription factors" (protein transcription factors that affect DNA methylation status and therefore enhance the ability of other transcription factors to subsequently interact with DNA) are consistent with the methods of the present disclosure. One example of a protein transcription factor is ASCL1, a gene not typically involved with myogenesis or adipogenesis, but which appears to enhance the progression toward myogenic lineages.

### Inducing lipid accumulation or steatosis

In some cases, the systems, methods, and compositions disclosed herein provide for inducing lipid accumulation or steatosis. Oftentimes, lipid accumulation or steatosis is induced in a population of cells for purposes of producing a cell cultured food product with increased lipid content. As used herein, steatosis is a pathologic state characterized by the abnormal retention of lipids within a cell. The excess lipids accumulate in vesicles that displace the cytoplasm. Macrovesicular steatosis describes when the vesicles are large enough to displace or distort the nucleus, while microvesicular steatosis lacks this phenotype. For example, **FIG. 4A** illustrates a process including the generation of steatotic hepatocytes **413** by genetic intervention and/or addition of exogenous compounds **409.**

In some aspects, lipid accumulation and/or steatosis is induced in a population of cells by genetic manipulation. For example, some methods disclosed herein provide for the preparation of foie gras comprising cultured avian liver tissue. Some such methods comprise: a) obtaining a population of avian derived cells capable of self-renewal; b) differentiating the population of avian derived cells into hepatocytes; and c) inducing steatosis in the hepatocytes to generate cultured avian liver tissue having high lipid content; and d) preparing the cultured avian liver tissue as foie gras. In certain cases, non-hepatocytes are induced to undergo lipid accumulation. Some methods produce cultured cells having high lipid accumulation for human consumption. One example of such a method comprises: a) culturing a population of cells; b) inducing differentiation within the population of cells; c) inducing high lipid accumulation within the population of cells; and d) processing the population of cells for human consumption.

Steatosis and/or lipid accumulation is accomplished by manipulating lipid metabolism. For example, the genetic profiles of hepatocytes that have undergone steatosis has been previously characterized by Chiappini et al., Exploration of global gene expression in human liver steatosis by high-density oligonucleotide microarray. Lab Invest. 2006 Feb;86(2):154-65. Affected intracellular signaling pathways in steatotic hepatocytes involve lipid metabolism, and lead to the accumulation of lipid droplets within the cytoplasm of hepatocytes. In some cases, the systems and methods described herein provide for reliable, high-efficiency induction of steatosis in a population of cells. Steatosis is often induced in a population of liver cells or hepatocytes. Sometimes, steatosis is induced by up or downregulation of genes involved in hepatic lipid metabolism. For example, in some cases, p53 depletion and/or upregulation of p63 (e.g. overexpression of N-terminal transactivation domain TAp63) induces lipid accumulation as described in Porteiro et al., Hepatic p63 regulates steatosis via IKK beta/ER stress. Nature Communications. 2017 May;8:15111. Genetic modification of cells such as hepatocytes may be carried out using the various protocols discussed (e.g. inducible expression of genes involved in steatosis). In some instances, steatosis and/or lipid accumulation is induced by manipulating at least one of lipid metabolism pathway(s) and ER pathway(s) involved in ER stress. Sometimes, steatosis or lipid accumulation is induced in hepatocytes or liver cells. Alternatively, in other cases, steatosis or lipid accumulation is induced in non-hepatocyte cells such as, for example, myocytes or skeletal muscle cells. Sometimes, steatosis is induced in fish myocytes such as salmon myocytes. In certain instances, steatosis or lipid accumulation is induced in non-hepatocyte organ cells such as, for example, kidney cells. On occasion, steatosis or lipid accumulation is induced in a pluripotent cell population or an adult progenitor cell population that is used as an intermediate cell line for expansion into a differentiated cell population. In these scenarios, steatosis or lipid accumulation is induced earlier during the production process before the population of cells is differentiated. In some cases, high lipid accumulation is induced in cells.

Conversely, in certain instances, steatosis is induced by disrupting lipid metabolism pathways without requiring genetic manipulation. For example, certain exogenous compounds are capable of inducing steatosis in hepatocytes grown *in vitro* or *ex vivo.* These exogenous compounds include toxins such as alcohols, and lipids such as fatty acids. In some cases, culturing cells in a media formulation having a high concentration of at least one lipid induces steatosis. Cells are cultured in a lipid rich media formulation to induce steatosis, in various embodiments. The cells cultured in the lipid rich media sometimes comprise a population of differentiated cells such as, for example, avian hepatocytes or fish myocytes. In certain cases, the cells cultured in the lipid rich media are pluripotent stem cells such as embryonic stem cells or induced pluripotent stem cells. Alternatively, the cells cultured in the lipid rich media are multipotent stem cells such as adult progenitor cells, in certain instances. Sometimes, cells are cultured in a lipid rich media with at least one lipid type selected from saturated fatty acids, mono-unsaturated fatty acids, poly-unsaturated fatty acids, and trans-fatty acids. Examples of lipids include palmitic acid, oleic acid, docosahexaenoic acid, stearic acid, linoleic acid, linolenic acid, arachidonic acid, and eicosapentaenoic acid. In some cases, the culture media is supplemented with linoleic acid, oleic acid, or a combination thereof to induce lipid accumulation or steatosis within a population of cells cultured in the media. In some cases, the culture media is supplemented with a lipid at a concentration of at least about 1mM, about 2mM, about 3mM, about 4mM, about 5mM, about 6mM, about 7mM, about 8mM, about 9mM, about 10mM, about 15mM, about or 20mM. Sometimes, the culture media is supplemented with a lipid at a concentration of no more than about 1mM, about 2mM, about 3mM, about 4mM, about 5mM, about 6mM, about 7mM, about 8mM, about 9mM, about 10mM, about 15mM, or about 20mM. In various embodiments, the culture media is supplemented with a lipid at a concentration of at least about 1 µM, about 2 µM, about 3 µM, about 4 µM, about 5 µM, about 6 µM, about 7 µM, about 8 µM, about 9 µM, about 10 µM, about 20 µM, about 30 µM, about 40 µM, about 50 µM, about 60 µM, about 70 µM, about 80 µM, about 90 µM, about 100 µM, about 200 µM, about 300 µM, about 400 µM, about 500 µM, about 600 µM, about 700 µM, about 800 µM, about 900 µM, or about 1000 µM. In certain instances, the culture media is supplemented with a lipid at a concentration of no more than about 1 µM, about 2 µM, about 3 µM, about 4 µM, about 5 µM, about 6 µM, about 7 µM, about 8 µM, about 9 µM, about 10 µM, about 20 µM, about 30 µM, about 40 µM, about 50 µM, about 60 µM, about 70 µM, about 80 µM, about 90 µM, about 100 µM, about 200 µM, about 300 µM, about 400 µM, about 500 µM, about 600 µM, about 700 µM, about 800 µM, about 900 µM, or about 1000 µM. Oftentimes, the cell culture media comprises a lipid concentration of about 1 mM to about 20 mM or of about 1 µM to about 1000 µM. The cell culture media often comprises a lipid concentration of at least about 1 µM. Typically, the cell culture media comprises a lipid concentration of at most about 20 mM.

In some cases, the culture media is supplemented with at least one culture media supplement such as IBMX (a methyl xanthine), rosiglitazone (a thiazolidinedione), increased glucose concentration, and/or a corticosteroid such as dexamethasone. Other examples of thiazolidinediones that can be used to supplement culture media include pioglitazone, lobeglitazone, ciglitazone, darglitazone, englitazone, netoglitazone, rivoglitazone, troglitazone, and balaglitazone. In certain instances, the culture media is supplemented with at least one thiazolidinedione selected from the group consisting of pioglitazone, lobeglitazone, ciglitazone, darglitazone, englitazone, netoglitazone, rivoglitazone, troglitazone, and balaglitazone. In one example, the thiazolidinedione is rosiglitazone. The culture media supplements described herein can be added to the culture media at various concentrations including the full range of concentrations described for lipid concentrations. For example, a culture media supplement can be added to the media at a concentration of at least about 1 µM, about 2 µM, about 3 µM, about 4 µM, about 5 µM, about 6 µM, about 7 µM, about 8 µM, about 9 µM, about 10 µM, about 20 µM, about 30 µM, about 40 µM, about 50 µM, about 60 µM, about 70 µM, about 80 µM, about 90 µM, about 100 µM, about 200 µM, about 300 µM, about 400 µM, about 500 µM, about 600 µM, about 700 µM, about 800 µM, about 900 µM, about 1mM, about 2mM, about 3mM, about 4mM, about 5mM, about 6mM, about 7mM, about 8mM, about 9mM, about 10mM, about 15mM, or about 20mM. In some cases, a culture media supplement can be added to the media at a concentration of no more than about 1 µM, about 2 µM, about 3 µM, about 4 µM, about 5 µM, about 6 µM, about 7 µM, about 8 µM, about 9 µM, about 10 µM, about 20 µM, about 30 µM, about 40 µM, about 50 µM, about 60 µM, about 70 µM, about 80 µM, about 90 µM, about 100 µM, about 200 µM, about 300 µM, about 400 µM, about 500 µM, about 600 µM, about 700 µM, about 800 µM, about 900 µM, about 1mM, about 2mM, about 3mM, about 4mM, about 5mM, about 6mM, about 7mM, about 8mM, about 9mM, about 10mM, about 15mM, or about 20mM.

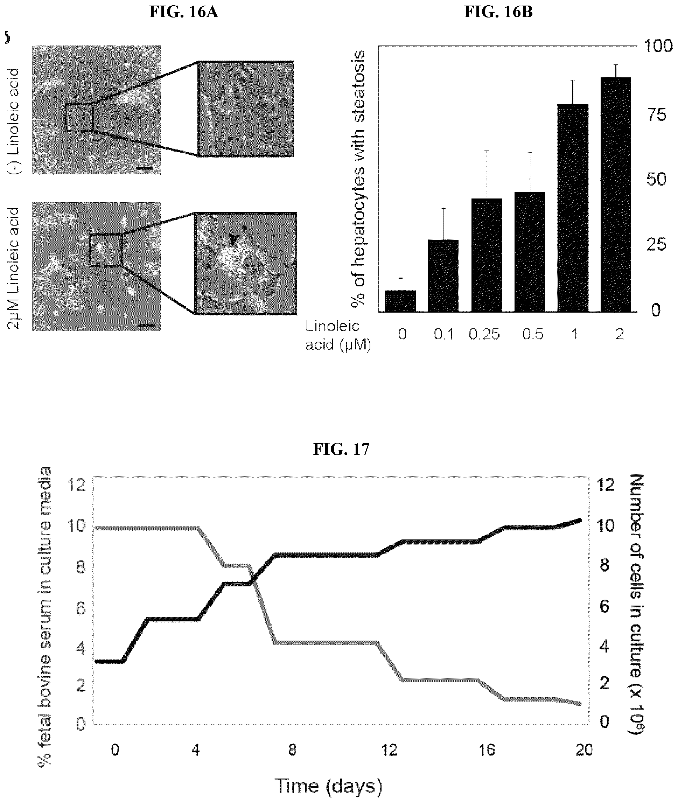

**FIG. 16A** shows successful induction of steatosis (accumulation of intracellular lipid-containing vesicles; arrowhead) in duck hepatocytes upon incubation with 2 µM linoleic acid (bottom panel) compared to the untreated control (top panel). **FIG. 16B** shows a dose response curve correlating the percentage of steatotic hepatocytes with the concentration of linoleic acid. Similar results have been achieved with oleic acid. In some cases, the protocols were augmented by incubating the hepatocytes with IBMX (a methyl xanthine), rosiglitazone (a thiazolidinedione), increased glucose concentration, or other fatty acid species, and corticosteroids such as dexamethasone.

In some cases, the cell culture media comprises a lipid concentration (or other media supplement described herein) of at least about 0.1 µM, about 0.2 µM, about 0.3 µM, about 0.4 µM, about 0.5 µM, about 0.6 µM, about 0.7 µM, about 0.8 µM, about 0.9 µM, about 1.0 µM, about 1.1 µM, about 1.2 µM, about 1.3 µM, about 1.4 µM, about 1.5 µM, about 1.6 µM, about 1.7 µM, about 1.8 µM, about 1.9 µM, about 2 µM, about 3 µM, about 4 µM, about 5 µM, about 6 µM, about 7 µM, about 8 µM, about 9 µM, about 10 µM, about 15 µM, or about 20 µM. In some cases, the cell culture media comprises a lipid concentration of at most about 0.1 µM, about 0.2 µM, about 0.3 µM, about 0.4 µM, about 0.5 µM, about 0.6 µM, about 0.7 µM, about 0.8 µM, about 0.9 µM, about 1.0 µM, about 1.1 µM, about 1.2 µM, about 1.3 µM, about 1.4 µM, about 1.5 µM, about 1.6 µM, about 1.7 µM, about 1.8 µM, about 1.9 µM, about 2 µM, about 3 µM, about 4 µM, about 5 µM, about 6 µM, about 7 µM, about 8 µM, about 9 µM, about 10 µM, about 15 µM, or about 20 µM. In some cases, the cell culture media comprises a lipid concentration of about 1 µM to about 2 µM, about 1 µM to about 3 µM, about 1 µM to about 4 µM, about 1 µM to about 5 µM, about 1 µM to about 6 µM, about 1 µM to about 7 µM, about 1 µM to about 8 µM, about 1 µM to about 9 µM, about 1 µM to about 10 µM, about 1 µM to about 15 µM, about 1 µM to about 20 µM, about 2 µM to about 3 µM, about 2 µM to about 4 µM, about 2 µM to about 5 µM, about 2 µM to about 6 µM, about 2 µM to about 7 µM, about 2 µM to about 8 µM, about 2 µM to about 9 µM, about 2 µM to about 10 µM, about 2 µM to about 15 µM, about 2 µM to about 20 µM, about 3 µM to about 4 µM, about 3 µM to about 5 µM, about 3 µM to about 6 µM, about 3 µM to about 7 µM, about 3 µM to about 8 µM, about 3 µM to about 9 µM, about 3 µM to about 10 µM, about 3 µM to about 15 µM, about 3 µM to about 20 µM, about 4 µM to about 5 µM, about 4 µM to about 6 µM, about 4 µM to about 7 µM, about 4 µM to about 8 µM, about 4 µM to about 9 µM, about 4 µM to about 10 µM, about 4 µM to about 15 µM, about 4 µM to about 20 µM, about 5 µM to about 6 µM, about 5 µM to about 7 µM, about 5 µM to about 8 µM, about 5 µM to about 9 µM, about 5 µM to about 10 µM, about 5 µM to about 15 µM, about 5 µM to about 20 µM, about 6 µM to about 7 µM, about 6 µM to about 8 µM, about 6 µM to about 9 µM, about 6 µM to about 10 µM, about 6 µM to about 15 µM, about 6 µM to about 20 µM, about 7 µM to about 8 µM, about 7 µM to about 9 µM, about 7 µM to about 10 µM, about 7 µM to about 15 µM, about 7 µM to about 20 µM, about 8 µM to about 9 µM, about 8 µM to about 10 µM, about 8 µM to about 15 µM, about 8 µM to about 20 µM, about 9 µM to about 10 µM, about 9 µM to about 15 µM, about 9 µM to about 20 µM, about 10 µM to about 15 µM, about 10 µM to about 20 µM, or about 15 µM to about 20 µM.

In some cases, the cell culture media comprises a lipid concentration (or other media supplement described herein) of at least about 0.1 mM, about 0.2 mM, about 0.3 mM, about 0.4 mM, about 0.5 mM, about 0.6 mM, about 0.7 mM, about 0.8 mM, about 0.9 mM, about 1.0 mM, about 1.1 mM, about 1.2 mM, about 1.3 mM, about 1.4 mM, about 1.5 mM, about 1.6 mM, about 1.7 mM, about 1.8 mM, about 1.9 mM, about 2 mM, about 3 mM, about 4 mM, about 5 mM, about 6 mM, about 7 mM, about 8 mM, about 9 mM, about 10 mM, about 15 mM, or about 20 mM. In some cases, the cell culture media comprises a lipid concentration of at most about 0.1 mM, about 0.2 mM, about 0.3 mM, about 0.4 mM, about 0.5 mM, about 0.6 mM, about 0.7 mM, about 0.8 mM, about 0.9 mM, about 1.0 mM, about 1.1 mM, about 1.2 mM, about 1.3 mM, about 1.4 mM, about 1.5 mM, about 1.6 mM, about 1.7 mM, about 1.8 mM, about 1.9 mM, about 2 mM, about 3 mM, about 4 mM, about 5 mM, about 6 mM, about 7 mM, about 8 mM, about 9 mM, about 10 mM, about 15 mM, or about 20 mM. In some cases, the cell culture media comprises a lipid concentration of about 1 mM to about 2 mM, about 1 mM to about 3 mM, about 1 mM to about 4 mM, about 1 mM to about 5 mM, about 1 mM to about 6 mM, about 1 mM to about 7 mM, about 1 mM to about 8 mM, about 1 mM to about 9 mM, about 1 mM to about 10 mM, about 1 mM to about 15 mM, about 1 mM to about 20 mM, about 2 mM to about 3 mM, about 2 mM to about 4 mM, about 2 mM to about 5 mM, about 2 mM to about 6 mM, about 2 mM to about 7 mM, about 2 mM to about 8 mM, about 2 mM to about 9 mM, about 2 mM to about 10 mM, about 2 mM to about 15 mM, about 2 mM to about 20 mM, about 3 mM to about 4 mM, about 3 mM to about 5 mM, about 3 mM to about 6 mM, about 3 mM to about 7 mM, about 3 mM to about 8 mM, about 3 mM to about 9 mM, about 3 mM to about 10 mM, about 3 mM to about 15 mM, about 3 mM to about 20 mM, about 4 mM to about 5 mM, about 4 mM to about 6 mM, about 4 mM to about 7 mM, about 4 mM to about 8 mM, about 4 mM to about 9 mM, about 4 mM to about 10 mM, about 4 mM to about 15 mM, about 4 mM to about 20 mM, about 5 mM to about 6 mM, about 5 mM to about 7 mM, about 5 mM to about 8 mM, about 5 mM to about 9 mM, about 5 mM to about 10 mM, about 5 mM to about 15 mM, about 5 mM to about 20 mM, about 6 mM to about 7 mM, about 6 mM to about 8 mM, about 6 mM to about 9 mM, about 6 mM to about 10 mM, about 6 mM to about 15 mM, about 6 mM to about 20 mM, about 7 mM to about 8 mM, about 7 mM to about 9 mM, about 7 mM to about 10 mM, about 7 mM to about 15 mM, about 7 mM to about 20 mM, about 8 mM to about 9 mM, about 8 mM to about 10 mM, about 8 mM to about 15 mM, about 8 mM to about 20 mM, about 9 mM to about 10 mM, about 9 mM to about 15 mM, about 9 mM to about 20 mM, about 10 mM to about 15 mM, about 10 mM to about 20 mM, or about 15 mM to about 20 mM.

In some cases, cells are cultured in a high lipid concentration for a period of time to induce steatosis. The length of time during which the cells are exposed to high lipid concentrations will vary depending on the cell type, size of the cell population, age of the cell population, number of passages, any genetic modification or manipulation of the cells, the type and components of the culture media, desired amount of lipid accumulation or steatosis, or any combination thereof. For example, certain cell types will intake exogenous lipids in the culture media at a slower rate than other cell types, and thus require a longer incubation period in a lipid rich media to induce the desired amount of steatosis. In various cases, cells are cultured in a cell culture media comprising at least one lipid for a period of time. Sometimes, cells are cultured in a culture media having a high lipid concentration for at least a certain period of time. In many cases, cells are cultured in a culture media having a high lipid concentration for about 1 day to about 20 days. Cells are often cultured in a culture media having a high lipid concentration for at least about 1 day. Typically, cells are cultured in a culture media having a high lipid concentration for at most about 20 days.

In certain instances, cells are cultured in a culture media having a high lipid (or other media supplement described herein) concentration for about 1 day to about 2 days, about 1 day to about 3 days, about 1 day to about 4 days, about 1 day to about 5 days, about 1 day to about 6 days, about 1 day to about 7 days, about 1 day to about 8 days, about 1 day to about 9 days, about 1 day to about 10 days, about 1 day to about 15 days, about 1 day to about 20 days, about 2 days to about 3 days, about 2 days to about 4 days, about 2 days to about 5 days, about 2 days to about 6 days, about 2 days to about 7 days, about 2 days to about 8 days, about 2 days to about 9 days, about 2 days to about 10 days, about 2 days to about 15 days, about 2 days to about 20 days, about 3 days to about 4 days, about 3 days to about 5 days, about 3 days to about 6 days, about 3 days to about 7 days, about 3 days to about 8 days, about 3 days to about 9 days, about 3 days to about 10 days, about 3 days to about 15 days, about 3 days to about 20 days, about 4 days to about 5 days, about 4 days to about 6 days, about 4 days to about 7 days, about 4 days to about 8 days, about 4 days to about 9 days, about 4 days to about 10 days, about 4 days to about 15 days, about 4 days to about 20 days, about 5 days to about 6 days, about 5 days to about 7 days, about 5 days to about 8 days, about 5 days to about 9 days, about 5 days to about 10 days, about 5 days to about 15 days, about 5 days to about 20 days, about 6 days to about 7 days, about 6 days to about 8 days, about 6 days to about 9 days, about 6 days to about 10 days, about 6 days to about 15 days, about 6 days to about 20 days, about 7 days to about 8 days, about 7 days to about 9 days, about 7 days to about 10 days, about 7 days to about 15 days, about 7 days to about 20 days, about 8 days to about 9 days, about 8 days to about 10 days, about 8 days to about 15 days, about 8 days to about 20 days, about 9 days to about 10 days, about 9 days to about 15 days, about 9 days to about 20 days, about 10 days to about 15 days, about 10 days to about 20 days, or about 15 days to about 20 days.

### Media formulations

Provided herein are systems and methods utilizing at least one media formulation that enables cultured food production. In some cases, the media formulation does not require the use of serum such as fetal bovine serum. Sometimes, the media formulation does not require one or more other supplements that are used in certain cell culture media. Cell culture media is generally divided into two categories: serum media and serum-free media. Conventional media formulations often utilize fetal bovine serum and other supplements that are cost prohibitive for large scale cultured food production. Serum (e.g. fetal bovine serum) tends to vary between batches since it is produced from animals. For example, fetal bovine serum (FBS) is extracted from the blood of calf fetuses and tends to have batch-to-batch variation in composition. In addition, the use of serum can create the possibility of contamination by viruses, mycoplasma, prions, toxins, and other undesirables present in the animal from which the serum is extracted. Finally, serum is cost prohibitive and requires the raising of livestock, which is contrary to some of the goals of providing cultured food products. The use of serum-free media, however, bypasses these challenges. Substitutes or supplements to serum are used in various media formulations for producing the cultured food products described herein. Serum substitutes or supplements are derived from non-livestock sources (e.g. not derived from cow fetuses). Examples include mammalian cell overexpression systems and transgene expression in yeast, larger fungi (e.g. mushrooms), bacteria, algae, or insect cell (e.g. baculovirus) systems. An exemplary embodiment is a mushroom-based system for producing a serum substitute for serum-free media formulation(s) such as described in Benjaminson et al. In vitro edible muscle protein production system (mpps): Stage 1, fish. Acta Astronautica (2002): 51(12), 879-889. Sometimes, the systems, methods, and compositions described herein comprise generating or obtaining at least one cell line suitable for being cultured using a mushroom-based culture media formulation. In some cases, a hepatocyte cell line, a pre-adipocyte cell line, or a satellite cell line is conditioned or modified to enable culturing in a mushroom-based serum-free media formulation.

In some cases, a media formulation comprises a natural media. Oftentimes, a media formulation comprises a synthetic media or a modification thereof. Examples of synthetic media include Minimum Essential Media (MEM), Essential 8 Media, Basal Medium Eagle (BME), Ham's F12, Ham's F-10, Fischer's Medium, CMRL-1066 Medium, Click's Medium, Medium 199, Dulbecco's Modified Eagle's Media (DMEM), RPMI-1640, L-15 medium, McCoy's 5A Modified Medium, William's Medium E, and Iscove's Modified Dulbecco's Medium (IMDM).

In some cases, the media formulation is modified for culturing embryonic stem cells, induced pluripotent stem cells, embryonic germ cells, differentiated cells (e.g. hepatocytes or myocytes), immortalized differentiated cells, or nascent hepatic stem cells. In certain instances, a media formulation for culturing isolated duck stem cell lines as described in WO2008129058A1 is used with one or more modifications. For example, interleukin-6 and Stem Cell Factor are optionally eliminated from the media formulation, in some cases. Sometimes, the media formulation is modified from WO2008129058A1. The media formulation usually enables the proliferation and/or maintenance of self-renewal ability of stem cells without requiring feeder cells. For example, Essential 8 Medium provides the most important components for maintaining pluripotent stem cells in a feeder cell-free environment. A feeder-free culture environment enhances the large-scale production of cultured food products because it avoids having to constantly re-seed feeder cell layers in order to grow pluripotent stem cells. Oftentimes, multiple media formulations are used during the culturing of a population of cells. In some cases, an initial population of cells with self-renewal ability is cultured with a media formulation that maintains the self-renewal ability such as, for example, maintaining a population of embryonic stem cells in an un-differentiated state. Next, sometimes, differentiation is induced in the population of cells having self-renewal ability. As an example, the embryonic stem cells are induced to differentiate into hepatocytes. This differentiation step sometimes requires a differentiation media formulation. For example, in some instances, certain differentiation factor(s) are added and/or factor(s) required for maintaining the self-renewal ability are removed in the differentiation media. Moreover, when differentiation in the population of cells leads to the generation of hepatocytes, there is often an additional step of inducing steatosis or lipid accumulation in the hepatocytes. In some cases, steatosis is induced at least in part by the use of a steatotic media formulation. For example, a steatotic media formulation sometimes comprises a lipid concentration of at least a certain concentration as described elsewhere herein.

In some cases, a media formulation comprises at least one nutrient or nutritional supplement for enhancing the nutritional content of the finished food product. A nutrient is a macronutrient or a micronutrient. Macronutrients are nutrients that are needed in large quantities and include proteins, fats, and carbohydrates. Micro-nutrients are required in small quantities and include vitamins, minerals, some amino acids, and certain compounds such as, for example, flavonoids. In certain instances, at least one nutrient is added to a media formulation for intake by a population of cultured cells. As an example, steatotic hepatocytes used to produce foie gras typically have high lipid accumulation inside of the cytoplasm. Culturing the hepatocytes in a media formulation having a specific lipid composition (e.g. omega-3 fatty acids) may induce the resulting steatotic hepatocytes to have a modified lipid profile partially reflecting the lipid composition of the culture media. Certain methods provide for production of cultured tissue having increased nutritional content for human consumption. For example, some such methods comprise: a) culturing a population of cells in a culture medium having at least one nutritional supplement; b) manipulating lipid metabolic pathways to induce steatosis in the population of differentiated cells such that the cells accumulate high lipid content; and c) processing the population of differentiated cells into non-textured tissue for human consumption. Other methods comprise: a) culturing a population of cells in a culture medium having at least one nutritional supplement; b) manipulating lipid metabolic pathways to induce steatosis in the population of differentiated cells such that the cells accumulate high lipid content; and c) processing the population of differentiated cells into homogeneously textured tissue for human consumption.

In some cases, media formulations are produced that have certain growth factors, proteins, lipids, hormones, or any combination thereof required for cell culturing. Oftentimes, mammalian cell overexpression systems are used to generate any of the foregoing media components. In some instances, transgene expression in yeast, certain fungi, bacteria, algae, or insect cell (baculovirus) systems are utilized. The expressed media components are then isolated and/or purified in certain cases. Sometimes, the media formulations are generated using media conditioning technique(s). Alternatively, cells are sometimes cultured using co-culture models. However, in various cases, cells are cultured without co-culturing, or without co-culturing with xenobiotic cells such as yeast (e.g. organisms or cells that do not belong in the same kingdom, phylum, and/or species as the cells being cultured to produce food). For example, some avian cells are co-cultured with non-yeast cells such as mouse feeder cells.

Successful reduction or elimination of fetal bovine serum from cell culture media has been demonstrated. **FIG. 17** shows a graph plotting the number of cells from an immortalized cell line derived from adult duck hepatocytes. These immortalized cells were cultured in progressively decreasing concentrations of fetal bovine serum (FBS) in the presence of soybean hydrolysate (10g/L). The media supplementation of soybean hydrolysate allowed the serum requirements of the cultured hepatocytes to be reduced by 92%.

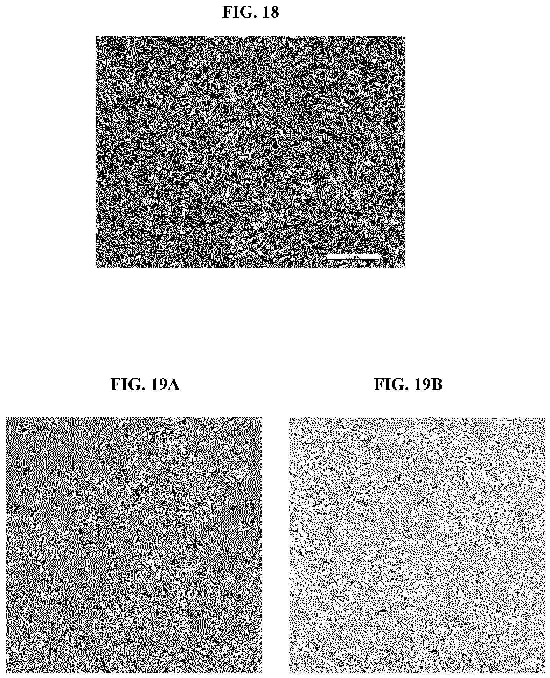

**FIG. 18** shows duck fibroblasts that have also been successfully grown in 10% shiitake mushroom extract after successive reduction of fetal bovine serum from the cell culture media. In some cases, the culture media is supplemented with at least 1%, 2%, 3%, 4%, 5%, 6%, 7%, 8%, 9%, 10%, 11%, 12%, 13%, 14%, or 15% mushroom extract. In certain instances, the culture media is supplemented with no more than 1%, 2%, 3%, 4%, 5%, 6%, 7%, 8%, 9%, 10%, 11%, 12%, 13%, 14%, or 15% mushroom extract. The culture media supplemented with mushroom extract can utilize a reduced serum concentration or no serum. In some cases, the supplemented culture media has no more than 1%, 2%, 3%, 4%, 5%, 6%, 7%, 8%, 9%, or 10% serum (e.g., animal serum such as FBS). Such reduced serum or no-serum culture media formulations can be utilized for growing or culturing any of the cells described herein including cells derived from various species such as avian cells, fish cells, porcine cells, bovine cells, and the cells of other edible species. In some cases, the cells are derived from domesticated species (e.g., cows, pigs, chickens, ducks, etc). In other cases, the cells are derived from non-domesticated species (trout, salmon, lobsters, crabs, etc). Various cell types can be cultured in the reduced serum or no-serum culture media formulations described herein, including multipotent cells, pluripotent cells, embryonic stem cells, induced pluripotent stem cells, myocytes, adipocytes, myosatellite cells, pre-adipocytes, mesenchymal stem cells, fibroblasts, hepatocytes, and other cell types.

In some cases, a population of cells or a cell line is adapted to grow in reduced serum or no-serum culture media formulations without requiring supplementation. **FIG. 19A** shows duck fibroblasts grown in serum-free media without additional supplementation; **FIG. 19B** shows a control culture grown in DMEM supplemented with 10% fetal bovine serum.

### Scalable production of cultured cells

Various methods are optionally used to scale up production of cultured cells for making food products for human consumption. The systems and methods disclosed herein enable the large-scale production of cultured foods **(****FIGs. 4A-4B****).** One method is to use 2-Dimensional surfaces such as tissue culture dishes or their functional equivalents (e.g. a cell culture chamber). A typical example would be a cell culture chamber having a polystyrene surface treated to increase hydrophilicity for enhancing attachment of adherent cells. Sometimes, the cell culture chamber is coated with a protein composition that acts as a substrate for cultured cells. The cell culture chamber often uses a media formulation as described herein. In many cases, the 2D surface approach is scaled up by combining a plurality of cell culture chambers. Sometimes, the cell culture chambers are stacked and arranged side by side. In some instances, cell culture chambers are stacked at least 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, or 100 chambers high. The stacks of cell culture chambers are usually arranged alongside each other. For example, in certain instances, the stacks of cell culture chambers are arranged side by side and/or front to back to maximize the usage of space. In many cases, the chambers are physically coupled (e.g. welded together as a single unit) and have common fill and vent ports connected by channels that allow liquid and air flow.

In certain embodiments, provided herein are bioreactor systems for culturing cells. Certain bioreactor systems facilitate production of cultured tissues suitable for human consumption. For example, some bioreactor systems comprise: a) a reactor chamber comprising a plurality of micro-scaffolds that provide adhesion surfaces for cellular attachment; b) a population of self-renewing cells cultivated within bioreactor; c) a first source providing at least one maintenance media comprising components for maintaining the population of self-renewing cells without spontaneous differentiation; and d) a second source providing at least one differentiation media comprising components for differentiating the population of self-renewing cells into a specific lineage; wherein the reactor chamber receives maintenance media from the first source to cultivate the population of cells and receives differentiation media from the second source to differentiate the population of cells, wherein the population of cells generated in a single batch comprises cultured tissues suitable for human consumption and having a dry weight of at least 1 kg.

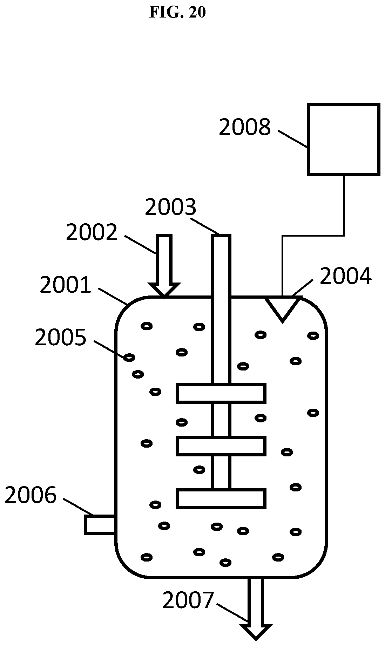

A bioreactor system is typically scalable for large-scale cell culture. **FIG. 20** illustrates a diagram of one embodiment of a bioreactor system comprising a reactor chamber **2001** for culturing cells. Oftentimes, the bioreactor system comprises stirring element **2003** for agitation of the contents of the reactor chamber **2001.** Continuous or periodic agitation helps keep cells, cell clumps, and/or micro-scaffolds in suspension. Fresh media is added into the reactor chamber via at least one input port **2002.** Fresh media is sometimes maintenance media, differentiation media, steatotic media, proliferation media, or any other media formulation disclosed herein. Depleted media or effluent is removed from the reactor chamber via at least one output port **2007.** In some cases, oxygen, carbon dioxide, and/or other gases are introduced through at least one input gas port **2006.** The input gas port **2006** is optionally coupled to an aerator positioned inside the reactor chamber. Oftentimes, the bioreactor system comprises at least one sensor **2004** for monitoring the reactor chamber. The at least one sensor **2004** is usually in communication with a control unit **2008** (e.g. a computer). In many cases, the reactor chamber is seeded with a plurality of micro-scaffolds **2005.** The micro-scaffolds **2005** enable adherence of certain adherent cells such as, for example, hepatocytes. For example, some methods of producing cultured fish meat for human consumption comprise: a) obtaining a population of self-renewing cells derived from fish; b) culturing the population of self-renewing cells in culture media comprising micro-scaffolds; c) inducing differentiation in the population of cells to form at least one of myocytes and adipocytes; and d) processing the population of cells into fish meat for human consumption.

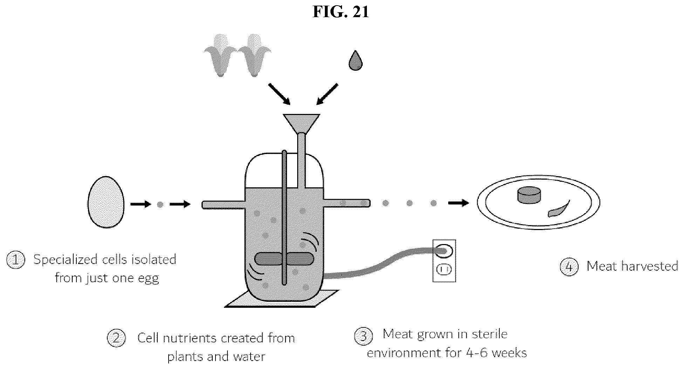

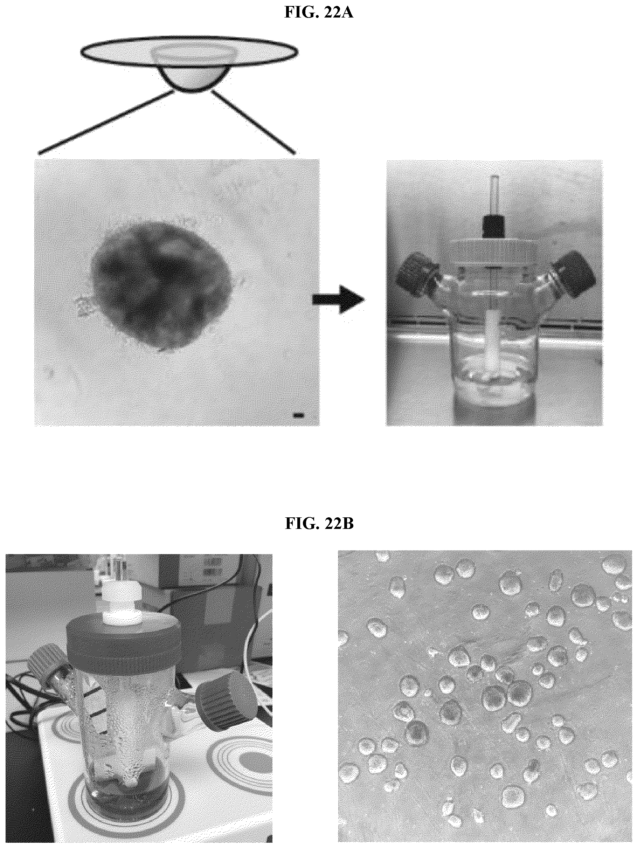

**FIG. 21** shows an illustrative process by which a bioreactor system is used for meat production. In this example, specialized cells such as embryonic, pluripotent, or multipotent cells are isolated from an egg and adapted for growth in the bioreactor (e.g. using a hanging drop method to form spheroid bodies as shown in **FIG. 22****).** The cells are grown using media comprising water and nutrients created from plants (e.g. using a plant-based substitute for animal-derived serum such as soybean hydrolysate or mushroom extract). The cells are grown in the sterile environment of the bioreactor for 4-6 weeks. In some cases, the cells are differentiated, and then harvested and/or processed into a meat product.

Transitioning cells from 2-D cell culture plates to 3-D bioreactors can be carried out using various methods such as the hanging drop method shown in **FIG. 22A****.** The hanging drop method entails placing cells in hanging drop culture and incubating them under physiological conditions until the cells form 3-D spheroids in which cells are in direct cell-cell contact and with extracellular matrix components. The illustrative example shown in **FIG. 22A** comprises suspending duck hepatocytes in hanging drops to initiate formation of spheroids (left panel). The spheroids are then transitioned into 3-D culture in a bioreactor **(****FIG. 22A** right panel). The 3-D culture allows for more rapid proliferation and/or growth of the cultured cells. Another exemplary bioreactor is shown in **FIG. 22B** (left panel). The cells from the spheroids are capable of propagating in the 3-D culture **(****FIG. 22B** right panel). In an exemplary embodiment of the hanging drop method, an adherent cell culture is washed with PBS and incubated with a 0.05% trypsin 1mM EDTA solution to dissociate the cells. The trypsinization is then neutralized by addition of culture media, and the cells are digested with DNAse for 5 minutes at room temperature. The cells are centrifuged at 250Gs for 5 minutes. Next, the supernatant is discarded, and the cells are resuspended in culture media. A hanging drop is formed by pipetting a volume (e.g. 10 µl) of culture media with cells onto the bottom of a lid from a tissue culture dish. Multiple hanging drops can be formed on a single lid. Several milliliters of PBS can be added to the bottom of the tissue culture dish to prevent dehydration of the hanging drops. The lid is then placed on the tissue culture dish followed by incubation at standard cell culture conditions (e.g. 5% CO₂, 37°C, 95% humidity) until spheroids are observed.

Sometimes, a bioreactor system comprises at least one bioreactor, bioreactor tank, or reactor chamber **2001.** For example, in certain instances, a bioreactor system comprises at least 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, or 100 reactor chambers. In some cases, a bioreactor system comprises about 1 reactor chamber to about 1,000 reactor chambers. Sometimes, a bioreactor system comprises about 1 reactor chamber. A bioreactor system usually comprises at most about 1,000 reactor chambers.

In some cases, the at least one reactor chamber has an internal volume suitable for large-scale cell culture. In some cases, a reactor chamber has an internal volume of about 1 L (liter) to about 100,000 L. In most instances, a reactor chamber has an internal volume of at least about 1 L, 10 L, 50 L, 100 L, 200 L, 300 L, 400 L, 500L, 1,000 L, 5,000 L, 10,000 L, 20,000 L, 30,000 L, 40,000 L, 50,000 L, or 100,000 L. Sometimes, a reactor chamber has an internal volume of at most about 10 L, 50 L, 100 L, 200 L, 300 L, 400 L, 500L, 1,000 L, 5,000 L, 10,000 L, 20,000 L, 30,000 L, 40,000 L, 50,000 L, or 100,000 L.

In some cases, disclosed herein are bioreactor systems suitable for large-scale production of cultured cells for generation of food products. Oftentimes, cells are cultured on a batch basis. Alternatively, or in combination, cells are cultured continuously. In both batch and continuous cultures, fresh nutrients are usually supplied to ensure the appropriate nutrient concentrations for producing the desired food product. As an example, in a fed-batch culture, nutrients (e.g. fresh culture media) is supplied to the bioreactor, and the cultured cells remain in the bioreactor until they are ready for processing into the finished food product. In a semi-batch culture, a base media is supplied to the bioreactor and supports an initial cell culture, while an additional feed media is then supplied to replenish depleted nutrients. Sometimes, a bioreactor system produces at least a certain quantity of cells per batch. In some cases, a bioreactor system produces a batch of about 1 billion cells to about 100,000,000 billion cells. Oftentimes, a bioreactor system produces a batch of at least about 1 billion cells, 10 billion cells, 100 billion cells, 1,000 billion cells, 10,000 billion cells, 100,000 billion cells, 1,000,000 billion cells, 10,000,000 billion cells, or 100,000,000 billion cells. A bioreactor system usually produces a batch of at most about 10 billion cells, 100 billion cells, 1,000 billion cells, 10,000 billion cells, 100,000 billion cells, 1,000,000 billion cells, 10,000,000 billion cells, or 100,000,000 billion cells.

In some cases, a bioreactor system produces a batch of cultured cells during a certain time period. For example, in some cases, a bioreactor system produces a batch of cultured cells at least once every 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 days.

In some cases, a bioreactor system produces a batch of cultured cells having at least a certain mass. Sometimes, the mass is measured as dry weight with excess media or supernatant removed. Usually, a bioreactor system produces a batch of cultured cells of about 1 kg to about 10,000 kg. In certain instances, a bioreactor system produces a batch of at least about 1 kg, 5 kg, 10 kg, 20 kg, 30 kg, 40 kg, 50 kg, 100 kg, 500 kg, 1,000 kg, 5,000 kg, or 10,000 kg. Oftentimes, a bioreactor system produces a batch of at most about 5 kg, 10 kg, 20 kg, 30 kg, 40 kg, 50 kg, 100 kg, 500 kg, 1,000 kg, 5,000 kg, or 10,000 kg.

Cells grown in bioreactor systems are typically grown in suspension. Oftentimes, the bioreactor system has components that enable the automated growth and maintenance of cell cultures. A bioreactor comprises at least one reactor chamber or reactor tank wherein the cultured cells grow. In some cases, the bioreactor system has at least one pump for circulating the media, introducing fresh media, and/or removing waste media. Oftentimes, the bioreactor system comprises a plurality of media tanks for introducing various types of media such as, for example, proliferation media, maintenance media (e.g. for maintenance of self-renewal ability), differentiation media, and steatotic media. Sometimes, the bioreactor system comprises at least one of oxygenator(s), carbon dioxide regulator(s), and a central control unit regulating components of the bioreactor system. In many instances, the bioreactor has a stirring element for maintaining cultured cells in suspension and/or keeping the media mixed. The bioreactor usually comprises at least one sensor for monitoring the environment inside the reactor chamber. A sensor is typically a biosensor, a chemosensor, or an optical sensor for monitoring parameters important to cell culture. In some cases, a sensor is configured to monitor at least one of pH, temperature, oxygen, carbon dioxide, glucose, lactate, ammonia, hypoxanthine, amino acid(s), dopamine, and lipid(s). Oftentimes, the at least one sensor is in communication with a control unit (e.g. a computer system) that monitors the sensor parameters. In certain instances, the control unit provides the sensor parameters to a user such as, for example, on a display screen. The control unit often comprises at least one input source for receiving commands from a user.

In some cases, cells are cultured in suspension in cell culture flasks. The cell culture flasks are optionally stacked and/or arranged side-by-side as described for the 2D surface cell culture. Cells cultured in suspension are usually non-adherent cells. In some cases, however, adherent cells are cultured on scaffolds in a suspension. Scaffolds provide structural support and a physical environment for cells to attach, grow, and migrate. In addition, scaffolds usually confer mechanical properties such as elasticity and tensile strength. Oftentimes, 3D scaffolds are used to culture adherent cells so as to enable 3D growth of the cells. Scaffolds sometimes have specific shapes or sizes for guiding the growth of the cultured cells. In some cases, scaffolds are composed of one or more different materials. Some scaffolds are solid scaffolds, while others are porous. Porous scaffolds allow cell migration or infiltration into the pores. Scaffolds are typically composed of a biocompatible material to induce the proper recognition from cells. In addition, the scaffold is made of a material with suitable mechanical properties and degradation kinetics for the desired tissue type that is generated from the cells. In certain instances, a scaffold comprises a degradable material to enable remodeling and/or elimination of the scaffold in the cultured food product. For example, in some cases, a 3D scaffold that shapes cultured hepatocytes into the shape of a liver biodegrades after the hepatocytes expand to fill up the interior space of the scaffold. In other instances, the scaffold comprises a material that remains in the cultured food product. For example, sometimes, at least a portion of a collagen scaffold providing support to cultured myocytes remains to provide texture and continuing structural support in the cultured food product. In some cases, a scaffold comprises a hydrogel, a biomaterial such as extracellular matrix molecule (ECM) or chitosan, or biocompatible synthetic material (e.g. polyethylene terephthalate). ECM molecules are typically proteoglycans, non-proteoglycan polysaccharides, or proteins. Potential ECM molecules for use in scaffolding include collagen, elastin, heparan sulfate, chondroitin sulfate, keratan sulfate, hyaluronic acid, laminin, and fibronectin. Sometimes, plant-based scaffolds are used for 3D culturing. Non-limiting examples of plant-based scaffolds include scaffolds obtained from plants such as apples, seaweed, or jackfruit. The plant-based scaffolds often comprise at least one plant-based material such as cellulose, hemicellulose, pectin, lignin, alginate, or any combination thereof. Sometimes, plant-based scaffolds are decellularized. In some cases, scaffolds are not required for 3D culturing. In various instances, scaffolds used in the methods and compositions described herein comprise at least one of hydrogel, chitosan, polyethylene terephthalate, collagen, elastin, heparan sulfate, chondroitin sulfate, keratan sulfate, hyaluronic acid, laminin, fibronectin, cellulose, hemicellulose, pectin, lignin, alginate, glucomannan, polycaprolactone (PCL), textured vegetable protein (TVP), and acrylates. An example of textured vegetable protein is textured soy protein (TSP), which typically comprises a high percentage of soy protein, soy flour, or soy concentrate. TVP and TSP can be used to provide a meat-like texture and consistency to the meat products described herein. In some cases, the meat product comprising TVP or TSP is seasoned to taste like meat (e.g., using various salts, herbs, and/or spices).

In some cases, cells are cultured with micro-scaffolds that enable cell adhesion. Micro-scaffolds are usually Micro-scaffolds allow adherent cells to be grown in a suspension bioreactor system. For example, a micro-scaffold provides a surface for adherent cells such as hepatocytes to attach even while the micro-scaffold itself is in suspension. In certain instances, scaffolds and/or micro-scaffolds are produced by 3D printing of an appropriate material (e.g. collagen). Micro-scaffolds often have a porous structure. Sometimes, a micro-scaffold has a solid nonporous structure. A micro-scaffold is smaller than a conventional scaffold. Scaffolds are typically used for providing macroscopic structure and/or shape for the cell population, whereas a micro-scaffold usually provides a seed or core structure for adherent cells to attach while remaining small enough to remain in suspension with stirring. The use of micro-scaffolds enables the culturing of adherent cells in a suspension culture. The culturing of cells using micro-scaffolds in a bioreactor system suspension culture enables the large-scale production of adherent cells. For example, adherent cells such as hepatocytes, myocytes, and adipocytes are capable of being grown on a large scale in bioreactor suspension cultures using micro-scaffolds. This allows for the production of luxury food items like foie gras or sushi grade salmon meat. Alternatively, cultured fish cells are sometimes processed into surimi such as salmon, tuna, or trout surimi.

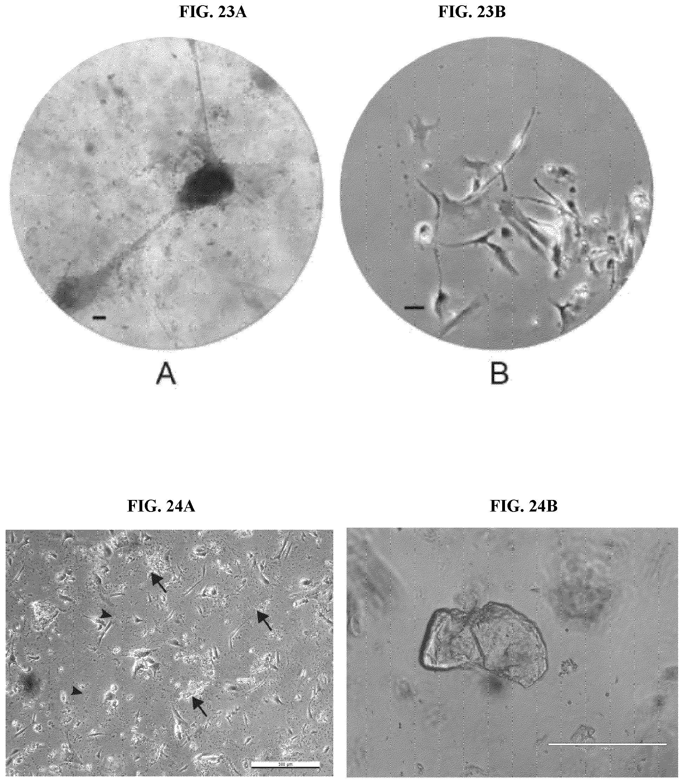

**FIG. 23A** shows trout myosatellite cells grown on glucomannan microscaffolds that have successfully differentiated into myotubes in the 3-dimensional culture. **FIG. 23B** shows a negative control of undifferentiated myosatellite cells. **FIG. 24A** shows duck fibroblasts (arrowheads) successfully grown on glucomannan microscaffolds (arrows). **FIG. 24B** shows a representative glucomannan microscaffold. Alternative materials can be used in producing microscaffolds. Microscaffolds can comprise at least one of glucomannan, alginate, chitosan, polycaprolactone (PCL), matrix proteins (e.g., collagen, fibronectin, laminin), textured vegetable protein (TVP), textured soy protein (TSP), and acrylates. In some cases, polycaprolactone (PCL) is used to generate PCL-woven scaffolds or PCL-fibrin composites. In some cases, the microscaffolds are modified. In certain instances, microscaffolds are conjugated to one or more factors relevant to cell attachment, growth, differentiation, or a combination thereof. As an example, glucomannan microscaffolds are conjugated to FGF2 to promote growth and differentiation of myosatellite cells into myotubes. In some cases, microscaffolds are conjugated to one or more growth or differentiation factors via covalent bonding such as chemical cross-linking or other reactions for immobilizing the factors to the microscaffold structure.

Alternatively, in some cases, scaffolds or micro-scaffolds are not needed for culturing of cells in suspension. Sometimes, the cells are non-adherent cells and do not require a substrate or surface for attachment. In certain instances, the cells have been modified or engineered to no longer require an adherence substrate. For example, hepatocytes are normally adherent cells, but in some instances, hepatocytes are modified to no longer require an extracellular matrix for attachment for survival and proliferation. Other adherent cells include myocytes and adipocytes, which are sometimes cultured to produce cultured meat products. For example, some methods of producing cultured meat for human consumption comprise: a) obtaining a population of self-renewing cells, said cells capable of growing in suspension culture; b) culturing the population of self-renewing cells in suspension; c) inducing differentiation in the population of cells to form at least one of myocytes and adipocytes; and d) processing the population of cells into meat for human consumption.

The cells culturing systems described herein enable the culturing of cells for food production in a pathogen-free environment. Generally, cells are grown in a culture environment free of dangerous contaminants that affect human health. Cell culture plates, flasks, and bioreactors typically provide cell culture conditions free of dangerous pathogens (e.g. H1N1), parasites, heavy metals, and toxins (e.g. bacterial endotoxins, pesticides, etc.). In some cases, the methods described herein do not utilize antibiotics. Sometimes, the methods use an inducing agent such as tetracycline for a one-time induction of cell differentiation and/or a cell phenotype, followed by removal of the inducing agent before the cells are processed into a food product.

### Tissue processing

Provided herein are systems and methods for processing cultured cells to create an appropriate taste, texture, consistency, or other desired quality in a food product. The cells are typically a differentiated cell population such as, for example, myocytes, adipocytes, or hepatocytes. Also provided herein are systems and method for creating food products which do not comprise any cultured cells in the final product. Approaches consistent with the present disclosure include spinning techniques such as electrospinning and solution blow spinning. Additional techniques compatible with the present disclosure include unidirectional freeze drying, wet spinning, casting, micromolding, 3D printing, or extrusion methods.

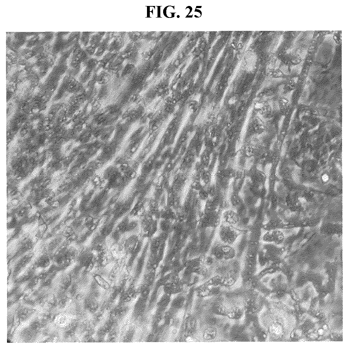

In various cases, the cells are animal cells. Sometimes, the cells are fish, animal, or avian cells. Examples of avian cells include cells derived from geese, ducks, chickens, Cornish game hens, pheasants, turkeys, Guinea hens, quails, pigeons, partridges, emus, ostriches, capons, grouses, swans, doves, woodcocks, chukars, and snipes. As an example, **FIG. 25** shows an image of duck muscle tissue created according to the methods disclosed herein. The successful generation of the duck muscle tissue in **FIG. 25** was confirmed by the muscle tissue's ability to spontaneously contract.

Some methods disclosed herein allow production of cultured non-textured muscle tissue for human consumption. Non-textured muscle tissue includes certain fish muscle tissues. Certain methods of generating non-textured muscle tissue comprise: a) obtaining a population of self-renewing cells; b) culturing the population of self-renewing cells; c) inducing differentiation in the population of cells to form non-textured muscle tissue; and d) processing the cultured non-textured muscle tissue for human consumption. In addition, some methods produce cultured fish tissue having enhanced nutritional content for human consumption. For example, certain methods comprise: a) culturing a population of fish myocytes in a culture media having at least one nutritional supplement; b) expanding the population of myocytes; and c) processing the population of myocytes into fish tissue for human consumption. The systems and methods described herein often allow production of edible compositions comprising fish tissue produced from cultured myocytes and adipocytes.

In some cases, the fish adipocytes and/or myocytes are processed into fish meat such as, for example, salmon meat. In various instances, fish adipocytes and/or myocytes are processed into a finished fish meat product. Other examples of processed fish products include minced fish meat, fish fillet, fish cutlet, and fish steak. These various shapes and sizes of the fish product are obtained by processing the myocytes and/or adipocytes together with various additional ingredients such as a binder, filler, or extender to provide structural cohesion and/or texture. In some instances, the meat product is cooked or cured. Processing the cultured cells into a meat product can include at least one of smoking, fermenting, salting, marinating, poaching, baking, barbecuing, casseroling, shallow frying, deep frying, oven frying, grilling, and microwaving. In some cases, the cells are processed into sushi grade fish meat suitable for raw consumption without being frozen. In certain cases, the fish meat is not cooked during processing. Examples of sushi grade fish meat produced according to the systems and methods disclosed herein include salmon and tuna. As used herein, sushi grade meat refers to meat that is produced free of parasites and bacteria. The cells are usually free of pathogens, parasites, toxins, heavy metals (e.g. mercury), antibiotics, or any combination thereof. Certain systems and methods described herein provide for the production of cultured food products without exposure to contaminant(s). Some methods enable production of cultured cells for human consumption without using antibiotics. For example, certain methods comprise: a) culturing a population of cells without using antibiotics; b) inducing differentiation within the population of cells; c) inducing high lipid accumulation within the population of cells; and d) processing the population of cells for human consumption. Also disclosed herein are methods of producing cultured cells for human consumption without exposure to pathogens. Some such methods comprise: a) culturing a population of cells in a pathogen-free culture environment; b) inducing differentiation within the population of cells; c) inducing high lipid accumulation within the population of cells; and d) processing the population of cells for human consumption. Certain methods allow cultured food production without exposure to toxins. For example, some such methods comprise: a) culturing a population of cells in a toxin-free culture environment; b) inducing differentiation within the population of cells; c) inducing high lipid accumulation within the population of cells; and d) processing the population of cells for human consumption.

In certain cases, cultured meat comprises a mixed population of myocytes and adipocytes. Oftentimes, pre-adipocytes and satellite cells are isolated from a source such as, for example, fish fingerlings. The pre-adipocytes and satellite cells are useful because they have some self-renewal capacity. The pre-adipocytes and satellite cells are typically cultured and expanded, and subsequently differentiated. In some cases, the pre-adipocytes and satellite cells are cultured together. Usually, they are cultured separately until after differentiation when they are co-cultured together at a certain ratio to produce a desired ratio in a final fish product. Alternatively, a population of cells is sometimes induced to differentiate into different cell types in the same culture. For example, in this scenario, some cells form into adipocytes and some form into myocytes. Usually, myocytes and adipocytes are cultured separately, and subsequently mixed. Sometimes, the myocytes and adipocytes are homogeneously mixed in equal proportions. In other cases, the myocytes and adipocytes are heterogeneously mixed in unequal proportions. For co-culturing or processing, the myocytes and adipocytes are typically combined at a certain ratio or proportion. For example, in some cases, myocytes and adipocytes are combined at a ratio of at least 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 26:1, 27:1, 28:1, 29:1, 30:1, 35:1, 40:1, 45:1, 50:1, 60:1, 70:1, 80:1, 90:1, or at least 100:1, respectively. Oftentimes, the myocytes and/or adipocytes are fish cells. In some instances, the myocytes and/or adipocytes are derived from salmon. Sometimes, the myocytes and/or adipocytes are derived from sea bass, tuna, mackerel, blue marlin, swordfish, yellowtail, salmon, trout, eel, abalone, squid, clams, ark shell, sweetfish, scallop, sea bream, halfbeak, shrimp, flatfish, cockle, octopus, or crab. Examples of tuna include yellowfin, southern Bluefin, northern Bluefin, Thunnus alalunga, Thunnus atlanticus, and Thunnus obesus. In certain cases, instead of combining myocytes and adipocytes, myocytes are induced to undergo steatosis to provide the desired lipid or fat content found in conventional salmon meat without requiring adipocytes.

Provided herein are systems and methods for producing meat having a certain ratio of fast twitch and slow twitch muscle cells and/or fibers. The meat produced according to the systems and methods disclosed herein usually comprises myocytes or skeletal muscle cells having a certain ratio or proportion of fast twitch (type II) and slow twitch (type I) muscle fibers. Slow twitch muscle fibers exhibit low-intensity contractions fueled by the oxidative pathway and demonstrate relatively higher endurance, while fast twitch muscle fibers have higher intensity contractions fueled by the glycolytic pathway. Fast twitch muscles are characterized by high glycolytic and anaerobic muscle fibers. The ratio of fast twitch and slow twitch muscle fibers in muscle tissue plays a role in the taste, color, texture, and other culinary properties of the meat. For example, fish meat is characterized by a high proportion of fast twitch muscle fibers compared to animal meat, which has some role in the culinary differences between the two categories of meat. Sometimes, myocytes such as salmon myocytes are cultured to comprise at least 10%, 20%, 30%, 40%, 50%, 60%, 70%, 80%, 90%, 95% or more fast twitch fibers (e.g. high glycolytic and anaerobic muscle fibers). The percentage of fast twitch muscle fibers can be characterized by evaluating tissue samples using various laboratory techniques such as microscopy-based imaging (e.g. staining tissue sections for fast twitch muscle fiber markers). In some cases, a myocyte generated according to the methods described herein has a length of at least about 10 µm, about 20 µm, about 30 µm, about 40 µm, about 50 µm, about 60 µm, about 70 µm, about 80 µm, about 90 µm, about 100 µm, about 110 µm, about 120 µm, about 130 µm, about 150 µm, about 200 µm, about 250 µm, about 300 µm, about 350 µm, about 400 µm, about 450 µm, about 500 µm, about 600 µm, about 700 µm, about 800 µm, about 900 µm, about 1000 µm, about 2000 µm, about 3000 µm, about 4000 µm, about 5000 µm, about 6000 µm, about 7000 µm, about 8000 µm, about 9000 µm, or about 10000 µm or more.

Certain muscle tissues lack the texture of animal skeletal muscle such as beef, pork, or chicken. For example, fish muscle tissue tends to have a non-textured or un-textured consistency. Fish muscle tissue such as salmon and tuna are often described as having a tasted texture that is delicate, soft, and/or having a homogeneous consistency. Other examples of non-textured meat include squid and octopus muscle tissue, which have a distinct non-textured consistency in comparison to animal skeletal muscle. Liver food products such as foie gras also have a non-textured characteristic. Certain methods described herein allow production of cultured non-textured tissue. Some such methods comprise: a) culturing a population of cells; b) inducing differentiation in the population of cells; c) manipulating lipid metabolic pathways to induce steatosis in the population of cells such that the cells accumulate high lipid content; and d) processing the population of cells into non-textured tissue.

Certain methods disclosed herein produce cultured non-muscle tissue for human consumption. Some such methods comprise: a) obtaining a population of self-renewing cells; b) culturing the population of self-renewing cells; c) inducing differentiation in the population of cells to form non-muscle tissue; and d) processing the cultured non-muscle tissue for human consumption. In some cases, the cells are hepatocytes. The hepatocytes are harvested after culturing for processing into a food product. Sometimes, the hepatocytes are processed into foie gras.

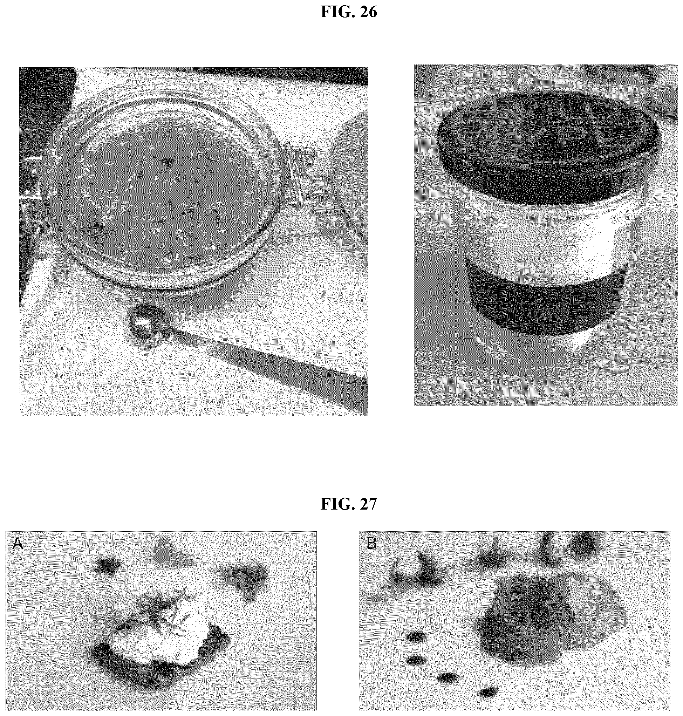

The systems and methods disclosed herein enable the production of culinary foie gras compositions comprising tissue cultured hepatocytes having high lipid content and processed for human consumption. Some food product compositions comprise cultured organ cells processed into a non-textured non-muscle food product for human ingestion. The food product is sometimes an edible foie gras composition comprising cultured steatotic avian liver cells and seasoning. Oftentimes, foie gras compositions comprise cultured liver cells having high lipid content and liver cells having low lipid content. Edible compositions are sometimes produced that comprise avian liver cells grown in cell culture and processed for human consumption. In some cases, the food product is packaged with an optional label. As an example, some packaged foie gras compositions comprise cultured liver cells and packaging having a label indicating the foie gras composition was produced without forced feeding. Other packaged foie gras compositions comprise cultured liver cells processed into foie gras and packaging having a label indicating the foie gras was produced in a pathogen-free environment. In certain aspects, packaged edible compositions comprise cultured cells processed into a food product and packaging having a label indicating the composition was produced without exposure to a toxin. **FIG. 26** shows exemplary food products produced from duck hepatocytes. The left panel shows a duck liver pâté made using duck steatotic liver cells. The right panel shows fois gras butter made using duck steatotic liver cells. **FIG. 27** shows exemplary food products for human consumption produced according to the methods disclosed herein. The left panel shows a salmon pâté produced using salmon myocytes. The right panel shows a duck meat pâté produced using duck myocytes. In addition, chicken meat pâtés have also been developed using chicken myocytes.

In some cases, the foie gras has a substantially identical texture and/or consistency with conventional foie gras. In many cases, the foie gras is rated as grade A, grade B, or grade C foie gras. The foie gras has no blemishes, in some cases. The foie gras usually has no more than 1, 2, 3, 4, 5, 6, 7, 8, 9, or 10 blemishes. Sometimes, the hepatocytes are processed as a single foie gras composition. For example, a single foie gras composition is typically a liver or liver-shaped. Methods of processing harvested cells for human consumption include centrifugation and compaction. In some cases, the harvested cells receive some degree of structural integrity from the scaffold and/or micro-scaffolds on which the cells are attached during culturing. Sometimes, the harvested cells are combined with at least one other ingredient. The harvested cells are often combined with at least one other ingredient to obtain a food product having a desired texture, moisture retention, product adhesion, or any combination thereof. An ingredient is typically a binder, filler, or extender. A filler or binder is oftentimes a non-meat substance comprising carbohydrates such as a starch. Examples of fillers and binders include potato starch, flour, eggs, gelatin, carrageenan, and tapioca flour. Alternatively, extenders tend to have high protein content. Examples of extenders include soy protein, milk protein, and meat-derived protein. Certain ingredients that provide flavor, texture, or other culinary properties are added in some instances. For example, sometimes, extracellular matrix proteins are used to modulate structural consistency and texture. Certain proteins such as heme and collagen are occasionally incorporated into the extracellular matrix to contribute to the taste and texture of the final food product.

In many cases, cells are grown in suspension culture on micro-scaffolds that comprise at least one natural protein with texture-modifying properties. Micro-scaffolds of varying compositions can be used to produce a desired texture and/or consistency in the final food product. Sometimes, textured vegetable protein such as soy protein is used. Micro-scaffolds optionally comprise at least one filler or binder material for providing texture to the food product. Sometimes, micro-scaffolds are made of materials that biodegrade such that the finished food product no longer has any micro-scaffold structures remaining. For example, a population of cells is seeded onto micro-scaffolds in a bioreactor. As the cells adhere to the micro-scaffolds and proliferate, the micro-scaffolds gradually biodegrade until all that remains are the clumps of cells that are now adhered to each other and the extracellular matrix materials that they have secreted. Accordingly, micro-scaffolds (and also larger 3-D scaffolds) can be used to guide the structure of the resulting cultured food product but do not remain in the food product for consumption by a human. Alternatively, micro-scaffolds and 3-D scaffolds may comprise materials that do not biodegrade and/or remain in the cultured food product for consumption. For example, certain materials described herein can be used to generate the scaffolds in order to confer a particular structure, texture, taste, or other desired property.

In certain instances, a foie gras composition weighs at least 1, 2, 3, 4, 5, 6, 7, 8, 9, 10, 12, 16, 20, 24, 28, 32, 36, 42, 48, 52, 56, 60, or 64 ounces. Oftentimes, a foie gras composition weighs about 1 ounce to about 64 ounces. In many cases, a foie gras composition weighs at least about 1 ounce. A foie gras composition usually weighs at most about 64 ounces.

In some embodiments, a foie gras composition weighs about 1 ounce to about 2 ounces, about 1 ounce to about 4 ounces, about 1 ounce to about 8 ounces, about 1 ounce to about 12 ounces, about 1 ounce to about 16 ounces, about 1 ounce to about 20 ounces, about 1 ounce to about 24 ounces, about 1 ounce to about 30 ounces, about 1 ounce to about 36 ounces, about 1 ounce to about 48 ounces, about 1 ounce to about 64 ounces, about 2 ounces to about 4 ounces, about 2 ounces to about 8 ounces, about 2 ounces to about 12 ounces, about 2 ounces to about 16 ounces, about 2 ounces to about 20 ounces, about 2 ounces to about 24 ounces, about 2 ounces to about 30 ounces, about 2 ounces to about 36 ounces, about 2 ounces to about 48 ounces, about 2 ounces to about 64 ounces, about 4 ounces to about 8 ounces, about 4 ounces to about 12 ounces, about 4 ounces to about 16 ounces, about 4 ounces to about 20 ounces, about 4 ounces to about 24 ounces, about 4 ounces to about 30 ounces, about 4 ounces to about 36 ounces, about 4 ounces to about 48 ounces, about 4 ounces to about 64 ounces, about 8 ounces to about 12 ounces, about 8 ounces to about 16 ounces, about 8 ounces to about 20 ounces, about 8 ounces to about 24 ounces, about 8 ounces to about 30 ounces, about 8 ounces to about 36 ounces, about 8 ounces to about 48 ounces, about 8 ounces to about 64 ounces, about 12 ounces to about 16 ounces, about 12 ounces to about 20 ounces, about 12 ounces to about 24 ounces, about 12 ounces to about 30 ounces, about 12 ounces to about 36 ounces, about 12 ounces to about 48 ounces, about 12 ounces to about 64 ounces, about 16 ounces to about 20 ounces, about 16 ounces to about 24 ounces, about 16 ounces to about 30 ounces, about 16 ounces to about 36 ounces, about 16 ounces to about 48 ounces, about 16 ounces to about 64 ounces, about 20 ounces to about 24 ounces, about 20 ounces to about 30 ounces, about 20 ounces to about 36 ounces, about 20 ounces to about 48 ounces, about 20 ounces to about 64 ounces, about 24 ounces to about 30 ounces, about 24 ounces to about 36 ounces, about 24 ounces to about 48 ounces, about 24 ounces to about 64 ounces, about 30 ounces to about 36 ounces, about 30 ounces to about 48 ounces, about 30 ounces to about 64 ounces, about 36 ounces to about 48 ounces, about 36 ounces to about 64 ounces, or about 48 ounces to about 64 ounces.

In some instances, cultured cells are processed into additional food products aside from foie gras and salmon or fish meat. For example, the cells are sometimes processed into chopped or whole liver for culinary purposes. In some cases, other tissues are generated for human consumption such as, for example, yakitori or other chicken organ products. Oftentimes, other organs are generated for avian and other species (e.g., thymus or pancreas for sweet breads). In some cases, fatty or steatotic hepatocytes are generated and blended with healthy liver cells to make foie gras pâté or other terrines. The stem cell isolation techniques described herein are optionally used for the purpose of growing chicken, duck meat, or those of other animals. In certain instances, the techniques described herein are also applicable to the production of other animal-based meats.

Some of the systems and methods disclosed herein allow production of cultured liver cells for human consumption. Certain methods enable production of cultured non-textured tissue having high lipid content, the methods comprising: a) culturing a population of cells; b) inducing differentiation in the population of cells; c) manipulating lipid metabolic pathways to induce steatosis in the population of cells such that the cells accumulate high lipid content; and d) processing the population of cells into non-textured tissue having high lipid content.

Certain cultured cells and/or tissues produced using the methods described herein are processed into food products. In some cases, the cultured food product is packaged and/or labeled. The cultured cells and/or tissues can be processed into a plurality of slices (e.g. slices of foie gras or salmon meat) to form the cultured food product. The plurality of slices can be at least 2, 3, 4, 5, 6, 7, 8, 9, 10, 11, 12, 13, 14, 15, 16, 17, 18, 19, 20, 25, 30, 35, 40, 45, 50, 60, 70, 80, 90, or 100 slices. Sometimes, the plurality of slices is no more than 2, 3, 4, 5, 6, 7, 8, 9, 10, 11, 12, 13, 14, 15, 16, 17, 18, 19, 20, 25, 30, 35, 40, 45, 50, 60, 70, 80, 90, or 100 slices. In certain instances, the cultured food product is processed into shaped portions. The portions may be individually packaged or packaged together. The portions may be any number of shapes such as rectangle, square, circular, triangle, doughnut, tube, pyramid, or other shapes. The portions can have a flat shape (e.g. thin slices). For example, a portion of the cultured food product can have a thickness of no more than about 5 mm, about 10 mm, about 20 mm, about 30 mm, about 40 mm, about 50 mm, about 60 mm, about 70 mm, about 80 mm, about 90 mm, or about 100 mm. Sometimes, a portion of the cultured food product has a thickness of at least about 5 mm, about 10 mm, about 20 mm, about 30 mm, about 40 mm, about 50 mm, about 60 mm, about 70 mm, about 80 mm, about 90 mm, or about 100 mm.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### Manufacturing Food Products

Provided herein are systems and methods for creating a food product having the appropriate taste, texture, consistency, or other desired quality. The food product can be configured to have the taste, texture, consistency, or other food characteristic that is similar to meat or a meat product. In some cases, the food product incorporates cultured cells such as cultured muscle cells, fat cells, liver cells, or any combination thereof. Alternatively, the food product may not have any cultured cells, and instead uses non-cell ingredients, for example, to produce a meat product having similar food characteristics as natural meat. The food product can include various ingredients such as cultured and/or non-cultured cells or tissues. Cultured cells or tissues can be generated according to the methods described herein. Non-cultured cells or tissues are not artificially grown (e.g., as cell cultures in a bioreactor) and are instead harvested from living organisms (e.g., myocytes harvested from an animal). Other ingredients consistent with the present disclosure include raw or processed sources of protein, carbohydrates, and/or lipids.

The cells can include a differentiated cell population such as, for example, myocytes, adipocytes, or hepatocytes. In some cases, the cells comprise fish roe such as, for example, salmon roe, sea bass roe, tuna roe, mackerel roe, blue marlin roe, swordfish roe, yellowtail roe, salmon roe, or trout roe.

In many cases, an ingredient comprises one or more natural proteins, carbohydrates, and/or lipids. The ingredient can be configured to provide texture-modifying properties.

In some cases, the ingredient comprises one or more vitamins, minerals, and/or nutritional supplements. Vitamins include water-soluble vitamins such as vitamin B1 (thiamine), vitamin B2 (riboflavin), vitamin B3 (niacin), vitamin B5 (pantothenic acid), vitamin B6 (pyridoxine), vitamin B9 (folic acid), vitamin B12 (cobalamin), vitamin B7 (biotin), and vitamin C (ascorbic acid). Fat soluble vitamins include vitamin A (retinoids), vitamin D (cholecalciferol, ergocalciferol), vitamin E (tocopherol), and vitamin K (phylloquinone, menaquinones). Minerals include macrominerals such as calcium, phosphorus, potassium, magnesium, and sodium, and microminerals such as iron, zinc, copper, chromium, fluoride, iodine, selenium, manganese, and molybdenum. Certain vitamins are unstable upon exposure to heat or at high temperatures. Thus, in some cases, the ingredient(s) are processed into the food product without requiring exposure to heat. For example, solution blow spinning can be used to assemble ingredients comprising polymeric fibers at various temperatures such as, for example, room temperature, thus allowing for ingredients that include heat-unstable vitamins to undergo the process without experiencing significant loss of the vitamins.

The ingredients used according to the present disclosure can include various compositions depending upon the desired texture, consistency, taste, nutritional content, appearance, and/or other considerations with regards to the final food product.

Various approaches for combining ingredients to form a food product are consistent with the present disclosure. In some instances, ingredients are combined using a spinning technique such as, for example, solution blow spinning, melt spinning, electrospinning, and melt blowing.

The ingredients used in the spinning approaches described herein can include monomers or molecules that can be formed into fibers. In some cases, the ingredients comprise polymers. Spinning techniques can be used for forming and/or combining fibers together to generate the food product.

Traditional solution blow spinning with heat and extrusion has been used for the formation of thermoplastic polymer fibers in the range of 1-50µm in diameter. Electrospinning is another method for making fibers and can produce fibers 0.17nm - 5µm in diameter. Electrospinning can produce smaller fibers than previously possible and offers a much greater variety of synthetic and natural polymers.

A new solution blow spinning technique has been recently developed that combines various advantages of traditional solution blow spinning and electrospinning. This solution blow spinning technique can be performed using commercially inexpensive and easy to use airbrushes and does not require the application of high voltages or heat. Moreover, solution blow spinning enables the use of an even greater number of natural and synthetic polymers and solvents, and produced fibers at orders of magnitude faster than previous approaches.

In some cases, solution blow spinning pumps monomers or other molecules (e.g., food proteins or starches that can be derived from various sources such as plants, algae, etc.) dissolved in a solvent through the central nozzle of an airbrush containing concentric nozzles with the outermost nozzle providing a high velocity gas or air stream (see FIG. 1). When the monomers hit the high pressure stream of gas or air, the low pressure and shear pull fibers from the inner nozzle or nozzles across the "working distance" where the solvent quickly evaporates, and entangled polymer fibers are collected as nano-/micro-fibers. The fibers can be gathered in a collector positioned to receive the fibers as they are formed. A collector consistent with this approach is a spinning cylinder that creates aligned fibers, which can provide a geometry or configuration similar to muscle fibrils. Described herein is a novel process that utilizes solution blow spinning to create biomimetic muscle fibers for the production of comestible meat.

A variety of parameters of the solution blow spinning process can be configured to achieve a desired polymeric fiber diameter or range. Examples of these parameters include monomer concentrations, mixing ratios, solvation processes, gas or air humidity, gas or air pressure, monomer pumping speeds, nozzle diameters, number of nozzles, nozzle sizes, internozzle distances, distance from nozzle to collector (working distance), collector geometry, collector rotational speed, and temperatures. In some cases, optimal fibers are formed from monomer solutions of sufficient viscosity to form a Taylor cone after exiting the nozzle. Additionally, in some cases, the solvent, temperature, and distance from the nozzle to the collector are sufficient to allow complete or near complete solvent evaporation before depositing the polymer on the collector. In some cases, the solvent is a volatile solvent. The amount of volatile present in the deposited fiber or polymer may be no more than 0.1 % (w/w), 1 % (w/w), 2 % (w/w), 3 % (w/w), 4 % (w/w), 5 % (w/w), 10 % (w/w), 15 % (w/w), 20 % (w/w), 25 % (w/w), 30 % (w/w), 35 % (w/w), 40 % (w/w), 45 % (w/w), or 50 % (w/w).

In some cases, the concentration of total monomer in the solution is about 3 % (w/w) to about 30 % (w/w). In some cases, the concentration of total monomer in the solution is about 3 % (w/w) to about 5 % (w/w), about 3 % (w/w) to about 7 % (w/w), about 3 % (w/w) to about 10 % (w/w), about 3 % (w/w) to about 15 % (w/w), about 3 % (w/w) to about 20 % (w/w), about 3 % (w/w) to about 25 % (w/w), about 3 % (w/w) to about 30 % (w/w), about 5 % (w/w) to about 7 % (w/w), about 5 % (w/w) to about 10 % (w/w), about 5 % (w/w) to about 15 % (w/w), about 5 % (w/w) to about 20 % (w/w), about 5 % (w/w) to about 25 % (w/w), about 5 % (w/w) to about 30 % (w/w), about 7 % (w/w) to about 10 % (w/w), about 7 % (w/w) to about 15 % (w/w), about 7 % (w/w) to about 20 % (w/w), about 7 % (w/w) to about 25 % (w/w), about 7 % (w/w) to about 30 % (w/w), about 10 % (w/w) to about 15 % (w/w), about 10 % (w/w) to about 20 % (w/w), about 10 % (w/w) to about 25 % (w/w), about 10 % (w/w) to about 30 % (w/w), about 15 % (w/w) to about 20 % (w/w), about 15 % (w/w) to about 25 % (w/w), about 15 % (w/w) to about 30 % (w/w), about 20 % (w/w) to about 25 % (w/w), about 20 % (w/w) to about 30 % (w/w), or about 25 % (w/w) to about 30 % (w/w). In some cases, the concentration of total monomer in the solution is about 3 % (w/w), about 5 % (w/w), about 7 % (w/w), about 10 % (w/w), about 15 % (w/w), about 20 % (w/w), about 25 % (w/w), or about 30 % (w/w). In some cases, the concentration of total monomer in the solution is at least about 3 % (w/w), about 5 % (w/w), about 7 % (w/w), about 10 % (w/w), about 15 % (w/w), about 20 % (w/w), or about 25 % (w/w). In some cases, the concentration of total monomer in the solution is at most about 5 % (w/w), about 7 % (w/w), about 10 % (w/w), about 15 % (w/w), about 20 % (w/w), about 25 % (w/w), or about 30 % (w/w). In some cases, the solution comprises a single monomer, two monomers, three monomers, four monomers, five monomers, or more monomers. For example, two or more different monomers may be combined in the solution used for solution blow spinning a particular desired polymer fiber.

The air or gas humidity should be kept to a minimum to allow for optimal solvent evaporation. In some cases, the air or gas humidity is about 0 % relative humidity (rh) to about 60 % rh. In some cases, the air or gas humidity is about 0 % rh to about 5 % rh, about 0 % rh to about 10 % rh, about 0 % rh to about 15 % rh, about 0 % rh to about 20 % rh, about 0 % rh to about 25 % rh, about 0 % rh to about 30 % rh, about 0 % rh to about 35 % rh, about 0 % rh to about 40 % rh, about 0 % rh to about 45 % rh, about 0 % rh to about 50 % rh, about 0 % rh to about 60 % rh, about 5 % rh to about 10 % rh, about 5 % rh to about 15 % rh, about 5 % rh to about 20 % rh, about 5 % rh to about 25 % rh, about 5 % rh to about 30 % rh, about 5 % rh to about 35 % rh, about 5 % rh to about 40 % rh, about 5 % rh to about 45 % rh, about 5 % rh to about 50 % rh, about 5 % rh to about 60 % rh, about 10 % rh to about 15 % rh, about 10 % rh to about 20 % rh, about 10 % rh to about 25 % rh, about 10 % rh to about 30 % rh, about 10 % rh to about 35 % rh, about 10 % rh to about 40 % rh, about 10 % rh to about 45 % rh, about 10 % rh to about 50 % rh, about 10 % rh to about 60 % rh, about 15 % rh to about 20 % rh, about 15 % rh to about 25 % rh, about 15 % rh to about 30 % rh, about 15 % rh to about 35 % rh, about 15 % rh to about 40 % rh, about 15 % rh to about 45 % rh, about 15 % rh to about 50 % rh, about 15 % rh to about 60 % rh, about 20 % rh to about 25 % rh, about 20 % rh to about 30 % rh, about 20 % rh to about 35 % rh, about 20 % rh to about 40 % rh, about 20 % rh to about 45 % rh, about 20 % rh to about 50 % rh, about 20 % rh to about 60 % rh, about 25 % rh to about 30 % rh, about 25 % rh to about 35 % rh, about 25 % rh to about 40 % rh, about 25 % rh to about 45 % rh, about 25 % rh to about 50 % rh, about 25 % rh to about 60 % rh, about 30 % rh to about 35 % rh, about 30 % rh to about 40 % rh, about 30 % rh to about 45 % rh, about 30 % rh to about 50 % rh, about 30 % rh to about 60 % rh, about 35 % rh to about 40 % rh, about 35 % rh to about 45 % rh, about 35 % rh to about 50 % rh, about 35 % rh to about 60 % rh, about 40 % rh to about 45 % rh, about 40 % rh to about 50 % rh, about 40 % rh to about 60 % rh, about 45 % rh to about 50 % rh, about 45 % rh to about 60 % rh, or about 50 % rh to about 60 % rh. In some cases, the air or gas humidity is about 0 % rh, about 5 % rh, about 10 % rh, about 15 % rh, about 20 % rh, about 25 % rh, about 30 % rh, about 35 % rh, about 40 % rh, about 45 % rh, about 50 % rh, or about 60 % rh. In some cases, the air or gas humidity is at least about 0 % rh, about 5 % rh, about 10 % rh, about 15 % rh, about 20 % rh, about 25 % rh, about 30 % rh, about 35 % rh, about 40 % rh, about 45 % rh, or about 50 % rh. In some cases, the air or gas humidity is at most about 5 % rh, about 10 % rh, about 15 % rh, about 20 % rh, about 25 % rh, about 30 % rh, about 35 % rh, about 40 % rh, about 45 % rh, about 50 % rh, or about 60 % rh.

The air or gas pressure should be optimized for monomer flow and Taylor cone development. In some cases, the air or gas pressure is about 10 pounds per square inch (psi) to about 100 psi per nozzle. In some cases, the air or gas pressure is about 10 psi to about 20 psi, about 10 psi to about 30 psi, about 10 psi to about 40 psi, about 10 psi to about 50 psi, about 10 psi to about 60 psi, about 10 psi to about 70 psi, about 10 psi to about 80 psi, about 10 psi to about 90 psi, about 10 psi to about 100 psi, about 20 psi to about 30 psi, about 20 psi to about 40 psi, about 20 psi to about 50 psi, about 20 psi to about 60 psi, about 20 psi to about 70 psi, about 20 psi to about 80 psi, about 20 psi to about 90 psi, about 20 psi to about 100 psi, about 30 psi to about 40 psi, about 30 psi to about 50 psi, about 30 psi to about 60 psi, about 30 psi to about 70 psi, about 30 psi to about 80 psi, about 30 psi to about 90 psi, about 30 psi to about 100 psi, about 40 psi to about 50 psi, about 40 psi to about 60 psi, about 40 psi to about 70 psi, about 40 psi to about 80 psi, about 40 psi to about 90 psi, about 40 psi to about 100 psi, about 50 psi to about 60 psi, about 50 psi to about 70 psi, about 50 psi to about 80 psi, about 50 psi to about 90 psi, about 50 psi to about 100 psi, about 60 psi to about 70 psi, about 60 psi to about 80 psi, about 60 psi to about 90 psi, about 60 psi to about 100 psi, about 70 psi to about 80 psi, about 70 psi to about 90 psi, about 70 psi to about 100 psi, about 80 psi to about 90 psi, about 80 psi to about 100 psi, or about 90 psi to about 100 psi per nozzle. In some cases, the air or gas pressure is about 10 psi, about 20 psi, about 30 psi, about 40 psi, about 50 psi, about 60 psi, about 70 psi, about 80 psi, about 90 psi, or about 100 psi per nozzle. In some cases, the air or gas pressure is at least about 10 psi, about 20 psi, about 30 psi, about 40 psi, about 50 psi, about 60 psi, about 70 psi, about 80 psi, or about 90 psi. In some cases, the air or gas pressure is at most about 20 psi, about 30 psi, about 40 psi, about 50 psi, about 60 psi, about 70 psi, about 80 psi, about 90 psi, or about 100 psi per nozzle.

The temperate of air, gas, and monomer solution should be as hot as possible to allow for solvent evaporation without degrading the solution. In some cases, the temperature of air, gas, and monomer solution is about 25 °C to about 99 °C. In some cases, the temperature of air, gas, and monomer solution is about 25 °C to about 35 °C, about 25 °C to about 45 °C, about 25 °C to about 50 °C, about 25 °C to about 55 °C, about 25 °C to about 60 °C, about 25 °C to about 65 °C, about 25 °C to about 70 °C, about 25 °C to about 80 °C, about 25 °C to about 90 °C, about 25 °C to about 99 °C, about 35 °C to about 45 °C, about 35 °C to about 50 °C, about 35 °C to about 55 °C, about 35 °C to about 60 °C, about 35 °C to about 65 °C, about 35 °C to about 70 °C, about 35 °C to about 80 °C, about 35 °C to about 90 °C, about 35° C to about 99 °C, about 45 °C to about 50 °C, about 45 °C to about 55° C, about 45 °C to about 60 °C, about 45 °C to about 65 °C, about 45 °C to about 70 °C, about 45 °C to about 80 °C, about 45 °C to about 90 °C, about 45 °C to about 99 °C, about 50 °C to about 55 °C, about 50 °C to about 60 °C, about 50 °C to about 65 °C, about 50 °C to about 70 °C, about 50 °C to about 80 °C, about 50 °C to about 90 °C, about 50 °C to about 99 °C, about 55 °C to about 60 °C, about 55 °C to about 65 °C, about 55 °C to about 70 °C, about 55 °C to about 80 °C, about 55 °C to about 90 °C, about 55 °C to about 99° C, about 60 °C to about 65 °C, about 60 °C to about 70 °C, about 60 °C to about 80 °C, about 60 °C to about 90 °C, about 60 °C to about 99 °C, about 65 °C to about 70 °C, about 65 °C to about 80 °C, about 65 °C to about 90° C, about 65 °C to about 99 °C, about 70 °C to about 80 °C, about 70 °C to about 90 °C, about 70 °C to about 99 °C, about 80 °C to about 90 °C, about 80 °C to about 99 °C, or about 90 °C to about 99 °C. In some cases, the temperature of air, gas, and monomer solution is about 25 °C, about 35 °C, about 45 °C, about 50 °C, about 55 °C, about 60 °C, about 65 °C, about 70 °C, about 80 °C, about 90 °C, or about 99 °C. In some cases, the temperature of air, gas, and monomer solution is at least about 25 °C, about 35 °C, about 45 °C, about 50 °C, about 55 °C, about 60 °C, about 65 °C, about 70 °C, about 80 °C, or about 90 °C. In some cases, the temperature of air, gas, and monomer solution is at most about 35 °C, about 45 °C, about 50 °C, about 55 °C, about 60 °C, about 65 °C, about 70 °C, about 80 °C, about 90 °C, or about 99 °C.

The distance from nozzle to the collector should allow for near complete solvent evaporation. In some cases, the distance from nozzle to the collector is about 20 cm to about 150 cm. In some cases, the distance from nozzle to the collector is about 20 cm to about 30 cm, about 20 cm to about 50 cm, about 20 cm to about 50 cm, about 20 cm to about 70 cm, about 20 cm to about 90 cm, about 20 cm to about 110 cm, about 20 cm to about 130 cm, about 20 cm to about 150 cm, about 30 cm to about 50 cm, about 30 cm to about 50 cm, about 30 cm to about 70 cm, about 30 cm to about 90 cm, about 30 cm to about 110 cm, about 30 cm to about 130 cm, about 30 cm to about 150 cm, about 50 cm to about 50 cm, about 50 cm to about 70 cm, about 50 cm to about 90 cm, about 50 cm to about 110 cm, about 50 cm to about 130 cm, about 50 cm to about 150 cm, about 50 cm to about 70 cm, about 50 cm to about 90 cm, about 50 cm to about 110 cm, about 50 cm to about 130 cm, about 50 cm to about 150 cm, about 70 cm to about 90 cm, about 70 cm to about 110 cm, about 70 cm to about 130 cm, about 70 cm to about 150 cm, about 90 cm to about 110 cm, about 90 cm to about 130 cm, about 90 cm to about 150 cm, about 110 cm to about 130 cm, about 110 cm to about 150 cm, or about 130 cm to about 150 cm. In some cases, the distance from nozzle to the collector is about 20 cm, about 30 cm, about 50 cm, about 50 cm, about 70 cm, about 90 cm, about 110 cm, about 130 cm, or about 150 cm. In some cases, the distance from nozzle to the collector is at least about 20 cm, about 30 cm, about 50 cm, about 50 cm, about 70 cm, about 90 cm, about 110 cm, or about 130 cm. In some cases, the distance from nozzle to the collector is at most about 30 cm, about 50 cm, about 50 cm, about 70 cm, about 90 cm, about 110 cm, about 130 cm, or about 150 cm.

In some cases, the systems and methods described herein generate fibers having a certain diameter or diameter range. In some cases, the fiber has a diameter of about 0.1 nm to about 500 nm. In some cases, the fiber has a diameter of about 0.1 nm to about 0.5 nm, about 0.1 nm to about 1 nm, about 0.1 nm to about 5 nm, about 0.1 nm to about 10 nm, about 0.1 nm to about 50 nm, about 0.1 nm to about 100 nm, about 0.1 nm to about 500 nm, about 0.1 nm to about 1 nm, about 0.1 nm to about 5 nm, about 0.1 nm to about 10 nm, about 0.1 nm to about 50 nm, about 0.5 nm to about 1 nm, about 0.5 nm to about 5 nm, about 0.5 nm to about 10 nm, about 0.5 nm to about 50 nm, about 0.5 nm to about 100 nm, about 0.5 nm to about 500 nm, about 0.5 nm to about 1 nm, about 0.5 nm to about 5 nm, about 0.5 nm to about 10 nm, about 0.5 nm to about 50 nm, about 1 nm to about 5 nm, about 1 nm to about 10 nm, about 1 nm to about 50 nm, about 1 nm to about 100 nm, about 1 nm to about 500 nm, about 1 nm to about 1 nm, about 1 nm to about 5 nm, about 1 nm to about 10 nm, about 1 nm to about 50 nm, about 5 nm to about 10 nm, about 5 nm to about 50 nm, about 5 nm to about 100 nm, about 5 nm to about 500 nm, about 5 nm to about 1 nm, about 5 nm to about 5 nm, about 5 nm to about 10 nm, about 5 nm to about 50 nm, about 10 nm to about 50 nm, about 10 nm to about 100 nm, about 10 nm to about 500 nm, about 10 nm to about 1 nm, about 10 nm to about 5 nm, about 10 nm to about 10 nm, about 10 nm to about 50 nm, about 50 nm to about 100 nm, about 50 nm to about 500 nm, about 50 nm to about 1 nm, about 50 nm to about 5 nm, about 50 nm to about 10 nm, about 50 nm to about 50 nm, about 100 nm to about 500 nm, about 100 nm to about 1 nm, about 100 nm to about 5 nm, about 100 nm to about 10 nm, about 100 nm to about 50 nm, about 500 nm to about 1 nm, about 500 nm to about 5 nm, about 500 nm to about 10 nm, about 500 nm to about 50 nm, about 1 nm to about 5 nm, about 1 nm to about 10 nm, about 1 nm to about 50 nm, about 5 nm to about 10 nm, about 5 nm to about 50 nm, or about 10 nm to about 50 nm. In some cases, the fiber has a diameter of about 0.1 nm, about 0.5 nm, about 1 nm, about 5 nm, about 10 nm, about 50 nm, about 100 nm, about 500 nm, about 1 nm, about 5 nm, about 10 nm, or about 50 nm. In some cases, the fiber has a diameter of at least about 0.1 nm, about 0.5 nm, about 1 nm, about 5 nm, about 10 nm, about 50 nm, about 100 nm, about 500 nm, about 1 nm, about 5 nm, or about 10 nm. In some cases, the fiber has a diameter of at most about 0.5 nm, about 1 nm, about 5 nm, about 10 nm, about 50 nm, about 100 nm, about 500 nm, about 1 nm, about 5 nm, about 10 nm, or about 50 nm.

The blow spinning and other spinning processes disclosed herein can be used to make scaffolds. Scaffolds can comprise 3D structures that act as a point of attachment for other ingredients of the edible composition. In some cases, cells, fats, proteins, carbohydrates, or other polymers can attach to scaffolds. In some cases, the scaffolds comprise polymer fibers or nanofibers generated using blow spinning, electrospinning, or other spinning processes disclosed herein.

The ingredients (e.g., monomers or other molecules) can include proteins or protein derivatives (gelatin, collagen, elastin, silk, soy, zein, gliadin, hordein, amaranth, casein, wheat, whey, avenin, secalin, corn protein meal, ovomucin, ovotransferrin, ovalbumin, 8S mung bean protein, and algae and marine source proteins, etc.) and/or polysaccharides (chitosan, starch, alginate, cellulose and cellulose derivatives, pullulan, dextran, cyclodextrins, agar, agarose, konjac, pectin, carrageenan, alginate, xanthan, Guar, locust bean gum, Tara gum, gum tragacanth, gum Arabic, fenugreek gum, potato starch, pea starch, Hylon 7 corn starch, hemicellulose, altanin, etc.). Non-limiting examples of acceptable proteins or protein derivatives include collagen, elastin, silk, soy derivatives, zein, gliadin, hordein, casein, wheat derivatives, gluten, whey, yeast extracts, kafirin, millet derivatives, quinoa derivatives, bean derivatives, textured vegetable proteins, egg white proteins, sea vegetable derivatives, algae proteins, transglutaminase, glutamic acid derivatives, gelatin, fish source proteins, pea proteins, or any hydrolysates thereof, any isolates thereof, or any combination thereof. These ingredients can be used pure or mixed together at any ratios. In some cases, the solution containing the ingredient(s) before the spinning process begins comprises at least two, at least three, at least four, at least five, at least six, at least seven, at least eight, at least nine, or at least ten or more ingredients. These ingredients are often dissolved in volatile solvents including ethanol, acetic acid, formic acid, acetone, ethyl acetate, or other suitable solvents. In some instances, before the ingredients are dissolved in volatile solvents, they are dissolved at a higher concentration in a small amount of DMSO or water. The ingredients may also be dissolved in non-volatile solvents, such as water, salt water, or various oils.

In some cases, the edible composition comprises the protein or protein derivatives at a concentration of about 1 % (w/w) to about 80 % (w/w). In some cases, the edible composition comprises the protein or protein derivatives at a concentration of about 1 % (w/w) to about 5 % (w/w), about 1 % (w/w) to about 10 % (w/w), about 1 % (w/w) to about 20 % (w/w), about 1 % (w/w) to about 30 % (w/w), about 1 % (w/w) to about 40 % (w/w), about 1 % (w/w) to about 50 % (w/w), about 1 % (w/w) to about 60 % (w/w), about 1 % (w/w) to about 70 % (w/w), about 1 % (w/w) to about 80 % (w/w), about 5 % (w/w) to about 10 % (w/w), about 5 % (w/w) to about 20 % (w/w), about 5 % (w/w) to about 30 % (w/w), about 5 % (w/w) to about 40 % (w/w), about 5 % (w/w) to about 50 % (w/w), about 5 % (w/w) to about 60 % (w/w), about 5 % (w/w) to about 70 % (w/w), about 5 % (w/w) to about 80 % (w/w), about 10 % (w/w) to about 20 % (w/w), about 10 % (w/w) to about 30 % (w/w), about 10 % (w/w) to about 40 % (w/w), about 10 % (w/w) to about 50 % (w/w), about 10 % (w/w) to about 60 % (w/w), about 10 % (w/w) to about 70 % (w/w), about 10 % (w/w) to about 80 % (w/w), about 20 % (w/w) to about 30 % (w/w), about 20 % (w/w) to about 40 % (w/w), about 20 % (w/w) to about 50 % (w/w), about 20 % (w/w) to about 60 % (w/w), about 20 % (w/w) to about 70 % (w/w), about 20 % (w/w) to about 80 % (w/w), about 30 % (w/w) to about 40 % (w/w), about 30 % (w/w) to about 50 % (w/w), about 30 % (w/w) to about 60 % (w/w), about 30 % (w/w) to about 70 % (w/w), about 30 % (w/w) to about 80 % (w/w), about 40 % (w/w) to about 50 % (w/w), about 40 % (w/w) to about 60 % (w/w), about 40 % (w/w) to about 70 % (w/w), about 40 % (w/w) to about 80 % (w/w), about 50 % (w/w) to about 60 % (w/w), about 50 % (w/w) to about 70 % (w/w), about 50 % (w/w) to about 80 % (w/w), about 60 % (w/w) to about 70 % (w/w), about 60 % (w/w) to about 80 % (w/w), or about 70 % (w/w) to about 80 % (w/w). In some cases, the edible composition comprises the protein or protein derivatives at a concentration of about 1 % (w/w), about 5 % (w/w), about 10 % (w/w), about 20 % (w/w), about 30 % (w/w), about 40 % (w/w), about 50 % (w/w), about 60 % (w/w), about 70 % (w/w), or about 80 % (w/w). In some cases, the edible composition comprises the protein or protein derivatives at a concentration of at least about 1 % (w/w), about 5 % (w/w), about 10 % (w/w), about 20 % (w/w), about 30 % (w/w), about 40 % (w/w), about 50 % (w/w), about 60 % (w/w), or about 70 % (w/w). In some cases, the edible composition comprises the protein or protein derivatives at a concentration of at most about 5 % (w/w), about 10 % (w/w), about 20 % (w/w), about 30 % (w/w), about 40 % (w/w), about 50 % (w/w), about 60 % (w/w), about 70 % (w/w), or about 80 % (w/w). In some cases, the protein or protein derivative is present at a concentration of above about 80% (w/w), about 90% (w/w), or about 95% (w/w). High amounts of proteins or protein derivatives may be present in certain edible compositions meant to imitate or approximate high-protein food products such as, for example, low-fat lean meat or meat jerky. Examples of low-fat mean include lean ground beef which is at least 92% lean with 8% fat or less and extra lean ground beef that is at least 96% lean with 4% fat or less.

In some cases, the edible composition comprises the polysaccharides at a concentration of about 1 % (w/w) to about 80 % (w/w). In some cases, the edible composition comprises the polysaccharides at a concentration of about 1 % (w/w) to about 5 % (w/w), about 1 % (w/w) to about 10 % (w/w), about 1 % (w/w) to about 20 % (w/w), about 1 % (w/w) to about 30 % (w/w), about 1 % (w/w) to about 40 % (w/w), about 1 % (w/w) to about 50 % (w/w), about 1 % (w/w) to about 60 % (w/w), about 1 % (w/w) to about 70 % (w/w), about 1 % (w/w) to about 80 % (w/w), about 5 % (w/w) to about 10 % (w/w), about 5 % (w/w) to about 20 % (w/w), about 5 % (w/w) to about 30 % (w/w), about 5 % (w/w) to about 40 % (w/w), about 5 % (w/w) to about 50 % (w/w), about 5 % (w/w) to about 60 % (w/w), about 5 % (w/w) to about 70 % (w/w), about 5 % (w/w) to about 80 % (w/w), about 10 % (w/w) to about 20 % (w/w), about 10 % (w/w) to about 30 % (w/w), about 10 % (w/w) to about 40 % (w/w), about 10 % (w/w) to about 50 % (w/w), about 10 % (w/w) to about 60 % (w/w), about 10 % (w/w) to about 70 % (w/w), about 10 % (w/w) to about 80 % (w/w), about 20 % (w/w) to about 30 % (w/w), about 20 % (w/w) to about 40 % (w/w), about 20 % (w/w) to about 50 % (w/w), about 20 % (w/w) to about 60 % (w/w), about 20 % (w/w) to about 70 % (w/w), about 20 % (w/w) to about 80 % (w/w), about 30 % (w/w) to about 40 % (w/w), about 30 % (w/w) to about 50 % (w/w), about 30 % (w/w) to about 60 % (w/w), about 30 % (w/w) to about 70 % (w/w), about 30 % (w/w) to about 80 % (w/w), about 40 % (w/w) to about 50 % (w/w), about 40 % (w/w) to about 60 % (w/w), about 40 % (w/w) to about 70 % (w/w), about 40 % (w/w) to about 80 % (w/w), about 50 % (w/w) to about 60 % (w/w), about 50 % (w/w) to about 70 % (w/w), about 50 % (w/w) to about 80 % (w/w), about 60 % (w/w) to about 70 % (w/w), about 60 % (w/w) to about 80 % (w/w), or about 70 % (w/w) to about 80 % (w/w). In some cases, the edible composition comprises the polysaccharides at a concentration of about 1 % (w/w), about 5 % (w/w), about 10 % (w/w), about 20 % (w/w), about 30 % (w/w), about 40 % (w/w), about 50 % (w/w), about 60 % (w/w), about 70 % (w/w), or about 80 % (w/w). In some cases, the edible composition comprises the polysaccharides at a concentration of at least about 1 % (w/w), about 5 % (w/w), about 10 % (w/w), about 20 % (w/w), about 30 % (w/w), about 40 % (w/w), about 50 % (w/w), about 60 % (w/w), or about 70 % (w/w). In some cases, the edible composition comprises the polysaccharides at a concentration of at most about 5 % (w/w), about 10 % (w/w), about 20 % (w/w), about 30 % (w/w), about 40 % (w/w), about 50 % (w/w), about 60 % (w/w), about 70 % (w/w), or about 80 % (w/w).

The edible composition can also include additional molecules, for example other polymers, such as block copolymers or block copolymer intermediates, or the monomers that make up the block copolymers. Examples of block copolymers include modified poly(ethylene glycol), modified ethylene glycol monomer, polyester, poly(caprolactone), polylactic acid, poly(lactic-co-glycolic acid), polyethylene oxide, polyethylene glycol, poly(hydroxybutyrate), poly(ester urethane), polyvinyl alcohol, poloxamer, or any combination thereof. In some cases, the edible composition comprises the other polymers at a concentration of about 1 % (w/w) to about 80 % (w/w). In some cases, the edible composition comprises the other polymers at a concentration of about 1 % (w/w) to about 5 % (w/w), about 1 % (w/w) to about 10 % (w/w), about 1 % (w/w) to about 20 % (w/w), about 1 % (w/w) to about 30 % (w/w), about 1 % (w/w) to about 40 % (w/w), about 1 % (w/w) to about 50 % (w/w), about 1 % (w/w) to about 60 % (w/w), about 1 % (w/w) to about 70 % (w/w), about 1 % (w/w) to about 80 % (w/w), about 5 % (w/w) to about 10 % (w/w), about 5 % (w/w) to about 20 % (w/w), about 5 % (w/w) to about 30 % (w/w), about 5 % (w/w) to about 40 % (w/w), about 5 % (w/w) to about 50 % (w/w), about 5 % (w/w) to about 60 % (w/w), about 5 % (w/w) to about 70 % (w/w), about 5 % (w/w) to about 80 % (w/w), about 10 % (w/w) to about 20 % (w/w), about 10 % (w/w) to about 30 % (w/w), about 10 % (w/w) to about 40 % (w/w), about 10 % (w/w) to about 50 % (w/w), about 10 % (w/w) to about 60 % (w/w), about 10 % (w/w) to about 70 % (w/w), about 10 % (w/w) to about 80 % (w/w), about 20 % (w/w) to about 30 % (w/w), about 20 % (w/w) to about 40 % (w/w), about 20 % (w/w) to about 50 % (w/w), about 20 % (w/w) to about 60 % (w/w), about 20 % (w/w) to about 70 % (w/w), about 20 % (w/w) to about 80 % (w/w), about 30 % (w/w) to about 40 % (w/w), about 30 % (w/w) to about 50 % (w/w), about 30 % (w/w) to about 60 % (w/w), about 30 % (w/w) to about 70 % (w/w), about 30 % (w/w) to about 80 % (w/w), about 40 % (w/w) to about 50 % (w/w), about 40 % (w/w) to about 60 % (w/w), about 40 % (w/w) to about 70 % (w/w), about 40 % (w/w) to about 80 % (w/w), about 50 % (w/w) to about 60 % (w/w), about 50 % (w/w) to about 70 % (w/w), about 50 % (w/w) to about 80 % (w/w), about 60 % (w/w) to about 70 % (w/w), about 60 % (w/w) to about 80 % (w/w), or about 70 % (w/w) to about 80 % (w/w). In some cases, the edible composition comprises the other polymers at a concentration of about 1 % (w/w), about 5 % (w/w), about 10 % (w/w), about 20 % (w/w), about 30 % (w/w), about 40 % (w/w), about 50 % (w/w), about 60 % (w/w), about 70 % (w/w), or about 80 % (w/w). In some cases, the edible composition comprises the other polymers at a concentration of at least about 1 % (w/w), about 5 % (w/w), about 10 % (w/w), about 20 % (w/w), about 30 % (w/w), about 40 % (w/w), about 50 % (w/w), about 60 % (w/w), or about 70 % (w/w). In some cases, the edible composition comprises the other polymers at a concentration of at most about 5 % (w/w), about 10 % (w/w), about 20 % (w/w), about 30 % (w/w), about 40 % (w/w), about 50 % (w/w), about 60 % (w/w), about 70 % (w/w), or about 80 % (w/w).

The ingredients can include cells such as cultured or non-cultured cells. In some cases, the cells are added to the other ingredients such as monomers before, during, or after the spinning process by which the polymers or fibers are generated. The polymers or fibers can be generated as scaffolding to be used in a food product. For example, cultured cells may be deposited onto the scaffolds or polymer fibers after they are generated via solution blow spinning. In some cases, the scaffolds or polymer fibers comprising the deposited cells may be further cultured or incubated under suitable conditions to further grow or culture the cells. In the case of food products using cultured cells, any ingredients that are added to the scaffolds or polymer fibers prior to, during, or after deposition of the cells may be selected to ensure the scaffolds or polymer fibers remain biocompatible. In some cases, the deposited cells are not fully differentiated upon deposition, and are subsequently differentiated after attachment or deposition to the scaffolding. For example, the scaffolds or fibers with deposited cells may be transferred to an incubator or bioreactor to facilitate attachment, growth, differentiation, or any combination thereof. In some cases, no cells are included as ingredients in the edible composition. Edible compositions not including any cells (cultured or un-cultured), the scaffolds and/or ingredients may not have to be biocompatible. The scaffolding can comprise

Before, during, or after solution blow spinning, the ingredients or fiber scaffolds can be further cross-linked using various physical processes such as UV photopolymerization, heat, or chemicals (e.g., tannic acid, divalent cations, genipin, glutaraldehyde, N, N-(3-dimethylaminopropyl)-N' ethyl-carbodi-imide hydrochloride (EDC), N-hydroxysuccinimide (NHS), Tyrosinase. polyphenoloxidase, citric acid, alginic acid, etc.). The cross-linking can be used to enhance the association or interaction between individual fibers in the scaffold, between the ingredients, or between the fibers and the ingredients. In some cases, the cross-linking helps modulate properties of the fibrous material or final edible composition such as, for example, texture, consistency, and/or taste.

Various processing conditions are consistent with the present disclosure and can include, for example, monomer concentrations, mixing ratios, solvation processes, humidity, gas or air pressure, monomer pumping speeds, nozzle diameters, number of nozzles, nozzle sizes, internozzle distances, multiple concentric nozzles, distances from nozzle to collector (working distance), collector geometry, collector rotational speed, and temperatures of monomer and solvent.

The approaches for generating fibers described herein have various applications. Non-limiting examples of applications include: creation of scaffolds alone (e.g., aligned or unaligned fibers) for food, scaffolds combined with cells (e.g., added after scaffold spinning or added to the stream of materials during spinning) to foster cell survival, elongation, differentiation, or other desired outcome or property for food or tissue engineering, combining the food or tissue engineered tissues with other cells and tissues (such as, e.g., muscle, fats, fibrous tissue combined to make a biomimetic steak), creation of edible wrappings for food and/or spraying of food or agricultural products to enhance flavor, texture, and/or reducing spoilage, through prevention of oxygen and moisture penetration, cutting the fibers to be shorter using a mixer or chopper or blender to then be added to fatty acids or other products to stablilize emulsifications and improve viscosities in margarines, pastries, shampoo, body lotions, and other fat-based products or emulsions. The above examples illustrate non-limiting embodiments of the present disclosure, and additional applications are contemplated. The present disclosure enables the application of electrospinning, blow spinning, and/or tissue engineering to create fibers that make up or are used to produce various food products and household goods.

In some cases, the method for generating fibers comprises solubilizing at least one monomer in a volatile solvent to generate a solution, and expelling the solution through a blow spinner to generate a polymeric fiber. The solution can be checked to ensure a certain level of viscosity. For example, the viscosity of the solution can affect the fiber diameter. Accordingly, the viscosity of the solution can be adjusted by altering the concentration of the at least one monomer. Other factors that impact fiber diameter include the various processing conditions described herein such as, for example, nozzle size, pumping speed, working distance, etc. In some cases, the method for generating fibers comprises adjusting spinning parameters until the fibers generated have a desired diameter or other property. The method can further comprise seeding cells onto the fibers.

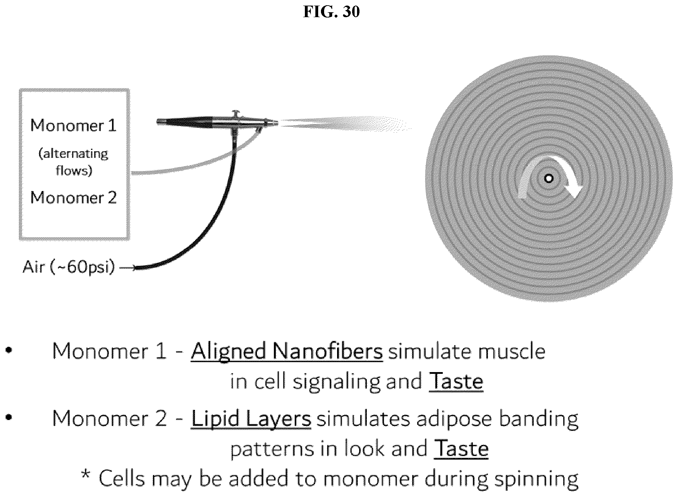

In some cases, multiple different fibers are generated at the same time and/or are combined to produce a food product. In some cases, multiple fibers (of the same or different composition) are combined to produce the food product. **FIG. 30** illustrates one embodiment of this process. As shown, two different monomer solutions are used during the spinning process. The fibers that are generated are rolled up by the collector. Monomer 1 comprises nanofibers that are aligned in order to mimic the banding patterns of muscle fibers so as to simulate muscle in taste and/or texture. The nanofibers can include muscle cells that are seeded onto the fibers. In some cases, the nanofibers comprise molecules that stimulate seeded cells to adhere, grow, differentiate, or any combination thereof. The nanofibers or polymer scaffolds can stimulate a population of seeded cells to differentiate into different cell types, for example, certain nanofibers may comprise differentiation factors or drivers of differentiation (e.g., a signaling molecule that drives differentiation or differentiation according to the inducible systems disclosed herein). The cells can be seeded in the monomer solutions, mixed with the nanofibers during the spinning process, and/or onto the nanofibers after they have been generated. As shown in **FIG. 30****,** the spinning process can utilize monomer 2 comprising lipid layer(s) to simulate adipose banding patterns in look and taste. The different monomers can be spun at the same time, e.g., simultaneously, to create mixed or entangled fibers of varying content (e.g., having fibers with muscle and fibers with adipose cells). **FIG. 30** shows a process by which monomer 1 and monomer 2 are both added simultaneously or via alternating flows to produce a mix or alternating pattern of the two monomers. A mix or alternating pattern of the two monomers can be used to produce food products that mimic certain food patterns such as, for example, high quality steaks that have high degree of marbling. In some cases, the alternating pattern of monomers containing or mimicking meat is varied (e.g., in length and/or thickness) to mimic a natural marbling pattern. For example, a brief snapshot of the spinning process can include generating a fiber or layer comprising 2 cm adipose fiber, 3 cm muscle fiber, 1 cm adipose fiber, and 2.5 cm muscle fiber. In some cases, the spinning system is configured to produce non-homogeneous fibers or fiber compositions. Alternatively or in combination, the spinning system is configured to produce homogeneous fibers or fiber compositions.

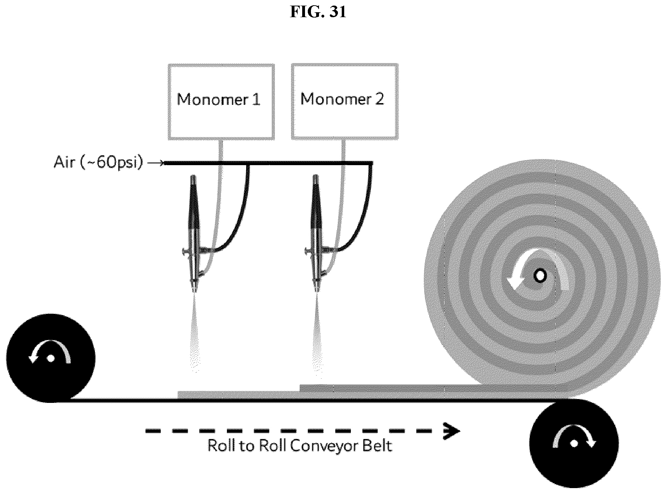

**FIG. 31** provides another illustrative approach for generating fibers. Monomer solution 1 is spun onto a conveyor belt surface to create a first layer of fibers, and a second monomer solution 2 is then spun onto the first layer of fibers to generate a second layer of fibers. Accordingly, **FIG. 31** shows a process by which monomer 1 and monomer 2 are added as separate layers to produce a layered food product. This process can be used to mimic certain food patterns such as, for example, bacon which often has alternating layers of fat and muscle. Cultured or non-cultured cells and/or tissues can be used to seed or are combined with the fibers and layers described herein to generate various edible compositions. In some instances, the solution comprises ingredients configured to provide texture or other desired properties. The ingredients can include at least one texturizer, bulking agent or thickener, preservative, flavor enhancer, antimicrobial agent, salt, pH modulator, desiccant, oxidizing or reducing agent, sweetener, humectant, drying agent, antioxidant, vitamin, mineral, curing or pickling agent, enzyme, or coloring agent (e.g., to impart, preserve, or enhance color).

Disclosed herein are various uses of cells as ingredients in edible food compositions or products. In some cases, the cells are processed to obtain one or more ingredients used in a food product. For example, cells may be cultured and then processed to extract the protein, fat, carbohydrate, or other biomolecules for use as an ingredient in a food production process such as blow spinning, casting, extrusion, or any method known in the field of food manufacturing. In this way, the cells are not used in their entirety as living components of the food composition, but rather are harvested, processed (e.g., using lyophilization or fat isolation), and used to create a scaffold / final meat product. In some cases, the final meat product is never seeded with live cells.

In some cases, the scaffolds disclosed herein are biocompatible and are seeded with cells such as those disclosed herein. As an example of this method, collagen created from salmon cell culture is used to create a biomimetic scaffold. Additional salmon cells are then seeded on this scaffold, obviating the need for collagen, gelatin, or other materials to be isolated from an animal.

In some cases, a single cell population is used to create different tissues (muscle, fat, etc.) by making a scaffold that guides cells down distinct differentiation pathways in different areas of the scaffolding. The cell population may include undifferentiated or partially differentiated cells. The scaffold may have certain growth and/or differentiation embedded or dispersed at distinct areas, which induce differentiation of the same cell population into distinct differentiated cell types corresponding to distinct areas of the scaffold. In this way, an identical initial cell population can be used to generate distinct cell populations corresponding to distinct cell types on the scaffold. In some cases, an area of the scaffold may have differentiation factors for multiple cell types, thus driving differentiation into multiple cell types in the same area to create a homogeneously mixed population.

One method of generating such scaffolds is to use different or alternating ingredients during scaffold production. For example, in blow spinning, alternating ingredients can be used to create a variegated pattern or spatial segregation. Multiple ingredient formulations can be used depending on the cell processes that are to be induced. For example, ingredient formulations can include a myogenic formulation and an adipogenic formulation of blow spinning ingredients. When these are alternated, there will be adipogenic scaffold interspersed between myogenic scaffold to form a combined heterogeneous scaffold configured to induce cellular differentiation into muscle and fat cells, respectively. Whether a cell becomes fat or muscle depends upon whether it is in contact with the adipogenic or myogenic scaffold when it is seeded. In this way, muscle and fat can be created at the same time without an assembly step.

Described herein are edible compositions comprising a biocompatible monomer, and methods of forming and depositing blow spun polymer fibers formed from the described compositions. The composition can comprise a monomer or a blend of monomers. The blow spun polymer fibers can be directly deposited onto various surfaces, thereby generating a polymer fiber composition such as a layer or scaffold. Such polymer fiber compositions can be deposited on a collector such as a rotating drum or cylinder or a flat plate to create fibers. A rotating collector allows for alignment of the fibers as they are deposited/generated, for example, in generating aligned fibers that mimic muscle fibers. Alternatively, a non-rotating collector can be any surface (flat or uneven) that allows collection of non-aligned fibers, which can create a web or tangle of fibers, for example.

Solution blow spinning can require an apparatus for expelling the monomer solution(s) and subjecting the solution to air or gas pressure, a concentrated monomer solution in a volatile solvent, and a compressed gas source. Examples of such an apparatus include airbrushes. This process can provide an ease of use and much faster deposition rates as compared to other methods such as electrospinning. Another advantage of solution blow spinning is it lacks the high voltage or conductivity requirements of electrospinning. Moreover, solution blow spinning can provide a portable and efficient alternative to electrospinning that allows direct deposition of polymer fiber compositions onto various surfaces.

According to some aspects of the present disclosure, a system for solution blow spun polymer fibers comprises a concentrated polymer solution, a blow spinning platform, and a high pressure gas source. The airbrush or other apparatus used in solution blow spinning is typically used with a high pressure gas source. The gas source can provide pressurized hydrogen, nitrogen, carbon dioxide, air, another gas or gas mixture, or any combination thereof.

The compositions described herein can be generated from a solution. The solution can include monomers or molecules such as proteins, polysaccharides, fats, or any combination thereof. The solution can also include additional molecules, for example other polymers, such as block copolymers or block copolymer intermediates, or the monomers that make up the block copolymers. Examples of block copolymers include modified poly(ethylene glycol), modified ethylene glycol monomer, polyester, poly(caprolactone), polylactic acid, poly(lactic-co-glycolic acid), polyethylene oxide, polyethylene glycol, poly(hydroxybutyrate), poly(ester urethane), polyvinyl alcohol, poloxamer, or any combination thereof. Examples of proteins include gelatin, collagen, elastin, silk, soy derivatives, zein, gliadin, hordein, casein, wheat derivatives, gluten, whey, millet derivatives, quinoa derivatives, algae proteins, marine source proteins, pea proteins, and other forms of proteins. Examples of polysaccharides include chitosan, carrageenan, starch, agar, tapioca starch, alginate, cellulose and cellulose derivatives, pullulan, dextran, glucomannan, cyclodextrin, agarose, konjac, pectin, carrageenan, alginate, xanthan, guar, locust bean gum, tara gum, gum tragacanth, gum arabic, fenugreek gum, modified starch, potato starch, pea starch, Hylon 7 corn starch, hemicellulose, altanan, or any combination thereof. Further non-limiting examples of polysaccharides are the carbohydrates chitosan, carrageenan, agar. tapioca starch, alginate, cellulose and cellulose derivatives, pullulan, dextran, glucomannan, cyclodextrin, agarose, konjac, pectin, sea vegetable derivatives, alginate, xanthan, guar gum, locust bean gum, tara gum, gum tragacanth, gum arabic, fenugreek gum, modified starch, potato starch, pea starch, Hylon 7 corn starch, hemicellulose, altanan, or any combination thereof. In some cases, the composition comprises one or more biocompatible polymers. The solution often comprises a volatile solvent. The volatile solvent can include acetone, acetic acid, ethyl acetate, formic acid, ethanol, or another suitable volatile solvent. Non-limiting examples of suitable volatile solvents include ethanol, ethyl acetate, methanol, acetic acid, formic acid, phenol, acetone, dimethylformamide (DMF), or any combination thereof.

The solution may also comprise a non-volatile solvent. The non-volatile solvent may include non-volatile solvent comprises water, salt water, or an oil. Examples of suitable salts include sodium chloride, potassium chloride, magnesium chloride, or calcium chloride. The concentration of salt in the salt water may be any concentration. In some cases, the salt in the salt water is present at a concentration of about 1 % (w/v) to about 99% (w/v). In some cases, the salt in the salt water is present at a concentration of about of about 1 % (w/v) to about 2 % (w/v), about 1 % (w/v) to about 3 % (w/v), about 1 % (w/v) to about 4 % (w/v), about 1 % (w/v) to about 5 % (w/v), about 1 % (w/v) to about 10 % (w/v), about 1 % (w/v) to about 20 % (w/v), about 1 % (w/v) to about 30 % (w/v), about 1 % (w/v) to about 40 % (w/v), or about 1 % (w/v) to about 50 % (w/v). In some cases, each the salt in the salt water is present at a concentration of at least about 1 % (w/v), at least about 2 % (w/v), at least about 3 % (w/v), at least about 4 % (w/v), at least about 5 % (w/v), at least about 10 % (w/v), at least about 20 % (w/v), at least about 30 % (w/v), or at least about 40 % (w/v). In some cases, the salt water comprises at least 1%, at least 2%, at least 5%, at least 10%, at least 20%, at least 30%, at least 40%, at least 50%, at least 60%, at least 70%, at least 80%, or at least 90% salt by weight. The oil may be any type of oil. In some cases, the oil comprises a linear, branched, saturated, or unsaturated fatty acid. In some cases, the oil comprises a fatty acid or a fatty acid derivative, such as a fatty ester or fatty alcohol. The fatty acid may be any size fatty acid. In some cases, the oil comprises a C₃-C₅₀ fatty acid. In some cases, the oil comprises a C₃-C₅₀ fatty acid, a C₃-C₄₀ fatty acid, a C₃-C₃₀ fatty acid, a C₃-C₂₀ fatty acid, a C₆-C₅₀ fatty acid, a C₆-C₄₀ fatty acid, a C₆-C₃₀ fatty acid, a C₃-C₂₀ fatty acid, a C₁₀-C₅₀ fatty acid, a C₁₀-C₄₀ fatty acid, or a C₁₀-C₂₀ fatty acid, or any derivative or combination thereof.

The spinning process utilizing the monomer solution can take place at room temperature or at above or below room temperature. In some cases, the solution is heated or cooled. The monomer solution can comprise one or more monomers at various concentrations and in various combinations.

In some cases, each monomer is present at a concentration of about 1 % (w/v) to about 50 % (w/v). In some cases, each monomer is present at a concentration of about 1 % (w/v) to about 2 % (w/v), about 1 % (w/v) to about 3 % (w/v), about 1 % (w/v) to about 4 % (w/v), about 1 % (w/v) to about 5 % (w/v), about 1 % (w/v) to about 10 % (w/v), about 1 % (w/v) to about 20 % (w/v), about 1 % (w/v) to about 30 % (w/v), about 1 % (w/v) to about 40 % (w/v), about 1 % (w/v) to about 50 % (w/v), about 2 % (w/v) to about 3 % (w/v), about 2 % (w/v) to about 4 % (w/v), about 2 % (w/v) to about 5 % (w/v), about 2 % (w/v) to about 10 % (w/v), about 2 % (w/v) to about 20 % (w/v), about 2 % (w/v) to about 30 % (w/v), about 2 % (w/v) to about 40 % (w/v), about 2 % (w/v) to about 50 % (w/v), about 3 % (w/v) to about 4 % (w/v), about 3 % (w/v) to about 5 % (w/v), about 3 % (w/v) to about 10 % (w/v), about 3 % (w/v) to about 20 % (w/v), about 3 % (w/v) to about 30 % (w/v), about 3 % (w/v) to about 40 % (w/v), about 3 % (w/v) to about 50 % (w/v), about 4 % (w/v) to about 5 % (w/v), about 4 % (w/v) to about 10 % (w/v), about 4 % (w/v) to about 20 % (w/v), about 4 % (w/v) to about 30 % (w/v), about 4 % (w/v) to about 40 % (w/v), about 4 % (w/v) to about 50 % (w/v), about 5 % (w/v) to about 10 % (w/v), about 5 % (w/v) to about 20 % (w/v), about 5 % (w/v) to about 30 % (w/v), about 5 % (w/v) to about 40 % (w/v), about 5 % (w/v) to about 50 % (w/v), about 10 % (w/v) to about 20 % (w/v), about 10 % (w/v) to about 30 % (w/v), about 10 % (w/v) to about 40 % (w/v), about 10 % (w/v) to about 50 % (w/v), about 20 % (w/v) to about 30 % (w/v), about 20 % (w/v) to about 40 % (w/v), about 20 % (w/v) to about 50 % (w/v), about 30 % (w/v) to about 40 % (w/v), about 30 % (w/v) to about 50 % (w/v), or about 40 % (w/v) to about 50 % (w/v). In some cases, each monomer is present at a concentration of about 1 % (w/v), about 2 % (w/v), about 3 % (w/v), about 4 % (w/v), about 5 % (w/v), about 10 % (w/v), about 20 % (w/v), about 30 % (w/v), about 40 % (w/v), or about 50 % (w/v). In some cases, each monomer is present at a concentration of at least about 1 % (w/v), about 2 % (w/v), about 3 % (w/v), about 4 % (w/v), about 5 % (w/v), about 10 % (w/v), about 20 % (w/v), about 30 % (w/v), or about 40 % (w/v). In some cases, each monomer is present at a concentration of at most about 2 % (w/v), about 3 % (w/v), about 4 % (w/v), about 5 % (w/v), about 10 % (w/v), about 20 % (w/v), about 30 % (w/v), about 40 % (w/v), or about 50 % (w/v).

As used herein, monomer solutions can refer to solutions containing monomers, polymers, other ingredients, or any combination thereof. Suitable polymers for use in the disclosed solutions and compositions can have a linear, branched, or star structure. The polymers may also be chemically modified to contain side chains or end groups. Examples of side or end groups include aldehyde, epoxide, aliphatic chains, quinones, amines, peptides, amino acids, cyanoacrylates, N-hydroxysulfosuccinimide (NHS), thiols, etc.

Polymer fiber compositions formed in accordance with the present disclosure can be biodegradable and/or biocompatible. The polymer solution's properties can be adjusted to affect the final fiber composition. Accordingly, the concentration, molecular weight, type of solvent, monomer/polymer used, and/or viscosity can be modulated. In addition, the flow properties of the blow spinning platform can also be modulated by adjusting, for example, nozzle size, temperature, gas type and/or flow rate, etc.). Through these approaches, various properties of the polymer fibers can be adjusted, including morphology, diameter size, and thickness.

Adjustments to the fiber morphology through varying one or more parameters in the spinning process are consistent with the present disclosure. Such adjustments can affect mechanical and adhesive properties. For example, adhesive properties can be increased by blending high molecular weight polymers with low molecular weight polymers. The fibers can be modified to achieve a desired property. For example, the polymers or fibers can be modified to contain biologically interacting groups such as proteins, peptides, carbohydrates, lipids, or other molecules. In some cases, the polymer fibers are configured or designed to degrade over time. For example, the fibers may be intended to provide a substrate for the deposition of cultured cells to form a desirable structure (e.g., muscle fibers in a meat product), but may later degrade or disintegrated to leave behind the cells while removing the polymer fibers. The degradation can be modulated by adjusting the various parameters in the spinning process and/or the solution of monomers that is used. For example, the concentration, type of monomer, viscosity, presence or absence of stabilizers, and other suitable parameters may be adjusted to produce polymer fibers having a certain level of stability. In some instances, the polymer fibers are configured to undergo at least 20%, 30%, 40%, 50%, 60%, 70%, 80%, 90%, 95%, or 99% or higher degradation over a period of time following synthesis. The period of time can be within 1, 2, 3, 4, 5, 6, 7, 8, 9, or 10 days or more. The period of time can be within 1, 2, 3, 4, 5, 6, 7, 8, 9, 10, 11, or 12 weeks. The period of time can be within 1, 2, 3, 4, 5, 6, 7, 8, 9, 10, 11, or 12 months.

Described herein are systems and methods for producing food products comprising microfibers and/or nanofibers can be produced by melt spinning, electrospinning, or by melt blowing. Melt spinning includes drawing down extruded strands of melted polymer to reduce the fiber diameter and typically requires ingredients having a viscoelasticity capable of withstanding the forces exerted upon the fibers. Electrospinning uses electric force to draw charged threads of polymer solutions, which can produce fibers having diameters in the nanometer scale, but is relatively slower compared to other approaches. Melt blowing extrudes molten polymer through a narrow opening and out into a stream of high velocity hot air that exerts a force upon the polymer to cause it to elongate into a fiber having a diameter in the micrometer scale. This process requires thermoplastic polymers and is traditionally not capable of achieving the low diameters of electrospinning.

The fibers produced according to the present disclosure are suitable for use in various edible compositions such as synthetic foods using cultured cells or tissues and processed food products using non-cultured cells or tissues.

Described herein are edible compositions produced according to the systems and methods of the present disclosure. In some cases, the composition comprises a fat phase containing one or more fats or lipids. The fat phase can make up between 1% and 50% of the edible composition. The fat phase can contain oils, fats, or both. The composition can comprise a polymer such as, for example, prolamins and lipophilic cellulose derivatives. The polymer can be formed from a spinning method such as electrospinning or solution blow spinning. In a preferred embodiment, solution blow spinning is used to generate the polymer.

As referred to herein, spinning is a process that can be used to create fibers of polymeric materials. Examples of spinning processes include extruding a polymer or material in a liquid form through one or more openings to form continuous filaments or fibers. In some cases, the polymer is a liquid formed by melting the polymer or by dissolving the polymer in a suitable solvent. Alternatively, in some cases, the polymer or material is generated by solution blow spinning. Some approaches include pressing a molten or melted polymer through an opening after which it solidifies as it cools (e.g., melt spinning). Other examples of spinning include wet spinning, dry spinning, shear-driven spinning, centrifugation spinning, jet spinning, and electrospinning.

The polymeric fibers or scaffolds provided herein can be prepared according to a variety of methods. Such methods include unidirectional freeze drying, electro-spinning, solution blow spinning, wet spinning, casting, micromolding, 3D printing, or extrusion techniques.

In some cases, scaffolds or fibers provided herein can be prepared using unidirectional freeze drying techniques. Unidirectional freeze drying techniques create structures by solidifying a solvent with a solution, gel, or dispersion by lowering the temperature of the solution, gel, or dispersion in such a way that the solvent separates from the dissolved and/or dispersed materials. The solidified solvent can then be removed, leaving behind a scaffold or fibrous material with desired properties. In some cases, the temperature gradient is created from one direction of the solution such that freezing occurs from one side of the solution, gel, or dispersion to the other.

Polymeric fibers or scaffolds herein can also be prepared using micromolding or casting techniques. In micromolding or casting, a cavity of the desired shape of the final material is created and the polymeric materials are place into the cavity. The polymeric materials are then solidified to produce the desired share. In micromolding, features on the scale of nanometers or micrometers are possible to develop.

Polymeric fibers or scaffolds provided herein can be prepared using 3D printing techniques. Examples of 3D printing techniques compatible with the compositions provided herein include stereolithography, digital light processing, fused deposition modeling, selective laser sintering, selective laser melting, electronic beam melting, laminated object manufacturing, binder jetting, and material jetting.

The scaffold or polymeric fibers (e.g., nanofibers/microfibers) disclosed herein can be used to approximate one or more texture characteristics of a corresponding conventional food product. For example, in the case of muscle tissue, blow spinning can be used to align nanofibers in a parallel orientation in order to yield a structure comparable to a conventional meat product. This is because in vivo muscle fibers similarly align in a parallel orientation, and these polymeric fibers serve to guide any deposited cells (before or after differentiation into muscle cells) in organizing with this orientation. Aligned nanofibers can also provide a better mouthfeel to the scaffold, with or without cells. The plurality of fibers can form the scaffold structure together, and optionally incorporate additional ingredients as disclosed herein to provide the desirable taste, texture, and nutrition profile of the corresponding conventional food product.

In some cases, the blow-spun scaffolds containing nanofibers/microfibers are combined with other scaffolding components such as created using casting or extrusion. This allows for an enhanced or increased complexity in the structure and texture of the food product. For example, blow-spun scaffolds could be used to mimic muscle tissue, while adjacent cast hydrogel scaffolds can have properties of adipose tissue or connective tissue to mimic the fat-laden striation patterns seen in conventional fish meat. For these varied scaffolds / fibers / hydrogels, there can be different ratios, depending upon the characteristics of the product. For example, a fatty product would contain a higher ratio of hydrogel to blow spun scaffold.

In some cases, cells are incorporated directly in the scaffold generation process instead of being deposited after the scaffolds have been generated. Incorporation of the cells into the scaffold directly during generation can be either as an addition to the base polymer in the case of a blow spun scaffold, or, in cast or extruded hydrogels, interspersed throughout the hydrogel. This is an important distinction because in both of these cases, cells are added as a final step, and would not expect significant differentiation or proliferation. However, with this method, the cells can be added by techniques such as vacuum seeding, injection, or simple gravity-based seeding.

The assembling process can be achieved in a variety of ways. One method comprises creating the different components separately and assembling them as a final step of production. Assembly can be done with conventional food ingredients such as egg whites, cream of tartar, gums, sugars, calcium lactate, gluconate, sodium alginate, gelatin, pectin, pectin esterase, transglutaminase, heat and other binders. A refinement of this step would be that, after assembly of these components, they are combined with cells. As the cells grow and proliferate, they form their own matrix that 'glues' or binds the layers together.

In some cases, the fat phase comprises a vegetable oil, an animal oil, a fish oil, an algae oil, or any combination thereof. In some embodiments, the fat phase comprises a butter, cheese, lard, or tallow. The fat phase can comprise a saturated fat, an unsaturated fat, or both. In some cases, cultured or un-cultured cells are processed to extract the fat content (e.g., from cultured adipose cells) that is then included in the fat phase used to make the edible composition. The fat may be extracted from non-animal cells.

In some cases, the edible composition comprises the fat phase at a concentration of about 1 % (w/w) to about 80 % (w/w). In some cases, the edible composition comprises the fat phase at a concentration of about 1 % (w/w) to about 5 % (w/w), about 1 % (w/w) to about 10 % (w/w), about 1 % (w/w) to about 20 % (w/w), about 1 % (w/w) to about 30 % (w/w), about 1 % (w/w) to about 40 % (w/w), about 1 % (w/w) to about 50 % (w/w), about 1 % (w/w) to about 60 % (w/w), about 1 % (w/w) to about 70 % (w/w), about 1 % (w/w) to about 80 % (w/w), about 5 % (w/w) to about 10 % (w/w), about 5 % (w/w) to about 20 % (w/w), about 5 % (w/w) to about 30 % (w/w), about 5 % (w/w) to about 40 % (w/w), about 5 % (w/w) to about 50 % (w/w), about 5 % (w/w) to about 60 % (w/w), about 5 % (w/w) to about 70 % (w/w), about 5 % (w/w) to about 80 % (w/w), about 10 % (w/w) to about 20 % (w/w), about 10 % (w/w) to about 30 % (w/w), about 10 % (w/w) to about 40 % (w/w), about 10 % (w/w) to about 50 % (w/w), about 10 % (w/w) to about 60 % (w/w), about 10 % (w/w) to about 70 % (w/w), about 10 % (w/w) to about 80 % (w/w), about 20 % (w/w) to about 30 % (w/w), about 20 % (w/w) to about 40 % (w/w), about 20 % (w/w) to about 50 % (w/w), about 20 % (w/w) to about 60 % (w/w), about 20 % (w/w) to about 70 % (w/w), about 20 % (w/w) to about 80 % (w/w), about 30 % (w/w) to about 40 % (w/w), about 30 % (w/w) to about 50 % (w/w), about 30 % (w/w) to about 60 % (w/w), about 30 % (w/w) to about 70 % (w/w), about 30 % (w/w) to about 80 % (w/w), about 40 % (w/w) to about 50 % (w/w), about 40 % (w/w) to about 60 % (w/w), about 40 % (w/w) to about 70 % (w/w), about 40 % (w/w) to about 80 % (w/w), about 50 % (w/w) to about 60 % (w/w), about 50 % (w/w) to about 70 % (w/w), about 50 % (w/w) to about 80 % (w/w), about 60 % (w/w) to about 70 % (w/w), about 60 % (w/w) to about 80 % (w/w), or about 70 % (w/w) to about 80 % (w/w). In some cases, the edible composition comprises the fat phase at a concentration of about 1 % (w/w), about 5 % (w/w), about 10 % (w/w), about 20 % (w/w), about 30 % (w/w), about 40 % (w/w), about 50 % (w/w), about 60 % (w/w), about 70 % (w/w), or about 80 % (w/w). In some cases, the edible composition comprises the fat phase at a concentration of at least about 1 % (w/w), about 5 % (w/w), about 10 % (w/w), about 20 % (w/w), about 30 % (w/w), about 40 % (w/w), about 50 % (w/w), about 60 % (w/w), or about 70 % (w/w). In some cases, the edible composition comprises the fat phase at a concentration of at most about 5 % (w/w), about 10 % (w/w), about 20 % (w/w), about 30 % (w/w), about 40 % (w/w), about 50 % (w/w), about 60 % (w/w), about 70 % (w/w), or about 80 % (w/w).

Examples of oils include vegetable oils such as include soybean oil, sunflower oil, linseed oil, low erucic rapeseed oil, corn oil, olive oil, palm oil, and palm kernel oil. Additional examples of oils include comprises soybean oil, sunflower oil, linseed oil, rapeseed oil, corn oil, algal oil, lecithin, olive oil, palm oil, palm kernel oil. Examples of oils can also include wheat germ oil, corn oil, sunflower oil, karite butter, castor oil, sweet almond oil, macadamia oil, apricot oil, soybean oil, rapeseed oil, cottonseed oil, alfalfa oil, poppy oil, pumpkin oil, sesame seed oil, marrow oil, avocado oil, hazelnut oil, grape seed oil, blackcurrant seed oil, evening primrose oil, millet oil, barley oil, quinoa oil, olive oil, rye oil, safflower oil, candlenut oil, passion flower oil or musk rose oil; or caprylic/capric acid triglycerides, synthetic oils. Examples of animal oils include milk fat such as butter or butter oil. Any combination of oils may be employed.

Suitable polymers for combination with the fat phase in an edible composition should have fat adhesion properties. For example, poor binding or adhesion to the fat phase can cause the composition to more easily fall apart during processing or lead to fiber aggregates to form. Examples of suitable polymers for combination with the fat phase include prolamins and lipophilic cellulose derivatives. The lipophilic cellulose derivative can be a cellulose derivative having a three-phase contact angle between a vegetable oil, a film of the lipophilic cellulose derivative, and air is less than 70° at 23°C. In some cases, the angle is less than 60°, less than 50°, or less than 40°.

Prolamins are plant storage proteins characterized by a high proline and glutamine content. Prolamins can be found in cereal grain seeds such as wheat (gliadin), barley (hordein), rye (secalin), and corn (zein).

Examples of lipophilic cellulose derivative include cellulose diacetate and alkylated celluloses such as methyl-ethylcellulose, ethylcellulose, propylcellulose or butylcellulose.

Preferably the fibrous material comprises one or more lipid compounds. Lipid compounds in the context of the present invention are lipophilic materials which often are from natural origin, but may also be a synthetic compound. Preferably the lipid compound comprises lecithin, fatty acid, monoglyceride, diglyceride, triglyceride, phytosterol, phytostanol, phytosteryl-fatty acid ester, phytostanyl-fatty acid ester, wax, fatty alcohol, carotenoid, oil-soluble colorant, oil-soluble vitamin, oil soluble flavor, or oil soluble fragrance. Also combinations of these compounds are within the scope of the present invention.

Various fats are consistent with the methods and compositions of the present disclosure. The fats can include fatty acids such as saturated and unsaturated fatty acids (e.g., monounsaturated, polyunsaturated) and various glycerides such as monoglycerides, diglycerides, and triglycerides. Examples of saturated fatty acids include caprylic acid, capric acid, lauric acid, myristic acid, palmitic acid, stearic acid, arachidic acid, behenic acid, lignoceric acid, and cerotic acid. Examples of unsaturated fatty acids include myristoleic acid, palmitoleic acid, sapienic acid, oleic acid, elaidic acid, vaccenic acid, linoleic acid, linoelaidic acid, arachidonic acid, eicosapentaenoic acid, erucic acid, and docosahexaenoic acid.

Examples of fats include plant oils such as apricot kernel oil, avocado oil, babassu oil, baobab oil, black seed oil, blackberry seed oil, blackcurrant seed oil, blueberry seed oil, camelina oil, camellia seed oil, castor oil, cocoa butter, coconut oil, corn oil, cottonseed oil, grape seed oil, hazelnut oil, hempseed oil, jojoba oil, lemon seed oil, lime seed oil, linseed oil, kukui nut oil, macadamia oil, maize oil, mango butter, mustard seed oil, olive oil, orange seed oil, palm oil, palm kernel oil, papaya seed oil, peach kernel oil, plum oil, pomegranate seed oil, poppy seed oil, pumpkins seed oil, rapeseed oil, red raspberry seed oil, sesame oil, shea butter, soy bean oil, strawberry seed oil, sunflower oil, sweet almond oil, and walnut oil.

Examples of fats include fish oils such as sardine oil, mackerel oil, herring oil, cod-liver oil. Examples of fats include animal oils such as butter, lard, and tallow.

The methods and compositions disclosed herein can be produced in sterile environments. In some cases, preservatives are not required. In some cases, when a composition is an edible food product containing cells, the scaffold is sterilized prior to, during, or after seeding of the cells. The shelf life of the edible food products disclosed herein that utilize cultured cells can be significantly extended (compared with conventional meat) because these products are grown in a completely sterile environment. Resident pathogens (bacteria, yeast, etc.) are not present, and the need for refrigeration is obviated by sterile packaging. In some cases, the products are tested for shelf life preservation. In some cases, the food products are hermetically-sealed in sterile packaging that enable distribution without the need for refrigeration. In other cases, refrigeration is used to further extend shelf life.

In the case of a cell-free product, the product can be packaged with or without sterilization. Sterilization can be accomplished using Pasteurization, energetic sterilization (e.g., gamma or UV irradiation), or chemical sterilization (such as with ethanol, which is then removed from the product). In the absence of sterilization, packaging and storage can be similar or identical to that of conventional meat (refrigeration and a defined shelf life). For a sterilized product, antibiotic preservatives would not be necessary, although preservatives that slow the rate of intrinsic decay may be added.

In some cases, one or more colorants are added during production of the food product. Non-limiting examples of colorants include annatto, amaranth, paprika, carmine, erythrosine, tumeric, beet extract, caramel, beta-carotene, tartrazine, and/or grape extracts, or a combination thereof.

Non-limiting examples of preservatives for extending shelf include vinegar, citric acid, ascorbic acid, tartaric acid, potassium bitartrate, malic acid, fumaric acid, and lactic acid, sodium salts, sorbic acid, acetic acid, benzoic acid, propionic acid, adipic acid, sodium nitrate, ferrous lactate, ferrous gluconate, stannous chloride, or a combination thereof.

### Synthetic Food Composition

Disclosed herein are synthetic food compositions. The food compositions may incorporate cultured cells such as cultured muscle, fat, or liver cells. The food compositions may be configured to have food characteristics similar to those of corresponding non-synthetic foods such as meat. The properties of the food composition or product can include texture and nutrition.

Texture characteristics include elasticity, plasticity, Young's modulus, toughness, firmness, cohesiveness, juiciness, stiffness, and other relevant metrics. These texture characteristics can be measured using a texture analyzer. In some cases, the food product is evaluated for a texture characteristic in comparison to a conventional food product such as, for example, a synthetic meat product compared to a conventional meat product. In some instances, the synthetic food product has a value for the texture characteristic that is no more than 10%, 20%, 30%, 40%, 50%, 60%, 70%, 80%, or 90% higher or lower than the texture characteristic of the conventional food product. The texture characteristic can be measured according to conventional techniques known in the field such as Warner-Bratzler or texture profile analysis.

The scaffold or nanofibers and/or microfibers may be modulated in terms of compositional makeup as well as the manufacturing conditions to obtain the desirable texture characteristic(s). For example, the ratio of scaffolds, nanofibers/microfibers, and hydrogel may be adjusted. For example, a fatty product with high fat content may be made using a higher ratio of hydrogel containing extruded fat relative to a blow spun scaffold.

The nutritional content of the food composition can be adjusted by controlling the ingredients that are incorporated into the scaffold and/or the final food product. In some cases, the nutritional content of the food product is configured to approximate that of conventional meat in terms of protein, fats, and other nutrients (e.g., omega 3 fatty acids and various vitamins). In the example of salmon, representative numbers are as follows: approximately 4-21% fat and 15-28% protein with negligible carbohydrates. Coho Salmon meat fatty acids based on % of total oils: Omega 3- 55.2%, Omega 6- 2.0%, Omega 9- 9.3%; Myristic Acid- 1.5%, Palmitic Acid- 16.9%, Palmitoleic Acid- 2.3%, Stearic Acid- 4.5%, Oleic Acid- 7.8%, Linoleic Acid- 0.6%, Linolenic Acid- 1.1%, Stearidonic Acid- 0.8%, Eicosenoic Acid- 1.5%, Eicosatrienoic Acid- 0.5%, Eicosatetraenoic Acid- 1.1%, Eicosapentaenoic Acid (EPA) - 11.35%, Arachidonic Acid- 1.0%, Docosapentaenoic Acid (DPA)- 2.2%, Docosahexaenoic Acid (DHA)- 38.6%.

In some cases, the food composition is made to have a certain appearance. This encompasses color, opacity, reflective properties, visual texture, and other relevant visual parameters.

In some cases, the food composition has a taste that is comparable to that of conventional food products such as conventional meat. Taste can be assessed by 3rd party sensory panels.

### Detailed Description of Figures

**FIG. 1** illustrates one embodiment of a process for producing cells for human consumption using self-renewing cells. In this process, the population of self-renewing cells is obtained **101,** and then cultured **102** (e.g., on microscaffolds). Differentiation is induced in the cell population **103** followed by induction of lipid accumulation **104.** Finally, the population of cells is processed for human consumption **105.**

**FIG. 2** illustrates one embodiment of a process for culturing muscle tissue for human consumption. In this example, a first and a second population of self-renewing cells are obtained **201, 204.** The two populations of cells are cultured **202, 205** to the desired population size. Next, differentiation is induced in the two populations to generate myocytes **203** and adipocytes **206.** Finally, the two populations of cells are processed for human consumption **207.**

**FIG. 3** shows an overview of an exemplary process for preparing cultured meat for consumption. First, stem cell identification, isolation, and characterization are carried out. These cells are then grown in two-dimensional culture such as on a feeder cell layer. These cells are transitioned into suspension culture in a bioreactor. Subsequent to transitioning to suspension culture, the cells are differentiated into muscle cells. The meat is then harvested, and finally prepared and cooked.

**FIG. 4A** shows illustrative approaches for growing a meat product for human consumption with avian hepatocytes as an exemplary example for making foie gras. First, the initial cell line is obtained. In one approach, embryonic stem cells are isolated from avian embryos **401.** Induced pluripotent stem cells can also be generated and differentiated from avian dermal fibroblasts **402.** Embryonic germ cells can also be isolated from avian embryos **403.** Direct reprogramming of avian dermal fibroblasts to hepatocytes is an option **404.** Another method entails immortalization of adult differentiated avian hepatocytes **405** such as by viral transduction. In some cases, adult differentiated avian hepatocytes can be serially passaged and grown, and selected for spontaneously transformed cells (e.g. spontaneously immortalized cells due to random mutation) **406.** Nascent hepatic stem cells in adult avian liver tissue can also be isolated **407.** In addition, cultured hepatocytes can be cultured and subjected to toxin or injury to enhance proliferative potential **408.** The isolated stem cells **411** are then differentiated into differentiated hepatocytes **412** and subjected to induction of steatosis **413.** Steatosis can be induced using a variety of methods including genetic intervention and exogenous treatments **409.** Finally, these processes are assessed for scaling strategies **410** to enable large-scale production.

**FIG. 4B** shows illustrative approaches for growing a fish meat product for human consumption. First, the initial cell line is obtained. In one approach, embryonic stem cells are isolated from fish embryos **421.** Induced pluripotent stem cells can also be generated and differentiated from somatic fish cells **422.** Primordial germ cells can also be generated from somatic fish cells **423.** Self-renewing fibroblasts can be selected such as through repeated passaging and selection of cell colonies that continue to proliferate **424.** For example, adult differentiated fish fibroblasts can be serially passaged and grown, and selected for spontaneously transformed cells (e.g. spontaneously immortalized cells due to random mutation). Another method entails direct reprogramming of fibroblasts into myocytes **425.** Another method entails immortalization of fish cells such as myosatellite cells or adipocyte precursor cells using various methods such as by viral transduction (e.g., TERT, SV40 Large T Antigen) **426.** Pluripotent cells (e.g., embryonic stem cells **421,** pluripotent stem cells **422,** and primordial germ calls **423**) can be grown to desired quantities **433** during the food production process. The pluripotent cells can then be differentiated into precursor cells such as pre-adipocytes **430** and/or myosatellite cells **429.** In some cases, cells can be induced to differentiate into the desired cell type (e.g., muscle and/or fat cells) using genetic techniques and/or exogenous treatments **427.** For example, pre-adipocytes (adipocyte precursors) **430** and myosatellite cells **429** can be induced to differentiate into adipocytes **432** and myocytes **431,** respectively, using genetic manipulations such as gene editing and/or construct expression. Exogenous treatments can include small molecule treatment to induce differentiation. This can also include exposing cells to extracellular structures and/or signals such as microscaffolds optionally conjugated with differentiation/growth factors (e.g., FGF2). Finally, these processes can be assessed for scaling strategies **428** to enable large-scale production. Various combinations of the techniques described in **FIGs. 4A-4B** can be used for production of cultured food products. In an exemplary embodiment, adult differentiated cells such as fish (e.g., bass or salmon) fibroblasts are serially passaged to identify cells that have undergone spontaneous immortalization **424.** These immortalized cells can be cultured to the desired quantity and then transdifferentiated **425** directly from the differentiated lineage into a desired lineage such as adipocytes and/or myocytes (or hepatocytes). Transdifferentiation can be accomplished using the various genetic modification techniques **427** such as using the expression constructs described herein.

Another approach not expressly shown in **FIGs. 4A-4B** utilizes mesenchymal stem cells (MSCs) for cultured food production. Mesenchymal stem cells are multipotent stromal cells capable of differentiating into various cell types including osteoblasts, chondrocytes, myocytes, and adipocytes. Mesenchymal stem cells can be derived from a variety of sources such as bone marrow and adipose tissue. For example, MSCs from the bone marrow can be isolated using flow cytometry or by plating directly on cell culture plates to form colony-forming unit fibroblasts. The MSCs can be grown in culture to desired quantities before they are induced to differentiate into a target differentiated cell type such as myocytes, adipocytes, hepatocytes, or any combination thereof. In some cases, the MSCs are split into separate cultures and differentiated separately before being combined to form a meat product (e.g., a mixture of myocytes and adipocytes, or hepatocytes and adipocytes). MSCs can be cultured using a variety of cell culture methods and can be grown using basal media supplemented with serum. In some cases, MSCs are cultured using a non-serum media. Sometimes, MSCs are cultured using a non-serum or low-serum media supplemented with a plant-based supplement such as mushroom-derived extract or soybean hydrolysate.

**FIGs. 5A-5D** show isolation and characterization of myosatellite cells isolated from trout. Where present, insets magnify image details, and the scale bar is equal to 10 µm in all micrographs. Substantially pure populations of piscine myosatellite cells are shown in **FIG. 5A** with the myosatellite cells making up about 80% of the isolated cells. **FIG. 5B** shows RT-PCR results confirming the presence of hallmark genes (Mstn1a, Myf5) expressed in these isolated myosatellite cells. **FIG. 5C** shows mature myocytes that were generated by differentiating myosatellite cells. The sheets of trout myotubes differentiated from the myosatellite cells are shown in **FIG. 5D****.**

**FIG. 6A** shows co-cultures of salmon myosatellite cells (arrowheads) and salmon pre-adipocytes (arrows) (scale bar is 500 µm). **FIG. 6B** shows successful myocyte differentiation into a mature myocyte within the co-culture (scale bar is 10 µm).

**FIG. 7A** shows salmon fibroblasts that have been induced to form spheroids for propagation in a bioreactor (scale bar is 500 µm). **FIG. 7B** shows a spheroid that has been returned to 2-dimensional culture conditions to assess viability, which is confirmed by the cells from the spheroid migrating circumferentially to form colonies (scale bar is 500 µm).

**FIG. 8** shows a culture of bass myosatellite cells that has been successfully cultivated according to the cell culture techniques disclosed herein.

**FIG. 9** shows an embryonic stem cell colony derived from a duck egg. The ESCs formed colonies growing on a monolayer of mouse embryonic fibroblast (MEF) feeder cells as shown in **FIG. 9****.**

**FIG. 10A** shows a population of duck hepatocytes in culture. These duck hepatocytes were assayed for markers of hepatocyte differentiation using reverse transcriptase polymerase chain reaction (RT-PCR). **FIG. 10B** shows the results of the RT-PCR assay comparing a control of undifferentiated cells (left lane) against the duck hepatocyte sample (right lane) for expression of the hepatocyte differentiation markers L-FABP, alpha-fetoprotein, and HNF3b. Beta actin was used as a control.

**FIG. 11A** shows self-renewing cells that were generated by culturing primary fibroblasts from duck and harvesting colonies of dividing cells after 6-8 weeks. **FIG. 11B** shows self-renewing cells that were generated by culturing primary fibroblasts from trout and harvesting colonies of dividing cells after 6-8 weeks. Both the duck and trout self-renewing cell colonies were characterized for morphology, proliferation rate, and proliferative capacity (number of passages achieved without changes in morphology, proliferative rate, and without genomic instability).

**FIG. 12** shows an exemplary embodiment of a construct that can be introduced into a cell to provide inducible differentiation into a hepatocyte. The construct comprises a tetracycline responsive element (TRE) and ORFs for the hepatocyte reprogramming factors HNF1A, FOXA1, and HNF4A. The construct can be stably transformed into a target cell such as a pluripotent or multipotent cell. In some cases, the construct can be stably transformed into a terminally differentiated cell such as a fibroblast. The TRE suppresses expression of the ORFs but allows the ORFs to be transcribed in the presence of tetracycline or doxycycline. Thus, a cell line stably incorporating this construct can be induced to differentiate into a hepatocyte via treatment with tetracycline/doxycycline.

**FIG. 13** shows an exemplary embodiment of a construct that can be introduced into a cell to allow inducible expression of one or more genes that predispose the cell to steatosis. The construct comprises a tetracycline responsive element (TRE) and the ORF for one or more genes involved in lipid metabolism such as ZFP423 (a zinc finger protein transcription factor) and/or ATF4 (activating transcription factor 4). The construct can be stably transformed into a target cell such as a pluripotent or multipotent cell. In some cases, the construct can be stably transformed into a terminally differentiated cell such as a fibroblast. The TRE suppresses expression of the ORFs but allows the ORFs to be transcribed in the presence of tetracycline or doxycycline. Thus, a cell line stably incorporating this construct can be induced to undergo steatosis or become predisposed to steatosis via treatment with tetracycline/doxycycline. In some cases, the construct comprises at least one, at least two, at least three, at least four, at least five, at least six, at least seven, at least eight, at least nine, at least ten, or at least eleven genes selected from the group consisting of: ATF4, ZFP423, LPIN1, PPAR, APOC3, APOE, ORL1, PEMT, MTTP, SREBP, STAT3, and KLF6.

**FIG. 14** shows an exemplary embodiment of a DNA construct system that can be introduced into a cell to allow a proliferation/differentiation switch from a pluripotent phenotype into a differentiated phenotype. This system has a first construct comprising a pluripotency cassette providing constitutive expression of the ORFs for pluripotency factors (e.g. Oct4, Sox2, Klf4, I-Myc). The pluripotency factors of the first construct are flanked by pLox sites. The system has a second construct comprising a differentiation cassette providing tetracycline inducible expression of MyoD and Cre recombinase. The addition of an inducing agent such as tetracycline or doxycycline can induce expression of MyoD and Cre recombinase. MyoD expression can help cause the cell to undergo differentiation into a muscle cell. The Cre recombinase enzyme can catalyze the excision of the pluripotency factors flanked by the pLox sites. Then inducing agent can be removed to cease induction of MyoD and Cre recombinase expression. An advantage of this system is the low footprint left by the system following excision of the pluripotency factors and removal of the inducing agent.

**FIG. 15** shows an exemplary construct that can be introduced into a cell to provide an inducible "off-switch". This construct comprises one or more genes of interest and an expression cassette comprising TRE and Cre recombinase, which are flanked by pLox sites. Addition of an inducing agent can cause a cell line stably incorporating this construct to express Cre recombinase for catalyzing the excision of the intervening sequence flanked by the pLox sites. Thus, the one or more genes (e.g., one(s) that promote differentiation) and the TRE and Cre recombinase expression cassette are removed, resulting in footprint-free excision of the genes of interest.

**FIG. 16A** shows cultured duck hepatocytes. The top panel shows a negative control culture of duck hepatocytes untreated with linoleic acid. The close-up of the top panel shows the cell morphology of the hepatocytes. The bottom panel shows duck hepatocytes treated with 2µM linoleic acid. The close-up of the bottom panel shows that the linoleic acid treated hepatocytes were successfully induced to undergo steatosis (an accumulation of lipid-containing vesicles - indicated by the arrowhead). **FIG. 16B** shows a dose response graph for linoleic acid treatment versus the percentage of hepatocytes having steatosis. As seen in the graph, increasing concentrations of linoleic acid (0, 0.1, 0.25, 0.5, 1, 2 µm) correlated with a corresponding increase in the percentage of hepatocytes with steatosis. At 2µM linoleic acid, the percentage of steatotic hepatocytes was above 85%. Similar results were achieved with oleic acid and using alternative protocols with the following components: IBMX (a methyl xanthine), rosiglitazone (a thiazolidinedione), increased glucose concentration, other fatty acid species, and/or corticosteroids such as dexamethasone.

**FIG. 17** shows a graph plotting the number of cells from an immortalized cell line derived from adult duck hepatocytes by selecting rapidly proliferating hepatocytes following serial passaging. These immortalized cells were cultured in progressively decreasing concentrations of fetal bovine serum (FBS) in the presence of soybean hydrolysate (10g/L). The number of hepatocytes and the percentage of FBS are graphed over time with the hepatocytes starting at below 4 million cells and 10% serum and gradually increasing in number until just above ten million cells at below 2% serum (0.8%) over a period of 20 days. The media supplementation of soybean hydrolysate allowed the serum requirements of the cultured cells to be reduced by 92%.

**FIG. 18** shows duck fibroblasts that have also been successfully grown in 10% shiitake mushroom extract after successive reduction of fetal bovine serum from the cell culture media.

**FIG. 19A** shows duck fibroblasts grown in serum-free media without additional supplementation; **FIG. 19B** shows a control culture grown in DMEM supplemented with 10% fetal bovine serum.

**FIG. 20** shows one embodiment of a bioreactor system used for cell culture. The bioreactor system comprises a reactor chamber **2001** for culturing cells and a stirring element **2003** for agitating the contents of the reactor chamber **2001.** Media is added into the reactor chamber via at least one input port **2002.** The media is sometimes maintenance media, differentiation media, steatotic media, proliferation media, or any other media formulation disclosed herein. Media is removed from the reactor chamber via at least one output port **2007.** In some cases, oxygen, carbon dioxide, and/or other gases are introduced through at least one input gas port **2006.** The input gas port **2006** is optionally coupled to an aerator positioned inside the reactor chamber. Oftentimes, the bioreactor system comprises at least one sensor **2004** for monitoring the reactor chamber. The at least one sensor **2004** is usually in communication with a control unit **2008** (e.g. a computer). In many cases, the reactor chamber is seeded with a plurality of micro-scaffolds **2005.** The micro-scaffolds **2005** enable adherence of certain adherent cells such as, for example, hepatocytes.

**FIG. 21** shows an illustrative process by which a bioreactor system is used for meat production. Specialized cells are isolated from an egg and grown in the bioreactor. The cells are grown using media comprising water and nutrients created from plants (e.g. as a substitute for serum). The cells are grown in the sterile environment of the bioreactor for 4-6 weeks. Finally, the cells are harvested and/or processed into a meat product.

**FIG. 22A** and **FIG. 22B** show a spheroid formed from duck hepatocytes growing in a hanging drop and a spinner flask into which the spheroid can be transferred for 3-dimensional suspension culture. As shown in **FIG. 22A****,** cells are grown in "hanging drops" of media and develop into spheroids and are then transferred to spinner flasks and grown in 3-dimensional suspension culture to allow scaling up of cell production.

**FIG. 23A** shows fish myosatellite cells grown on glucomannan microscaffolds (10% w/v) that have differentiated to form 3-dimensional myotubes. **FIG. 23B** shows a negative control of undifferentiated myosatellite cells from the same preparation grown in identical cell culture conditions.

**FIG. 24A** shows duck fibroblasts (arrowheads) successfully grown on glucomannan microscaffolds (arrows). **FIG. 24B** shows a representative glucomannan microscaffold.

**FIG. 25** shows duck muscle tissue that was created by differentiation of myosatellite cells. This figure represents a still photo from a movie demonstrating spontaneous myocyte contraction.

**FIG. 26** shows additional exemplary food products for human consumption generated according to the methods disclosed herein. The left panel shows a duck liver pâté made using duck steatotic liver cells. The right panel shows a fois gras butter made using duck steatotic liver cells.

**FIG. 27** shows exemplary food products for human consumption generated according to the methods disclosed herein. The left panel shows a salmon pâté. The right panel shows a duck meat pâté. In addition, chicken meat pâtés have also been developed.

**FIG. 28A** shows an exemplary embodiment of a method of Cre delivery for the purpose of activating / silencing particular genes. **FIG. 28B** shows different methods of using Cre to induce a "switch" between activated gene sets relevant to meat creation (e.g., proliferation and differentiation).

**FIG. 29** shows an embodiment of a method of transposon-mediated genetic self-excision for purpose of footprint-free gene editing. In this example, pLox sequences of the same color in parallel configurations undergo an excision event (removing the sequences between these triangles) in the presence of Cre enzyme. In contrast, pLox sequences in anti-parallel configurations (similarly-colored triangles that face each other) undergo recombination events, whereby the DNA sequences between the triangles flip back and forth in a stochastic manner. In the current figure, the transgenes are flanked by a transposon flanking sequence (TFS in the legend) permitting excision of the entire transgene cassette. The excision transposase gene is under the control of an inducible promoter (Pro in the figure). This may be a classically-described inducible promoter (such as tetracycline-inducible with a tetracycline-responsive element [TRE], an estrogen receptor, or similar system), or it may be a promoter corresponding to a reporter of a differentiated cell lineage. In the latter case, this reporter may be used to excise the entire transgene cassette once a cell has undergone terminal differentiation and maturation. This principle may be applied to adipogenesis, vasculogenesis, and differentiation into any other lineage.

**FIG. 30** shows an embodiment of a solution blow spinning process for generating fibers. In this example, two separate monomer solutions are used to create the resulting fiber layer. The first monomer 1 comprises aligned nanofibers that simulate muscle fibers. The second monomer 2 comprises lipid layers that simulate adipose banding. The muscle and/or fat cells may be added before, during, or after the blow spinning process. The spun fibers are collected using a collector, in this case, a rotating cylinder.

**FIG. 31** shows another embodiment of a solution blow spinning process for generating fibers. In this example, the first and second monomer solutions are blow spun onto a surface as two separate layers to create a multi-layered fiber composition. The layers can include different cells or tissues such as muscle and fat cells to create a composition that mimics certain properties of meat such as, for example, texture and/or composition. The spun fibers are collected using a collector, in this case, a rotating cylinder used in combination with a conveyor belt.

### Certain definitions

Unless otherwise defined, all technical terms used herein have the same meaning as commonly understood by one of ordinary skill in the art to which this invention belongs. As used in this specification and the appended claims, the singular forms "a," "an," and "the" include plural references unless the context clearly dictates otherwise. Any reference to "or" herein is intended to encompass "and/or" unless otherwise stated.

As used herein, the term "monomer" refers to an ingredient or component that can be processed into a fiber or fibrous material according to spinning techniques described herein. Monomer can refer to monomeric units of a polymer such as, for example, a glucose monomer in a polysaccharide polymer; however, the term is given a more expansive meaning in the present disclosure and includes "monomer" subunits that do not necessarily form covalent bonds to create a repeating polymeric structure (unless such a structure is specified). For example, individual molecules of cellulose (a polymer) can be the "monomer" subunits that may form "polymer" fibers through covalent and/or non-covalent interactions. Likewise, as used herein, the term "polymer" refers to a fiber or fibrous material that is generated from a composition or solution of monomers according to the spinning techniques described herein.

As used herein, "hepatocyte" refers to liver cells, hepatocytes, and hepatocyte-like cells. Hepatocytes are of animal origin and can be derived from various avian species including, as non-limiting examples, duck (e.g. Mulard duck, Barbary duck), goose (e.g. grey Landes goose), chicken, turkey, emu, Cornish chicken, Japanese quail, Plymouth Rock chicken, and ostrich.

As used herein, "high lipid accumulation" refers to the formation of lipid-containing vacuoles or vesicles inside of at least 10%, 20%, 30%, 40%, 50%, 60%, 70%, 80%, 90%, 95%, or 99% of the cells in a population of cells. In some cases, "high lipid accumulation" refers to the formation of lipid-containing vacuoles or vesicles inside of a majority (e.g. greater than half) of the cells in a population of cells.

As used herein, "self-renewal" or "self-renewing" refers to cell division or proliferation while maintaining a certain cell type (e.g. an undifferentiated state). Examples of self-renewing cells include embryonic stem cells, induced pluripotent stem cells, and multipotent stem cells (e.g. myosatellite cells, hepatoblasts, and other progenitor cells). In some cases, self-renewing cells include differentiated cells that have been immortalized (e.g. via spontaneous immortalization).

As used herein, "about" refers to a range of 10% around a particular quantity, unless stated otherwise. For example, about 10 liters (L) refers to 9 to 11 L.

In all cases where the term "about" is used in relation to a number or range, it is contemplated in some cases to mean about, or to optionally replace "about" with "exactly."

### Numbered Embodiments

The following embodiments recite nonlimiting permutations of combinations of features disclosed herein. Other permutations of combinations of features are also contemplated. In particular, each of these numbered embodiments is contemplated as depending from or relating to every previous or subsequent numbered embodiment, independent of their order as listed.

1. An edible composition for human consumption, comprising: a) a population of cells; and b) a plurality of polymeric fibers. 2. The composition of embodiment 1, wherein the polymeric fibers comprise an edible ingredient. 3. The composition of embodiment 2, wherein the edible ingredient comprises a texturizer, bulking agent or thickener, preservative, flavor enhancer, antimicrobial agent, pH modulator, desiccant, sweetener, curing or pickling agent, coloring agent, or any combination thereof. 4. The composition of embodiment 2, wherein the edible ingredient comprises a protein, a carbohydrate, a fat, or any combination thereof. 5. The composition of embodiment 2, wherein the protein comprises gelatin, collagen, elastin, silk, soy derivatives, zein, gliadin, hordein, casein, wheat derivatives, gluten, whey, millet derivatives, quinoa derivatives, algae proteins, marine source proteins, pea proteins, or any combination thereof. 6. The composition of embodiment 2, wherein the carbohydrate comprises chitosan, carrageenan, starch, agar, tapioca starch, alginate, cellulose and cellulose derivatives, pullulan, dextran, glucomannan, cyclodextrin, agarose, konjac, pectin, carrageenan, alginate, xanthan, guar, locust bean gum, tara gum, gum tragacanth, gum arabic, fenugreek gum, modified starch, potato starch, pea starch, Hylon 7 corn starch, hemicellulose, altanan, or any combination thereof. 7. The composition of embodiment 2, wherein the fat comprises soybean oil, sunflower oil, linseed oil, rapeseed oil, corn oil, olive oil, palm oil, palm kernel oil, butter, cheese, or any combination thereof. 8. The composition of embodiment 1, wherein the polymeric fiber comprises a supplement. 9. The composition of embodiment 8, wherein the supplement comprises a vitamin, a mineral, or both. 10. The composition of embodiment 1, wherein the population of cells comprises a cultured population of cells. 11. The composition of embodiment 1, wherein the population of cells comprises myocytes. 12. The composition of embodiment 11, wherein the plurality of fibers comprises a layer of fibers incorporating the myocytes and aligned to mimic muscle fibers. 13. The composition of embodiment 10, wherein the population of cells further comprises adipocytes. 14. The composition of embodiment 13, wherein the plurality of fibers comprises a layer of fibers incorporating the adipocytes and configured to mimic adipose banding patterns. 15. The composition of embodiment 1, wherein the population of cells is deposited onto and within the polymeric fibers. 16. The composition of embodiment 1, wherein the population of cells is integrated into the polymeric fibers. 17. The composition of embodiment 1, wherein the population of cells comprises a non-cultured population of cells. 18. The composition of embodiment 17, wherein the non-cultured population of cells is derived or obtained from an animal, fish, or bird. 19. The composition of embodiment 1, wherein the polymeric fibers are generated using a fiber spinning technique. 20. The composition of embodiment 19, wherein the fiber spinning technique is electrospinning or solution blow spinning. 21. The composition of embodiment 1, wherein the polymeric fibers and the population of cells are arranged as alternating layers of fibers and cells. 22. A method of producing an edible composition, comprising: a) forming a plurality of polymer fibers using a solution blow spinning process, wherein said polymer fibers are formed from a composition comprising an edible ingredient dissolved in a volatile solvent; and b) depositing said plurality of blow spun polymer fibers onto a surface to form a layer of polymer fibers. 23. The method of embodiment 22, wherein said composition comprises between about 1% and about 95% w/v protein, carbohydrate, fat, or a combination thereof. 24. The method of embodiment 22, wherein the volatile solvent comprises ethanol, ethyl acetate, acetic acid, acetone, dimethylformamide (DMF), or any combination thereof. 25. The method of embodiment 24, wherein the volatile solvent is acetone or ethyl acetate. 26. The method of embodiment 22, wherein the edible ingredient comprises a texturizer, bulking agent or thickener, preservative, flavor enhancer, antimicrobial agent, pH modulator, desiccant, sweetener, curing or pickling agent, coloring agent, or any combination thereof. 27. The method of embodiment 22, wherein the edible ingredient comprises a protein, a carbohydrate, a fat, or any combination thereof. 28. The method of embodiment 27, wherein the protein comprises gelatin, collagen, elastin, silk, soy derivatives, zein, gliadin, hordein, casein, wheat derivatives, gluten, whey, millet derivatives, quinoa derivatives, algae proteins, marine source proteins, pea proteins, or any combination thereof. 29. The method of embodiment 27, wherein the carbohydrate comprises chitosan, carrageenan, starch, agar, tapioca starch, alginate, cellulose and cellulose derivatives, pullulan, dextran, glucomannan, cyclodextrin, agarose, konjac, pectin, carrageenan, alginate, xanthan, guar, locust bean gum, tara gum, gum tragacanth, gum arabic, fenugreek gum, modified starch, potato starch, pea starch, Hylon 7 corn starch, hemicellulose, altanan, or any combination thereof. 30. The method of embodiment 27, wherein the fat comprises soybean oil, sunflower oil, linseed oil, rapeseed oil, corn oil, olive oil, palm oil, palm kernel oil, butter, cheese, or any combination thereof. 31. The method of embodiment 27, wherein the composition comprises a supplement. 32. The method of embodiment 31, wherein the supplement comprises a vitamin, a mineral, or both. 33. The method of embodiment 27, further comprising depositing a population of cells onto the layer of polymeric fibers. 34. The method of embodiment 33, wherein the population of cells comprises a cultured population of cells. 35. The method of embodiment 33, wherein the population of cells comprises myocytes. 36. The method of embodiment 35, wherein the layer of polymeric fibers incorporates the myocytes and comprises fibers that are aligned to mimic muscle fibers. 37. The method of embodiment 35, wherein the population of cells further comprises adipocytes. 38. The method of embodiment 37, wherein the layer of polymeric fibers incorporates the adipocytes and is configured to mimic adipose banding patterns. 39. The method of embodiment 33, wherein the population of cells comprises a non-cultured population of cells. 40. The method of embodiment 39, wherein the non-cultured population of cells is derived or obtained from an animal, fish, or bird. 41. The method of embodiment 33, wherein the layer of polymeric fibers and the population of cells are arranged as alternating layers of fibers and cells. 42. The method of embodiment 27, further comprising collecting the layer of polymeric fibers on a collector. 43. The method of embodiment 42, wherein the collector is a rotating drum. 44. The method of embodiment 27, wherein the solution blow spinning process is configured to achieve a desired polymeric fiber diameter or diameter range. 45. A method of producing cultured tissue for human consumption, the method comprising: a) obtaining a population of self-renewing cells; b) culturing the population of self-renewing cells; c) inducing differentiation in the population of self-renewing cells to form cultured tissue; and d) processing the cultured tissue for human consumption. 46. The method of embodiment 45, wherein the population of self-renewing cells comprises at least one cell that has been modified to undergo inducible differentiation. 47. The method of embodiment 46, wherein the at least one cell has been modified to incorporate at least one genetic construct comprising: a) an open reading frame (ORF) of at least one pluripotency gene; and b) an open reading frame (ORF) of a regulatory factor configured to inactivate the ORF of the at least one pluripotency gene. 48. The method of embodiment 47, wherein the at least one genetic construct further comprises: a) an open reading frame (ORF) of at least one differentiation gene; and b) a promoter controlling expression of the open reading frame (ORF) of the regulatory factor. 49. The method of embodiment 47 or 48, wherein: a) the regulatory factor is a transposase; and b) the open reading frame (ORF) of the at least one pluripotency gene is flanked by transposon flanking sequences recognized by the transposase such that expression of the transposase catalyzes excision of the open reading frame (ORF) of at least one pluripotency gene. 50. The method of any one of embodiments 47-49, wherein inducing differentiation comprises exposing the at least one cell to an induction agent to induce expression of the ORF of the regulatory factor, wherein expression of transposase results in excision of the ORF of the at least one pluripotency gene. 51. The method of any one of embodiments 47-49, wherein inducing differentiation comprises exposing the at least one cell to an induction agent to induce expression of the ORF of the regulatory factor, wherein expression of transposase results in excision of the at least one differentiation gene. 52. The method of any one of embodiments 47-49, further comprising inducing expression of the ORF of the transposase, wherein expression of transposase results in excision of the at least one genetic construct. 53. The method of embodiment 52, wherein expression of the ORF of the transposase is controlled by a marker of cell differentiation. 54. The method of embodiment 53, wherein the marker of cell differentiation is MyoD, myosin heavy chain (MHC), alpha-actinin, Myh1, Myh4, Myh7, titin, nebulin, or myosin light polypeptide 2 (mylz2), and expression of the ORF of the transposase is controlled by a constitutively active promoter. 55. The method of embodiment 52, wherein excision of the at least one genetic construct leaves no genetic footprint of the at least one genetic construct in the at least one cell. 56. The method of embodiment 48, wherein the at least one differentiation gene comprises an ORF of at least one hepatocyte differentiation factor selected from Hepatocyte Nuclear Factor 1 Alpha (HNF1A), Forkhead Box A2 (FOXA2), and Hepatocyte Nuclear Factor 4 Alpha (HNF4A). 57. The method of embodiment 48, wherein the at least one differentiation gene comprises a myogenic factor selected from Myogenin (MyoG), Myogenic Differentiation 1 (MyoD), Myogenic Factor 6 (MRF4), Myogenic Factor 5 (MYF5), PAX3, PAX7, and MEF2. 58. The method of embodiment 48, wherein the at least one differentiation gene comprises at least one adipogenic factor selected from Fatty Acid Binding Protein 4 (FABP4), Insulin-Responsive Glucose Transporter Type 4 (GLUT4), Adiponectin, C1Q And Collagen Domain Containing (ADIPOQ), 1-Acylglycerol-3-Phosphate O-Acyltransferase 2 (AGPAT2), Perilipin 1 (PLIN1), Leptin (LEP), Lipoprotein Lipase (LPL), CEBPalpha, CEBPbeta, PPARgamma, and ZFP423. 59. The method of embodiment 45, wherein cells in the population of self-renewing cells are maintained in a self-renewing state using microRNAs targeting at least one differentiation gene. 60. The method of embodiment 59, wherein inducing differentiation comprises removing microRNAs targeting at least one differentiation gene. 61. The method of embodiment 45, wherein inducing differentiation comprises exposing the population of self-renewing cells to microRNAs targeting at least one pluripotency gene. 62. The method of embodiment 45, wherein inducing differentiation comprises exposing the population of self-renewing cells to episomal DNA constructs targeting at least one pluripotency gene. 63. The method of embodiment 45, wherein inducing differentiation comprises using exosomes to target at least one pluripotency gene in the population of self-renewing cells. 64. The method of embodiment 45, wherein inducing differentiation comprises using exosomes to deliver nucleic acids, small molecules, or growth factors targeting at least one pluripotency gene in the population of self-renewing cells. 65. The method of any one of embodiments 61-64, wherein the at least one pluripotency gene comprises at least one of an anti-differentiation gene, an anti-proliferation gene, and a combination thereof. 66. The method of embodiment 61, wherein microRNAs are delivered into the population of self-renewing cells through lipofection, nucleofection, transfection, or transduction. 67. The method of any one of embodiments 45-65, wherein inducing differentiation comprises incubating the population of self-renewing cells with scaffolds embedded with DNA vectors containing microRNAs, wherein cells in the population internalize the DNA vectors and subsequently express the microRNAs. 68. The method of embodiment 45, wherein cells in the population of self-renewing cells are maintained in a self-renewing state through exposure to synthetic RNA coding for at least one pluripotency gene. 69. The method of embodiment 45, wherein inducing differentiation comprises exposing the population of self-renewing cells to synthetic RNA coding for at least one differentiation gene. 70. The method of embodiment 1, wherein the population of self-renewing cells is maintained in a self-renewing state through modulation of DNA methylation. 71. The method of embodiment 1, wherein inducing differentiation comprises modulating DNA methylation in the population of self-renewing cells. 72. A method for producing cultured fish roe for human consumption, the method comprising: a) obtaining a population of fish cells capable of self-renewal; b) inducing formation of primordial germ cells in the population of fish cells; and c) culturing the primordial germ cells to maturate into fish roe. 73. The method of embodiment 72, wherein the population of fish cells comprises cells selected from embryonic stem cells or induced pluripotent stem cells. 74. The method of embodiment 72, wherein inducing formation of primordial germ cells comprises incubating the population of fish cells in a media formulation comprising BMP, retinoic acid, EGF, or any combination thereof. 75. The method of embodiment 74, wherein the media formulation further comprises luteinizing hormone, DHP, or both. 76. The method of embodiment 72, wherein inducing formation of primordial germ cells further comprises increasing expression of VASA, deadend, DAZL, CXCR4b, Buckyball, or any combination thereof. 77. A method of making a cultured food product, comprising: a) forming polymer fibers using solution blow spinning; b) seeding cultured cells onto the polymer fibers to form synthetic muscle tissue; and c) processing the cultured cells and polymer fibers to produce the cultured food product. 78. The method of embodiment 77, wherein the synthetic muscle tissue comprises a first configuration comprising cultured muscle cells and nanofibers arranged in parallel alignment to simulate muscle fibers. 79. The method of embodiment 78, wherein the synthetic muscle tissue comprises a second configuration comprising cultured adipose cells and nanofibers arranged to simulate adipose banding patterns. 80. The method of embodiment 79, wherein the first configuration and second configuration are combined to form the cultured food product configured to simulate a taste and texture of non-synthetic muscle tissue. 81. The method of embodiment 77, wherein the step of forming the polymer fibers in (a) occurs concurrently with the step of seeding cultured cells onto the polymer fibers in (b). 82. The method of embodiment 77, wherein the polymer fibers are made of monomers selected from proteins, polysaccharides, or both. 83. The method of embodiment 82, wherein the monomers comprise proteins selected from gelatin, collagen, elastin, silk, soy, zein, gliadin, hordein, amaranth, casein, wheat, whey, avenin, secalin, corn protein meal, ovomucin, ovotransferrin, ovalbumin, 8S mung bean protein, and algae and marine source proteins, or any combination thereof. 84. The method of embodiment 82, wherein the monomers comprise polysaccharides selected from chitosan, carrageenan, starch, agar, tapioca starch, alginate, cellulose and cellulose derivatives, pullulan, dextran, glucomannan, cyclodextrin, agarose, konjac, pectin, carrageenan, alginate, xanthan, guar, locust bean gum, tara gum, gum tragacanth, gum arabic, fenugreek gum, modified starch, potato starch, pea starch, Hylon 7 corn starch, hemicellulose, altanan, or any combination thereof. 85. The method of embodiment 77, wherein the step of forming polymer fibers in (a) comprises dissolving monomers in a solvent to form a pre-polymer solution, ejecting the solution into a gas or air stream to generate the polymer fibers as the solvent evaporates. 86. A cultured food product formed according to the method of any of embodiments 77-85. 87. A method of producing a food composition having a fat phase, comprising: a) forming a plurality of polymer fibers using a solution blow spinning process, wherein said polymer fibers are formed from a composition comprising an edible ingredient dissolved in a volatile solvent; and b) dispersing said plurality of blow spun polymer fibers in a fat phase. 88. The method of embodiment 87, wherein the polymer fibers comprise a lipophilic material selected from prolamins and lipophilic cellulose derivatives. 89. The method of embodiment 87, further comprising homogenizing a mixture of the plurality of blow spun polymer fibers and the fat phase. 90. The method of embodiment 89, further comprising fragmenting the mixture into particles. 91. The method of embodiment 87, wherein the fat phase comprises a vegetable oil, a dairy oil, a fish oil, an algae oil, or any combination thereof. 92. The method of embodiment 87, wherein the food composition comprises up to 95% by weight of an aqueous phase. 93. The method of embodiment 87, wherein the food composition comprises an emulsion comprising 1 to 99% by weight within the fat phase. 94. The method of embodiment 87, wherein the polymer fibers make up between 1% and 50% (w/w) of the food composition. 95. The food composition produced according to the method of any of embodiments 87-94. 96. An edible composition for human consumption, comprising: a) a population of cells; and b) a plurality of polymeric fibers or scaffolds. 97. The composition of embodiment 96, wherein the polymeric fibers or scaffolds comprise at least one edible ingredient. 98. The composition of embodiment 97, wherein the edible ingredient comprises a texturizer, bulking agent or thickener, preservative, flavor enhancer, antimicrobial agent, pH modulator, desiccant, sweetener, curing or pickling agent, coloring agent, or any combination thereof. 99. The composition of embodiment 97, wherein the edible ingredient comprises a protein, a carbohydrate, a fat, or any combination thereof. 100. The composition of embodiment 97, wherein the protein comprises collagen, elastin, silk, soy derivatives, zein, gliadin, hordein, casein, wheat derivatives, gluten, whey, yeast extracts, kafirin, millet derivatives, quinoa derivatives, bean derivatives, textured vegetable proteins, egg white proteins, sea vegetable derivatives, algae proteins, transglutaminase, glutamic acid derivatives, gelatin, fish source proteins, pea proteins, or any hydrolysates thereof, any isolates thereof, or any combination thereof. 101. The composition of embodiment 97, wherein the carbohydrate comprises chitosan, carrageenan, agar. tapioca starch, alginate, cellulose and cellulose derivatives, pullulan, dextran, glucomannan, cyclodextrin, agarose, konjac, pectin, sea vegetable derivatives, alginate, xanthan, guar gum, locust bean gum, tara gum, gum tragacanth, gum arabic, fenugreek gum, modified starch, potato starch, pea starch, Hylon 7 corn starch, hemicellulose, altanan, or any combination thereof. 102. The composition of embodiment 97, wherein the fat comprises soybean oil, sunflower oil, linseed oil, rapeseed oil, corn oil, algal oil, lecithin, olive oil, palm oil, palm kernel oil, butter, cheese, or any combination thereof. 103. The composition of embodiment 96, wherein the polymeric fiber or scaffold comprises a supplement. 104. The composition of embodiment 103, wherein the supplement comprises a vitamin, a mineral, or both. 105. The composition of embodiment 96, wherein the population of cells comprises a cultured population of cells. 106. The composition of embodiment 96, wherein the population of cells comprises myocytes. 107. The composition of embodiment 106, wherein the plurality of fibers comprises a layer of fibers incorporating the myocytes and aligned to mimic muscle fibers. 108. The composition of embodiment 105, wherein the population of cells further comprises adipocytes. 109. The composition of embodiment 108, wherein the plurality of fibers comprises a layer of fibers incorporating the adipocytes and configured to mimic adipose banding patterns. 110. The composition of embodiment 96, wherein the population of cells is deposited onto and within the polymeric fibers. 111. The composition of embodiment 96, wherein the population of cells is integrated into the polymeric fibers. 112. The composition of embodiment 96, wherein the population of cells comprises a non-cultured population of cells. 113. The composition of embodiment 112, wherein the non-cultured population of cells is derived or obtained from an animal, fish, or bird. 114. The composition of embodiment 96, wherein the polymeric fibers or scaffolds are generated using a fiber spinning technique. 115. The composition of embodiment 114, wherein the fiber spinning technique is electrospinning or solution blow spinning. 116. The composition of embodiment 96, wherein the polymeric fibers or scaffolds are generated using unidirectional freeze-drying, electro-spinning, solution blow spinning, wet spinning, casting, micromolding, 3D printing or extrusion, or any combination thereof. 117. The composition of embodiment 96, wherein the composition comprises polymeric fibers generated using a fiber spinning technique and scaffolds generated using unidirectional freeze-drying, electro-spinning, solution blow spinning, wet spinning, casting, micromolding, 3D printing or extrusion, or any combination thereof. 118. The composition of embodiment 96, wherein the polymeric fibers or scaffolds and the population of cells are arranged as alternating layers of fibers and cells. 119. A method of producing an edible composition, comprising: a) forming a plurality of polymer fibers using a solution blow spinning process, wherein said polymer fibers are formed from a composition comprising an edible ingredient dissolved in a solvent; and b) depositing said plurality of blow spun polymer fibers onto a surface to form a layer of polymer fibers. 120. The method of embodiment 119, wherein said composition comprises between about 1% and about 95% w/v protein, carbohydrate, fat, other polymer or a combination thereof. 121. The method of embodiment 119, wherein the solvent comprises a non-volatile solvent. 122. The method of embodiment 121, wherein the non-volatile solvent comprises water, salt water, an oil, or any combination thereof. 123. The method of embodiment 122, wherein the salt water comprises at least 1%, at least 2%, at least 5%, at least 10%, at least 20%, at least 30%, at least 40%, at least 50%, at least 60%, at least 70%, at least 80%, or at least 90% salt by weight. 124. The method of embodiment 122, wherein the oil comprises linear, branched, saturated, or unsaturated fatty acids or fatty esters, or any combination or derivative thereof. 125. The method of embodiment 122, wherein the oil comprises a C3-C50 fatty acid or derivative thereof. 126. The method of embodiment 122, wherein the oil is wheat germ oil, corn oil, sunflower oil, karite butter, castor oil, sweet almond oil, macadamia oil, apricot oil, soybean oil, rapeseed oil, cottonseed oil, alfalfa oil, poppy oil, pumpkin oil, sesame seed oil, marrow oil, avocado oil, hazelnut oil, grape seed oil, blackcurrant seed oil, evening primrose oil, millet oil, barley oil, quinoa oil, olive oil, rye oil, safflower oil, candlenut oil, passion flower oil or musk rose oil; or caprylic/capric acid triglycerides, synthetic oils, or any combination thereof. 127. The method of embodiment 122, wherein the solvent comprises a volatile solvent. 128. The method of embodiment 127, wherein the volatile solvent comprises ethanol, ethyl acetate, methanol, acetic acid, formic acid, phenol, acetone, dimethylformamide (DMF), or any combination thereof. 129. The method of embodiment 127, wherein the volatile solvent is acetone or ethyl acetate. 130. The method of embodiment 24, wherein the surface is a stationary or rotating surface. 131. The method of embodiment 119, wherein the edible ingredient comprises a texturizer, bulking agent or thickener, preservative, flavor enhancer, antimicrobial agent, pH modulator, desiccant, sweetener, curing or pickling agent, coloring agent, or any combination thereof. 132. The method of embodiment 119, wherein the edible ingredient comprises a protein, a carbohydrate, a fat, or any combination thereof. 133. The method of embodiment 132, wherein the protein comprises collagen, elastin, silk, soy derivatives, gelatin, zein, gliadin, hordein, casein, wheat derivatives, gluten, whey, yeast extract, kafirin millet derivatives, quinoa derivatives, bean derivatives, textured vegetable proteins, egg white proteins, sea vegetable derivatives, algae proteins, transglutaminase, glutamic acid derivatives, fish source proteins, pea proteins, or any combination, isolate, or derivative thereof. 134. The method of embodiment 132, wherein the carbohydrate comprises chitosan, carrageenan, agar, tapioca starch, alginate, cellulose and cellulose derivatives, pullulan, dextran, glucomannan, cyclodextrin, agarose, konjac, pectin, sea vegetable derivatives, alginate, xanthan, guar gum, locust bean gum, tara gum, gum tragacanth, gum arabic, fenugreek gum, modified starch, potato starch, pea starch, Hylon 7 corn starch, hemicellulose, altanan, or any combination thereof. 135. The method of embodiment 132, wherein the fat comprises soybean oil, sunflower oil, linseed oil, rapeseed oil, corn oil, algal oil, lecithin, olive oil, palm oil, palm kernel oil, butter, cheese, or any combination thereof. 136. The method of embodiment 131, wherein the other polymer comprises a block copolymer, block copolymer intermediate, modified poly(ethylene glycol), modified ethylene glycol monomer, polyester, poly(caprolactone), polylactic acid, poly(lactic-co-glycolic acid), polyethylene oxide, polyethylene glycol, poly(hydroxybutyrate), poly(ester urethane), polyvinyl alcohol, poloxamer, or any combination thereof. 137. The method of embodiment 132, wherein the composition comprises a supplement. 138. The method of embodiment 137, wherein the supplement comprises a vitamin, a mineral, or both. 139. The method of embodiment 132, further comprising depositing a population of cells onto the layer of polymeric fibers. 140. The method of embodiment 139, wherein the population of cells comprises a cultured population of cells. 141. The method of embodiment 139, wherein the population of cells comprises myocytes. 142. The method of embodiment 141, wherein the layer of polymeric fibers incorporates the myocytes and comprises fibers that are aligned to mimic muscle fibers. 143. The method of embodiment 141, wherein the population of cells further comprises adipocytes. 144. The method of embodiment 143, wherein the layer of polymeric fibers incorporates the adipocytes and is configured to mimic adipose banding patterns. 145. The method of embodiment 139, wherein the population of cells comprises a non-cultured population of cells. 146. The method of embodiment 145, wherein the non-cultured population of cells is derived or obtained from an animal, fish, or bird. 147. The method of embodiment 139, wherein the layer of polymeric fibers and the population of cells are arranged as alternating layers of fibers and cells. 148. The method of embodiment 132, further comprising collecting the layer of polymeric fibers on a collector. 149. The method of embodiment 148, wherein the collector is a rotating drum or stationary surface. 150. The method of embodiment 132, wherein the solution blow spinning process is configured to achieve a desired polymeric fiber diameter or diameter range. 151. A method of producing cultured tissue for human consumption, the method comprising: a) obtaining a population of self-renewing cells; b) culturing the population of self-renewing cells; c) inducing differentiation in the population of self-renewing cells to form cultured tissue; and d) processing the cultured tissue for human consumption. 152. The method of embodiment 151, wherein the population of self-renewing cells comprises at least one cell that has been modified to undergo inducible differentiation. 153. The method of embodiment 152, wherein the at least one cell has been modified to incorporate at least one genetic construct comprising: a) an open reading frame (ORF) of at least one pluripotency gene; and b) an open reading frame (ORF) of a regulatory factor configured to inactivate the ORF of the at least one pluripotency gene. 154. The method of embodiment 153, wherein the at least one genetic construct further comprises: a) an open reading frame (ORF) of at least one differentiation gene; and b) a promoter controlling expression of the open reading frame (ORF) of the regulatory factor. 155. The method of embodiment 153 or 154, wherein: a) the regulatory factor is a transposase; and b) the open reading frame (ORF) of the at least one pluripotency gene is flanked by transposon flanking sequences recognized by the transposase such that expression of the transposase catalyzes excision of the open reading frame (ORF) of at least one pluripotency gene. 156. The method of any one of embodiments 153-155, wherein inducing differentiation comprises exposing the at least one cell to an induction agent to induce expression of the ORF of the regulatory factor, wherein expression of transposase results in excision of the ORF of the at least one pluripotency gene. 157. The method of any one of embodiments 153-155, wherein inducing differentiation comprises exposing the at least one cell to an induction agent to induce expression of the ORF of the regulatory factor, wherein expression of transposase results in excision of the at least one differentiation gene. 158. The method of any one of embodiments 153-155, further comprising inducing expression of the ORF of the transposase, wherein expression of transposase results in excision of the at least one genetic construct. 159. The method of embodiment 158, wherein expression of the ORF of the transposase is controlled by a marker of cell differentiation. 160. The method of embodiment 159, wherein the marker of cell differentiation is MyoD, myosin heavy chain (MHC), alpha-actinin, Myh1, Myh4, Myh7, titin, nebulin, or myosin light polypeptide 2 (mylz2), and expression of the ORF of the transposase is controlled by a constitutively active promoter. 161. The method of embodiment 158, wherein excision of the at least one genetic construct leaves no genetic footprint of the at least one genetic construct in the at least one cell. 162. The method of embodiment 154, wherein the at least one differentiation gene comprises an ORF of at least one hepatocyte differentiation factor selected from Hepatocyte Nuclear Factor 1 Alpha (HNF1A), Forkhead Box A2 (FOXA2), and Hepatocyte Nuclear Factor 4 Alpha (HNF4A). 163. The method of embodiment 154, wherein the at least one differentiation gene comprises a myogenic factor selected from Myogenin (MyoG), Myogenic Differentiation 1 (MyoD), Myogenic Factor 6 (MRF4), Myogenic Factor 5 (MYF5), PAX3, PAX7, and MEF2. 164. The method of embodiment 154, wherein the at least one differentiation gene comprises at least one adipogenic factor selected from Fatty Acid Binding Protein 4 (FABP4), Insulin-Responsive Glucose Transporter Type 4 (GLUT4), Adiponectin, C1Q And Collagen Domain Containing (ADIPOQ), 1-Acylglycerol-3-Phosphate O-Acyltransferase 2 (AGPAT2), Perilipin 1 (PLIN1), Leptin (LEP), Lipoprotein Lipase (LPL), CEBPalpha, CEBPbeta, PPARgamma, and ZFP423. 165. The method of embodiment 151, wherein cells in the population of self-renewing cells are maintained in a self-renewing state using microRNAs targeting at least one differentiation gene. 166. The method of embodiment 165, wherein inducing differentiation comprises removing microRNAs targeting at least one differentiation gene. 167. The method of embodiment 151, wherein inducing differentiation comprises exposing the population of self-renewing cells to microRNAs targeting at least one pluripotency gene. 168. The method of embodiment 151, wherein inducing differentiation comprises exposing the population of self-renewing cells to episomal DNA constructs targeting at least one pluripotency gene. 169. The method of embodiment 151, wherein inducing differentiation comprises using exosomes to target at least one pluripotency gene in the population of self-renewing cells. 170. The method of embodiment 151, wherein inducing differentiation comprises using exosomes to deliver nucleic acids, small molecules, or growth factors targeting at least one pluripotency gene in the population of self-renewing cells. 171. The method of any one of embodiments 167-170, wherein the at least one pluripotency gene comprises at least one an anti-differentiation gene. 172. The method of embodiment 167, wherein microRNAs are delivered into the population of self-renewing cells through lipofection, nucleofection, transfection, or transduction. 173. The method of any one of embodiments 151-171, wherein inducing differentiation comprises incubating the population of self-renewing cells with scaffolds embedded with DNA vectors containing microRNAs, wherein cells in the population internalize the DNA vectors and subsequently express the microRNAs. 174. The method of embodiment 151, wherein cells in the population of self-renewing cells are maintained in a self-renewing state through exposure to modified RNA coding for at least one pluripotency gene. 175. The method of embodiment 151, wherein inducing differentiation comprises exposing the population of self-renewing cells to synthetic RNA coding for at least one differentiation gene. 176. The method of embodiment 151, wherein the population of self-renewing cells is maintained in a self-renewing state through modulation of DNA methylation. 177. The method of embodiment 151, wherein inducing differentiation comprises modulating DNA methylation in the population of self-renewing cells. 178. A method of making a cultured food product, comprising: a) forming polymer fibers using solution blow spinning; b) seeding cultured cells onto the polymer fibers to form synthetic muscle tissue; and c) processing the cultured cells and polymer fibers to produce the cultured food product. 179. The method of embodiment 178, wherein the synthetic muscle tissue comprises a first configuration comprising cultured muscle cells and nanofibers arranged in parallel alignment to simulate muscle fibers. 180. The method of embodiment 179, wherein the synthetic muscle tissue comprises a second configuration comprising cultured adipose cells and nanofibers arranged to simulate adipose banding patterns. 181. The method of embodiment 180, wherein the first configuration and second configuration are combined to form the cultured food product configured to simulate a taste and texture of non-synthetic muscle tissue. 182. The method of embodiment 178, wherein the step of forming the polymer fibers in (a) occurs concurrently with the step of seeding cultured cells onto the polymer fibers in (b). 183. The method of embodiment 178, wherein the polymer fibers are made of monomers selected from proteins, polysaccharides, or both. 184. The method of embodiment 183, wherein the monomers comprise proteins selected from comprises collagen, elastin, silk, soy derivatives, zein, gliadin, hordein, casein, wheat derivatives, gluten, whey, yeast extracts, kafirin, millet derivatives, quinoa derivatives, bean derivatives, textured vegetable proteins, egg white proteins, sea vegetable derivatives, algae proteins, transglutaminase, glutamic acid derivatives, gelatin, fish source proteins, and pea proteins, or any hydrolysates thereof, any isolates thereof, or any combination thereof. 185. The method of embodiment 183, wherein the monomers comprise polysaccharides selected from chitosan, carrageenan, starch, agar. tapioca starch, alginate, cellulose and cellulose derivatives, pullulan, dextran, glucomannan, cyclodextrin, agarose, konjac, pectin, sea vegetable derivatives, alginate, xanthan, guar gum, locust bean gum, tara gum, gum tragacanth, gum arabic, fenugreek gum, modified starch, potato starch, pea starch, Hylon 7 corn starch, hemicellulose, and altanan, or any combination thereof. 186. The method of embodiment 183, wherein the step of forming polymer fibers in (a) comprises dissolving monomers in a solvent to form a pre-polymer solution, ejecting the solution into a gas or air stream to generate the polymer fibers as the solvent evaporates. 187. A cultured food product formed according to the method of any of embodiments 178-786-. 188. A method of producing a food composition having a fat phase, comprising: a) forming a plurality of polymer fibers using a solution blow spinning process, wherein said polymer fibers are formed from a composition comprising an edible ingredient dissolved in a solvent; and b) dispersing said plurality of blow spun polymer fibers in a fat phase. 189. The method of embodiment 188, wherein the polymer fibers comprise a lipophilic material selected from prolamins and lipophilic cellulose derivatives. 190. The method of embodiment 188, further comprising homogenizing a mixture of the plurality of blow spun polymer fibers and the fat phase. 191. The method of embodiment 190, further comprising fragmenting the mixture into particles. 192. The method of embodiment 188, wherein the fat phase comprises a vegetable oil, a dairy oil, a fish oil, an algae oil, or any combination thereof. 193. The method of embodiment 188, wherein the food composition comprises up to 95% by weight of an aqueous phase. 194. The method of embodiment 188, wherein the food composition comprises an emulsion comprising 1 to 99% by weight within the fat phase. 195. The method of embodiment 188, wherein the polymer fibers make up between 1% and 50% (w/w) of the food composition. 196. The food composition produced according to the method of any of embodiments 188-195. 197. An edible food composition for human consumption, comprising: a scaffold comprising a plurality of polymeric fibers, wherein the edible composition has a textural characteristic comparable to a corresponding conventional food composition. 198. The composition of embodiment 197, wherein the polymeric fibers comprise at least one edible ingredient. 199. The composition of embodiment 198, wherein the edible ingredient comprises a texturizer, bulking agent or thickener, preservative, flavor enhancer, antimicrobial agent, pH modulator, desiccant, sweetener, curing or pickling agent, coloring agent, or any combination thereof. 200. The composition of embodiment 198, wherein the edible ingredient comprises a protein, a carbohydrate, a fat, or any combination thereof. 201. The composition of embodiment 198, wherein the protein comprises collagen, elastin, silk, soy derivatives, zein, gliadin, hordein, casein, wheat derivatives, gluten, whey, yeast extracts, kafirin, millet derivatives, quinoa derivatives, bean derivatives, textured vegetable proteins, egg white proteins, sea vegetable derivatives, algae proteins, transglutaminase, glutamic acid derivatives, gelatin, fish source proteins, pea proteins, or any hydrolysates thereof, any isolates thereof, or any combination thereof. 202. The composition of embodiment 198, wherein the carbohydrate comprises chitosan, carrageenan, agar. tapioca starch, alginate, cellulose and cellulose derivatives, pullulan, dextran, glucomannan, cyclodextrin, agarose, konjac, pectin, sea vegetable derivatives, alginate, xanthan, guar gum, locust bean gum, tara gum, gum tragacanth, gum arabic, fenugreek gum, modified starch, potato starch, pea starch, Hylon 7 corn starch, hemicellulose, altanan, or any combination thereof. 203. The composition of embodiment 198, wherein the fat comprises soybean oil, sunflower oil, linseed oil, rapeseed oil, corn oil, algal oil, lecithin, olive oil, palm oil, palm kernel oil, butter, cheese, or any combination thereof. 204. The composition of embodiment 197, wherein the polymeric fiber comprises a supplement. 205. The composition of embodiment 204, wherein the supplement comprises a vitamin, a mineral, or both. 206. The composition of embodiment 197, wherein the polymeric fibers are generated using a fiber spinning technique. 207. The composition of embodiment 206, wherein the fiber spinning technique is electrospinning or solution blow spinning. 208. The composition of embodiment 197, wherein the polymeric fibers are generated using unidirectional freeze-drying, electro-spinning, solution blow spinning, wet spinning, casting, micromolding, 3D printing or extrusion, or any combination thereof. 209. The composition of embodiment 197, wherein the composition comprises polymeric fibers generated using a fiber spinning technique and scaffolds generated using unidirectional freeze-drying, electro-spinning, solution blow spinning, wet spinning, casting, micromolding, 3D printing or extrusion, or any combination thereof.

The present application provides aspects and embodiments as set forth in the following Statements 1-40:
Statement 1. An edible composition for human consumption, comprising:
   (i) a population of cells; and
   (ii) a plurality of polymeric fibers or scaffolds.
Statement 2. The composition of statement 1, wherein the polymeric fibers or scaffolds comprise at least one edible ingredient.
Statement 3. The composition of statement 2, wherein the edible ingredient comprises a texturizer, bulking agent or thickener, preservative, flavor enhancer, antimicrobial agent, pH modulator, desiccant, sweetener, curing or pickling agent, coloring agent, or any combination thereof.
Statement 4. The composition of statement 2, wherein the edible ingredient comprises a protein, a carbohydrate, a fat, or any combination thereof.
Statement 5. The composition of statement 1, wherein the polymeric fiber or scaffold comprises a supplement.
Statement 6. The composition of statement 5, wherein the supplement comprises a vitamin, a mineral, or both.
Statement 7. The composition of statement 1, wherein the population of cells comprises a cultured population of cells.
Statement 8. The composition of statement 1, wherein the population of cells comprises myocytes.
Statement 9. The composition of statement 8, wherein the plurality of fibers comprises a layer of fibers incorporating the myocytes and aligned to mimic muscle fibers.
Statement 10. The composition of statement 1, wherein the population of cells further comprises adipocytes.
Statement 11. The composition of statement 10, wherein the plurality of fibers comprises a layer of fibers incorporating the adipocytes and configured to mimic adipose banding patterns.
Statement 12. The composition of statement 1, wherein the population of cells is deposited onto and within the polymeric fibers.
Statement 13. The composition of statement 1, wherein the population of cells is integrated into the polymeric fibers.
Statement 14. The composition of statement 1, wherein the population of cells comprises a non-cultured population of cells.
Statement 15. The composition of statement 14, wherein the non-cultured population of cells is derived or obtained from an animal, fish, or bird.
Statement 16. The composition of statement 1, wherein the polymeric fibers or scaffolds are generated using a fiber spinning technique.
Statement 17. The composition of statement 16, wherein the fiber spinning technique is electrospinning or solution blow spinning.
Statement 18. The composition of statement 1, wherein the polymeric fibers or scaffolds are generated using unidirectional freeze-drying, electro-spinning, solution blow spinning, wet spinning, casting, micromolding, 3D printing or extrusion, or any combination thereof.
Statement 19. The composition of statement 1, wherein the composition comprises polymeric fibers generated using a fiber spinning technique and scaffolds generated using unidirectional freeze-drying, electro-spinning, solution blow spinning, wet spinning, casting, micromolding, 3D printing or extrusion, or any combination thereof.
Statement 20. The composition of statement 1, wherein the polymeric fibers or scaffolds and the population of cells are arranged as alternating layers of fibers and cells.
Statement 21. A method of producing an edible composition, comprising:
   (i) forming a plurality of polymer fibers using a solution blow spinning process, wherein said polymer fibers are formed from a composition comprising an edible ingredient dissolved in a solvent; and
   (ii) depositing said plurality of blow spun polymer fibers onto a surface to form a layer of polymer fibers.
Statement 22. The method of statement 21, wherein the solvent comprises a volatile solvent.
Statement 23. The method of statement 22, wherein the composition comprises a supplement.
Statement 24. The method of statement 23, wherein the supplement comprises a vitamin, a mineral, or both.
Statement 25. A method of making a cultured food product, comprising:
   (i) forming polymer fibers using solution blow spinning;
   (ii) seeding cultured cells onto the polymer fibers to form synthetic muscle tissue; and
   (iii) processing the cultured cells and polymer fibers to produce the cultured food product.
Statement 26. The method of statement 25, wherein the synthetic muscle tissue comprises a first configuration comprising cultured muscle cells and nanofibers arranged in parallel alignment to simulate muscle fibers.
Statement 27. The method of statement 26, wherein the synthetic muscle tissue comprises a second configuration comprising cultured adipose cells and nanofibers arranged to simulate adipose banding patterns.
Statement 28. The method of statement 27, wherein the first configuration and second configuration are combined to form the cultured food product configured to simulate a taste and texture of non-synthetic muscle tissue.
Statement 29. The method of statement 25, wherein the step of forming the polymer fibers in (a) occurs concurrently with the step of seeding cultured cells onto the polymer fibers in (b).
Statement 30. The method of statement 25, wherein the polymer fibers are made of monomers comprising proteins, polysaccharides, or both.
Statement 31. A method of producing a food composition having a fat phase, comprising:
   (i) forming a plurality of polymer fibers using a solution blow spinning process, wherein said polymer fibers are formed from a composition comprising an edible ingredient dissolved in a solvent; and
   (ii) dispersing said plurality of blow spun polymer fibers in a fat phase.
Statement 32. The method of statement 31, wherein the polymer fibers comprise a lipophilic material selected from prolamins and lipophilic cellulose derivatives.
Statement 33. The method of statement 31, further comprising homogenizing a mixture of the plurality of blow spun polymer fibers and the fat phase.
Statement 34. The method of statement 33, further comprising fragmenting the mixture into particles.
Statement 35. The method of statement 31, wherein the fat phase comprises a vegetable oil, a dairy oil, a fish oil, an algae oil, or any combination thereof.
Statement 36. An edible food composition for human consumption, comprising: a scaffold comprising a plurality of polymeric fibers, wherein the edible composition has a textural characteristic comparable to a corresponding conventional food composition.
Statement 37. The composition of statement 36, wherein the polymeric fibers comprise at least one edible ingredient.
Statement 38. The composition of statement 37, wherein the edible ingredient comprises a texturizer, bulking agent or thickener, preservative, flavor enhancer, antimicrobial agent, pH modulator, desiccant, sweetener, curing or pickling agent, coloring agent, or any combination thereof.
Statement 39. The composition of statement 36, wherein the polymeric fibers are generated using a fiber spinning technique.
Statement 40. The composition of statement 39, wherein the fiber spinning technique is electrospinning or solution blow spinning.

### EXAMPLES

The following illustrative examples are representative of embodiments of the systems, methods, and compositions described herein and are not meant to be limiting in any way.

### Example 1 - cultured fish meat produced using embryonic stem cells

Embryonic stem cells are isolated from salmon embryos. The embryonic stem cells are first cultured using optimized media substrates and media formulations to achieve persistent cellular proliferation and maintenance of the de-differentiated state. The media formulations utilize synthetic serum-free media. The cells are cultured in a pathogen-free cell culture system. Next, the embryonic stem cells are induced to differentiate into myosatellite cells and pre-adipocytes. The myosatellite cells and pre-adipocytes are cultured and expanded to a desired quantity of cells. Next, the myosatellite cells and pre-adipocytes are differentiated into myocytes and adipocytes, which are then harvested and processed by centrifugation and compaction to generate the texture and consistency of fish meat.

### Example 2 - cultured fish meat produced using induced pluripotent stem cells

Fish fibroblasts are isolated from salmon. An episomal reprogramming strategy is employed to create induced pluripotent stem cells from the isolated fish fibroblasts without the use of classic viral reprogramming techniques. The induced pluripotent stem cells are cultured using optimized media substrates and media formulations to achieve persistent cellular proliferation and maintenance of the de-differentiated state. The media formulations utilize synthetic serum-free media. The cells are cultured in a pathogen-free cell culture system. Next, the iPS cells are expanded to a desired quantity of cells and then induced to differentiate into myocytes and adipocytes. Finally, the myocytes and adipocytes are harvested and processed by centrifugation and compaction to generate the texture and consistency of fish meat.

### Example 3 - cultured fish meat produced using direct cell reprogramming

Differentiated fish fibroblasts are isolated from salmon. The fibroblasts are serially passaged until a cell line is selected that has capacity for continuous self-renewal (e.g., immortalized). The immortalized fibroblasts are grown in culture to a desired quantity, and then transdifferentiated. A reprogramming strategy using the overexpression of select genes is employed to directly reprogram the fibroblasts into myocytes and adipocytes without creating an intermediate pluripotent cell type. Accordingly, transdifferentiation allows for the immortalized fibroblasts to be converted into the desired cell type without requiring the use of stem cells.

### Example 4 - micro-scaffolding system for culturing synthetic foods

Cells are cultured using any of the techniques described in examples 1-6 using a bioreactor containing micro-scaffolds that allow for the attachment and growth of the adherent hepatocytes to generate small cellular structures capable of being grown in suspension. The micro-scaffolds are composed of a biocompatible material that biodegrades over time such that the cultured hepatocytes structures eventually no longer have any scaffolding material remaining. The hepatocyte structures are subsequently processed to produce foie gras.

### Example 5 - 3D scaffolding system for culturing synthetic foods

Cells are cultured using any of the techniques described in examples 1-6 using 3D scaffolds that guide the growth of the adherent hepatocytes to generate cellular structures approximating the size and shape of conventional avian livers. The 3D scaffolds are composed of a biocompatible material such as alginate that biodegrades over time such that the finished foie gras product no longer has any scaffolding material remaining. As a result, the hepatocytes are grown to approximate the conventional avian liver without requiring centrifugation and processing to generate foie gras having the desired texture and consistency.

### Example 6 - foie gras produced using conventional techniques

Baby geese are raised and spend the first four weeks of their lives eating and growing. They are then transferred to cages and fed a high protein, high starch diet for another four weeks. At about eight to ten weeks, the birds are force fed by gavage, wherein two to four pounds of grain and fat are forced down the birds' throats using a feeding tube on a daily basis. The excess consumption of food causes the birds' livers to undergo steatosis in which the livers enlarge up to ten times or more than their normal size. During this process, the birds are exposed to various pathogens in the crowded and unsanitary conditions. Finally, the geese are slaughtered, and their livers harvested and sold as foie gras.

### Example 7 - sushi-grade salmon produced using embryonic stem cells

Embryonic stem cells are isolated from salmon embryos. The embryonic stem cells are first cultured using optimized media substrates and media formulations to achieve persistent cellular proliferation and maintenance of the de-differentiated state. The media formulations utilize synthetic serum-free media. The cells are cultured in a pathogen-free cell culture system without exposure to toxins or heavy metals such as mercury that is often found in fish meat. Next, separate populations of embryonic stem cells are induced to differentiate into myocytes and adipocytes, respectively. In this case, the myocytes are differentiated to produce a desired ratio of around 80% fast twitch and around 20% slow twitch muscle fibers. The myocytes and adipocytes are cultured and expanded to a desired quantity of cells. Afterwards, the myocytes and adipocytes are harvested and processed by centrifugation and compaction to generate the texture and consistency of conventional sushi-grade salmon, or alternatively, surimi-style salmon.

### Example 8 - sushi-grade salmon

Pre-adipocytes and satellite cells are isolated from salmon fingerlings, and subsequently characterized and cultivated in cell culture as separate cell lines. Each cell line is cultured using optimized media formulations to adapt the cell lines to suspension culture. Afterwards, adipocyte and myocyte differentiation are induced in the pre-adipocyte and satellite cell lines, respectively. Next, the cell lines are co-cultured at an optimized ratio to produce a desired final ratio of myocytes to adipocytes. The media formulations utilize synthetic serum-free media comprising mushroom-derived extracts that replace fetal bovine serum. The cells are cultured in a pathogen-free cell culture system without exposure to toxins or heavy metals such as mercury that is often found in fish meat. The culture media is supplemented with high concentrations of free fatty acids such as oleic acid, thereby inducing the adipocytes to take in and storing an excess amount of extracellular fatty acids. The co-cultured myocytes and adipocytes are then harvested and processed by centrifugation and compaction to generate the texture and consistency of salmon surimi.

### Example 9 - Isolation and cultivation of salmonid stem cells (pre-adipocyte, and myosatellite)

Fish myocytes and adipocytes were targeted for development of fish-related foods based on their intrinsic regenerative capacity during early developmental stages. The cultivation of relevant salmon tissues ex vivo, including myocytes, adipocytes, hepatocytes, fibroblasts, and undifferentiated multipotent cell lineages is characterized and optimized. First, trout pre-adipocytes and myosatellite cells (capable of differentiating into myocytes) are isolated, cultured, and characterized. Trout myosatellite cells were isolated and then characterized as shown in **FIGs. 5A-5D****.** Where present, insets magnify image details, and the scale bar is equal to 10 µm in all micrographs. Substantially pure populations of piscine myosatellite cells were successfully isolated and are shown in **FIG. 5A** with the myosatellite cells making up about 80% of the isolated cells. Next, these cells were characterized with relevant transcriptional markers. **FIG. 5B** shows RT-PCR results confirming the presence of hallmark genes (Mstn1a, Myf5) expressed in these isolated cells. Next, culture conditions were optimized for these cell lines. Culture media protocols were used to successfully differentiate myosatellite cells into mature myocytes (**FIG. 5C**). The sheets of myotubes differentiated from the myosatellite cells are shown in **FIG. 5D****.** Pre-adipocytes were also differentiated into adipocytes.

In addition, salmon myosatellite cells (arrowheads) were co-cultured with salmon pre-adipocytes (arrows) for producing a food product comprising both muscle and fat cells or tissue as shown in **FIG. 6A** (scale bar is 100 µm). The pre-adipocytes were differentiated into adipocytes, and the myosatellite cells differentiated into myocytes (arrowhead) as shown in **FIG. 6B** (scale bar is 10 µm).

### Example 10 - De novo creation of induced pluripotent stem cells using episomal reprogramming

Pluripotent stem cells were targeted for development of cultured foods based on their lack of commitment to a cell lineage and having great versatility with respect to inducible differentiation. For example, myocytes, adipocytes, fibroblasts and other tissue components can be created from a single pool of pluripotent stem cells.

Induced pluripotent stem cells (iPSCs) are generated using episomal (non-integrating) reprogramming. The iPSCs form colonies growing on a monolayer of mouse embryonic fibroblast (MEF) feeder cells. These cells are then characterized for pluripotency markers, proliferative capacity, and efficiency of differentiation. Furthermore, culture conditions are optimized for the generated cell line with regards to cost, large scale synthesis, and maintenance of genomic / phenotypic stability.

### Example 11 - Stem cell adaptation to suspension culture

Stem cell-based meat production represents an approach with multiple advantages over alternative cellular agriculture methodologies. First, the use of pluripotent stem cells is preferable to conventional myosatellite culture because the former possess indefinite replicative potential; this property obviates the need to repeatedly acquire muscle biopsies from animals during the process of production. Second, pluripotent stem cells are generally preferred over characterized immortal cell lines because they do not contain perturbations in cell cycle genetic networks or other related mutations. Finally, while the genetic and phenotypic characteristics of cell lines change in culture over time, the use of pluripotent stem cells with established differentiation protocols ensures the reproducibility of the final product. One challenge of stem cell-based meat production, however, is that stem cells generally are grown in 2-dimensional culture on a feeder cell line. Successfully adapting stem cells to 3-dimensional suspension culture for meat production would dramatically decrease the resource costs for cell culture.

Transitioning cells from 2-dimensional (e.g., cell culture dishes) to 3-dimensional suspension cultures is carried out and optimized to validate a method of scaling up cultured food production. Three dimensional suspension culture represents the first stage of scaling for food production because it offers the opportunity to grow significantly larger quantities of biological material with more efficient growth media utilization.

Growth of induced pluripotent stem cells (iPSCs) in suspension culture is facilitated by the creation of embryoid bodies (EBs), which are collections of stem cells that adhere to each other in lieu of an attachment surface on a plate. Like most mammalian cells, embryonic stem cells require signals from extracellular attachment points, and the process of acclimatizing them to the EB mode of growth often results in a high rate of cellular mortality and experimental variability. Protocols are refined to reliably acclimatize stem cells to growth in suspension culture, and to establish optimal culture conditions for growth and maintenance of pluripotency. One such method is the "hanging drop" technique, whereby cells are grown within a droplet of media, resulting in spontaneous formation of spheroids that are then acclimated to 3-dimensional culture conditions.

Cells are grown in media as "hanging drops" for 72 hours to form embryoid bodies. The embryoid bodies are then transferred to spinner flasks and grown in 3-dimensional suspension culture to allow scaling up of cell production.

Next, the proliferation kinetics under differing conditions of shear stress / laminar flow are assessed.

Finally, the induced pluripotent stem cells are differentiated in 3-dimensional cell culture.

### Example 12 - Immortalized cell line adaptation to suspension culture

An immortalized cell line derived from adult duck hepatocytes was generated by serial passaging the cultured hepatocytes and selecting colonies that proliferated at the highest rates. The immortalized cell line was then grown in media as "hanging drops" for 72 hours (**FIG**. 22A), after which the formation of spheroids became apparent. As shown in **FIG. 22A** and **22B****,** the spheroids were then transferred to spinner flasks and grown in 3-dimensional suspension culture to allow scaling up of cell production.

Next, the proliferation kinetics under differing conditions of shear stress / laminar flow are assessed.

Finally, the immortalized duck hepatocytes are differentiated in 3-dimensional cell culture.

### Example 13 - Cell adaption to cell suspension culture and differentiation using microscaffold technology

In the case of multipotent stem cells (e.g., myosatellite cells or pre-adipocytes), the transition to 3-dimensional suspension culture was facilitated by the development of microscaffolds (also known as microcarriers). These molecules offer foci of attachment, promote survival in the context of 3-dimensional shear forces, and stimulate both growth and differentiation.

Novel microscaffolds were developed to overcome a fundamental challenge of 3-dimensional cell culture of ensuring that nutrients and other protective factors are accessible to cells deep within a growing tissue when these cells do not directly contact the cell culture medium. The microscaffolds provided an added benefit of enhancing cellular proliferative capacity by engaging integrins and other extracellular-sensing transmembrane proteins found in the extracellular environment. Finally, these microscaffolds are engineered to contribute gustatory and structural properties that determine the product's final taste and texture.

Glucomannan (a water-soluble polysaccharide derived from konjac) was identified after an initial screen for inexpensive, abundant, neutral-tasting, and partially-soluble polysaccharides. Glucomannan has a relatively neutral taste profile, while the concentration used could be used to influence the tensile elasticity of the final product. Next, microscaffolds were generated using glucomannan and tested in various formulations to promote differentiated duck and piscine cell growth. **FIG. 23A** shows fish myosatellite cells grown on glucomannan microscaffolds (10% w/v). These myosatellite cells were assessed for their ability to differentiate into myocytes on the microscaffolds. Five days after isolation and plating, myosatellite cells were able to differentiate into myocytes and form 3-dimensional myotubes more readily than with standard 2-dimensional conditions (e.g., plastic culture dishes without glucomannan microscaffolds). Both 2-dimensional and 3-dimensional cultures demonstrated improved cell proliferation, with enhanced 3-dimensional myotube formation observed in the case of myosatellite cells. **FIG. 23B** shows a negative control of myosatellite cells from the same preparation grown in identical cell culture conditions prior to differentiation.

Next, glucomannan-based gels used for meat production are characterized for thermostability and tensile elasticity. **FIG. 24A** shows duck fibroblasts (arrowheads) grown on glucomannan microscaffolds (arrows). **FIG. 24B** shows a representative glucomannan microscaffold. Thus, duck fibroblasts can successfully attach to and grow on glucomannan microscaffolds, demonstrating the potential to generate larger 3-D structures such as, for example, a liver (e.g. following differentiation of the fibroblasts into hepatocytes).

### Example 14 - Cell culture media optimization

Cell culture media (e.g., differentiated cell culture media) was optimized for reduced serum concentrations that continue to support cell viability and proliferative capacity. **FIG. 17** shows the number of immortalized hepatocytes cultured in progressively decreasing concentrations of fetal bovine serum (FBS) in the presence of soybean hydrolysate (10g/L). The number of hepatocytes and the percentage of FBS are graphed over time with the hepatocytes continuing to increase while serum concentrations gradually dropped from 10% to 0.8% by 20 days. The media supplementation of soybean hydrolysate allowed the serum requirements of the cultured cells to be reduced by 92%.

The cell culture media is improved and optimized through sourcing of animal-free alternatives to serum to reduce and/or eliminate animal-derived components from production. Duck fibroblasts were successfully grown in 10% shiitake mushroom extract after successive reduction of fetal bovine serum from the cell culture media (**FIG. 18**). Duck fibroblasts grown in serum-free Essential 8 media (**FIG. 19A**) were compared to control cultures grown in DMEM supplemented with 10% fetal bovine serum (**FIG. 19B**).

### Example 15 - Salmonid meat product

The following components were used to create 1 gram of cell mass for a salmonid meat product prototype: 50ml fetal bovine serum (FBS), 500ml Dulbecco's Modified Eagle Media (DMEM), additional supplements such as pyruvate and non-essential amino acids, pipettes and culture dishes, and approximately 4 hours of labor. The successful transition to 3-dimensional culture reduced the labor time by half through elimination of the need for daily cell culture media changes and cell culture dishes, and reduction of pipette use, resulting in a relative decrease in resources needed to grow the cell mass. This corresponded to a roughly 50% reduction in price per pound. In addition, the transition to a plant-based media (e.g., soybean and/or cottonseed hydrolysate supplements) as described in example 17 further reduced the resource cost and price per pound by another 20%.

### Example 16 - Microscaffold (microcarrier) optimization

Microscaffolds are generated using various naturally-occurring substrates such as agar, alginate, and long-chain neutral charge polysaccharides derivatives for more extensive testing with respect to promotion of cell growth, maintenance of phenotype, and the preservation of flavor and fundamental culinary properties. For example, glucomannan and other sugars can either be dissolved or polymerized by a chemical reaction (e.g., heating and cooling at a defined pH). Other microscaffolds can be generated by either dissolved in solution or polymerized and then broken into small pieces. Microscaffolds are generally irregularly shaped, and serve as points of attachment for small numbers of cells (as opposed to macroscaffolds, which are porous and enable cells to grow within them).

In addition to serving as components of cellular adhesion, certain extracellular matrix proteins (e.g., laminin, vitronectin, and others) also inhibit the differentiation of stem cells. Accordingly, microscaffold structures are developed that include recombinant extracellular matrix proteins. Such microscaffold hybrids (polysaccharide + matrix protein) can serve the dual purpose of promoting cell attachment and proliferation, while simultaneously inhibiting spontaneous and premature differentiation. Preliminary studies indicate that these engineered microscaffolds offer significant benefits in terms of cellular proliferation and maintenance of genotypic stability. These microscaffolds can be generated using materials that allow bioresorbtion during cell propagation, obviating the need to extract these materials prior to harvest of the resultant food products.

The microscaffolds are also engineered to incorporate protein growth factors, proteoglycans, and lipids for enhancement of microscaffold function. These hybrid particles/microscaffolds can modulate intracellular signaling toward enhanced proliferation and extended maintenance of the desired cellular phenotype. Various expression systems are tested for optimal yield and cost-effectiveness.

Accordingly, hybrid microscaffolds composed of polysaccharide / protein growth factor / extracellular matrix protein are generated and characterized. In addition, encapsulated lipids are incorporated within 3-dimensional cell culture. Some of these microscaffolds use neutral charge, long-chain polysaccharides as described above with extracellular matrix components, protein growth factors, and encapsulated fatty acids. Specific metrics for these engineering optimizations include cellular proliferative capacity and rate, genomic stability (particularly as it applies to telomerase expression / cellular senescence), efficiency of differentiation, and morphologic consistency during culture.

Finally, the microscaffolds are optimized to refine taste and texture. Similar to conventional meat, much of the structural / textural properties of the final product can result from the composition and physical chemistry of extracellular components. Fibrous and connective tissue, adhesive proteins, and the underlying architectural arrangement of all these components can collectively determine properties such as elasticity, fracture characteristics, thermostability, and taste. The engineered tissue generated using the methods described herein can incorporate one or more of these features and components to yield meats that are structurally indistinguishable from their conventional counterparts. Moreover, chemical profiling of conventional and ex vivo meat by mass spectrometry is carried out to further refine the composition and taste of its products. Additional development is carried out according to the following:
i. Characterization of microscaffold hybrids consisting of polysaccharides and extracellular matrix proteins

Refine chemical synthesis methodology for polysaccharide / protein immobilization.

Characterize protein bioactivity and stability in 3-dimensional culture.

ii. Establish functional cellular assays pertaining to microscaffold growth, namely: proliferative capacity, proliferative rate, morphologic metrics, and efficiency of differentiation

Synthesize polysaccharide / protein growth factor / extracellular matrix protein hybrid microscaffolds.

Efficiently immobilize recombinant protein growth factors onto described neutral-charge polysaccharide backbones.

Quantify cellular responses to growth on these hybrid microscaffolds, including analysis of relevant intracellular signaling pathways, proliferative capacity and rate, cell morphology, and genetic profile (transcriptome analysis).

### iii. Incorporate encapsulated lipids within 3-dimensional cell culture

Efficient production of encapsulated lipid microparticles (lipid species can include: unsaturated, polyunsaturated, saturated, omega-3 fatty acids, etc.).

Incorporation into the described polysaccharide / protein hybrid microscaffolds, with quantification of resultant cellular response (functional assays including relevant intracellular signaling, proliferative capacity and rate, cell morphology, and transcriptome analysis).

### iv. Refine taste / texture

Refine final product preparation to develop a minimal viable product for piscine cell lines.

Optimize protocols for controlling lipid oxidation for piscine adipocytes.

Develop controlled taste tests to isolate the impact of each upstream product development decision on taste, texture, and appearance.

Optimize growth media formulation, scaffold choice, and lipid additions for each final product.

### Example 17 - Characterization and optimization of low-cost, animal component-free, culture media

Fetal bovine serum (FBS) is the largest initial contributor to production costs in cellular agriculture. However, FBS is poorly-defined with respect to its components, inconsistent from lot to lot, and relies upon animal sources for harvest. Accordingly, described herein are novel species- and cell-specific media formulations that are 1) free of animal serum, 2) completely defined, 3) cost-effective for large scale production, and 4) appropriate for food production.

### Media for stem cells

Serum-free formulations that promote stem cell proliferation and inhibit differentiation of stem cells are generated. Such defined media formulations can be optimized for fish stem cells and use animal-free components such as plant-derived supplements. These formulations are optimized with respect to various growth factors to both promote stem cell growth and inhibit spontaneous differentiation in culture.

One approach is the in-house reconstitution of defined media formulations. An example of this approach is the published formulation of Essential 8^{™} medium, for which the eight base components (including recombinant proteins) may be separately sourced to support large-scale stem cell growth. Of these eight components, the price of four (recombinant transferrin, TGF-beta, insulin, and FGF2) by far outweigh the others (basal media, ascorbic acid, selenium and sodium bicarbonate) because of the costs associated with recombinant protein expression and purification. An additional requisite component in existing media for stem cell cultures is an extracellular matrix protein (such as laminin) that must similarly be expressed and purified, adding to the cost of these culture systems.

Various approaches to the production of cultured meat products can include recombinant expression of components, with in-house media reconstitution (detailed below) and development of a conditioned media system (detailed below).

### Recombinant protein expression

Recombinant proteins are expressed, purified, and then incorporated into media formulations which are assessed for ability to support and promote cell growth and proliferation. Expression systems include algae, bacteria, yeast, insect, and mammalian cell cultures. Although the expression and purification of individual proteins carries an initially high cost, this process permits a higher level of precision for the titration of protein concentrations in cell culture. Such precision aids in the process of defining requisite cell culture components. A peptide and protein screen is developed to aid in the optimization of cell culture components in each established cell line.

### Conditioned media

The ability of cell lines to secrete growth factors that promote the viability and proliferation of other cells is the central principle underlying conditioned media or co-culture systems. Certain cell lines are evaluated for their ability to condition media with particular growth factors necessary for large-scale production of meat. In particular, cell lines that overexpress factors such as hepatocyte growth factor (HGF), fibroblast growth factor-2 (FGF2), and leukemia inhibitory factor (LIF) can improve cost-efficiency over expression and purification of the recombinant forms of these proteins.

Various development paths are pursued in parallel towards the creation of a cultured meat product. In the case of minced salmon, precursor cells (myosatellite cells and pre-adipocytes) are terminally differentiated into myocytes and adipocytes both together and separately, in order to establish an optimal method for salmon meat production. In addition, studies are carried out assessing the role of fibroblasts in promoting stable cell culture and in defining the textural qualities of the finished product.

### Characterization and optimization of low-cost, animal component-free, culture media formulations

The various approaches can be refined and optimized to increase production efficiency and decrease costs.

Plant-based media optimization. FBS is reduced or eliminated from cell culture growth media by gradually transitioning cell lines to plant-based culture media supplemented with a plant-derived extract such as soybean hydrolysate or mushroom extract (see **FIGs. 17-19**). In addition, studies are carried out identifying lower cost, plant-based alternatives for supplements such as pyruvate and non-essential amino acids either through custom formulations or off-the-shelf products available through industrial-scale suppliers.

Proprietary low-cost media formulation. Further refine the plant-based media formulation, reducing pre-mixed DMEM requirements by moving to lower cost formulations.

Proprietary low-cost media is further optimized. Refine the plant-based media formulation by moving from higher-cost sources of animal-free recombinant proteins (e.g., albumin, transferrin, insulin) to industrial quantities and pricing for plant-derived protein components. In addition, continue improving the workflow and process automation, reducing labor requirements.

Final optimization to media components. Move from small scale spinning flasks and mechanical rocker systems to industrial grade bioreactors permitting reuse of media, larger scale tissue culture, and significant reductions in labor costs.

Approaches to the development of low-cost stem-cell growth media include:

Create conditioned media system for the expression of growth factors that support both stem cell and differentiated cell proliferation.

Optimize recombinant protein expression for cell culture components.

Conduct peptide / protein screen to improve cell proliferation rate, maintain de-differentiated state of stem cells, and increase efficiency of terminal differentiation.

Continue to improve workflow and process automation to reduce the labor required.

### Example 18 - Transition to medium-scale production using bioreactors

Bioreactors are adapted for the efficient growth of 3-dimensional cultures. This process is achieved by satisfying 3 sub-objectives: i) refining small-scale bioreactors for efficient tissue growth, ii) procuring and adapting medium-scale bioreactors to supply a minimum viable product to two restaurants, and iii) developing large-scale bioreactors under an economic model for large-scale meat production.

### i. Refine current small-scale bioreactors

Efficient bioreactor design can minimize waste in culture media (with the recycling of components such as buffers) and provide the seamless transition from one culture media to another (e.g. stem cell growth media versus myocyte differentiation media). Preliminary studies entail optimizing laminar and shear flow in small-scale 3-dimensional cultures to optimize long-term cellular spheroid growth. These initial studies assess growth rates among different differentiated piscine cell lines (with or without microscaffolds).

### ii. Procure and adapt medium-scale bioreactors

Approximately 100 liters of bioreactor volume is used to produce about two pounds of cell mass per week. This volume is supplied through five 20L rocker bioreactors, which can supply the desired cell mass at a steady quantity per week based on a 4-6 week cell growth time. Alternatively, this volume is supplied through twenty 5L bioreactors. In some cases, the bioreactors (or other containers used for cell culturing, growth, and/or differentiation) have a volume no larger than 5 liters.

Commercially-available bioreactors are configured for pharmaceutical applications and require the use of costly disposable bags for each tissue harvest. These bioreactors are down-scaled to remove unnecessary features to enable increased longer-term resource efficiency in cultured tissue production.

### iii. Develop model for large-scale meat production

Large-scale bioreactors (e.g., disposable bag and stainless steel designs) are assessed for suitability for large scale ex vivo meat production.

The transition to medium-scale production using bioreactors can be accomplished according to the methods described below.

### a. Refine current small-scale bioreactors

Evaluate optimal media change frequency. Assess opportunity for media reuse / recycling. Identify optimal density at the time of harvest. Identify methods for tracking cell growth (spheroids versus individual cells).

### b. Procure and adapt medium-scale bioreactors

Determine optimal media volume per kilogram produced (validate 1:50 ratio assumption). Optimize media changing regimen for each cell line. Evaluate optimal fluid dynamics (shear stress, impeller type, speed) for various cell cultures and temperatures. Integrate a conditioned media model of cell growth within the bioreactor system.

### Example 19 - Cultured food product optimization and refinement

Further work is performed to refine product texture, taste, and nutritional composition. Increases to production efficiency are obtained by continued optimization of growth media formulations, engineering media recycling fluidics, minimizing sterile production waste (e.g., disposable plastics), and incorporating automation in the production pipeline.

### Example 20 - cultured foie gras produced using embryonic stem cells

Embryonic stem cells are isolated from Eyal-Giladi and Kochav Stage 10 (EGK-X) avian embryos. The embryonic stem cells are first cultured using optimized media substrates and media formulations to achieve persistent cellular proliferation and maintenance of the de-differentiated state. The media formulations utilize synthetic serum-free media. The cells are cultured in a pathogen-free cell culture system. Next, the embryonic stem cells are induced to differentiate into hepatocytes. The hepatocytes are cultured and expanded to a desired quantity of cells. The culture media is supplemented with high concentrations of free fatty acids such as oleic acid, thereby inducing the hepatocytes to undergo steatosis by taking in and storing an excess amount of extracellular fatty acids. The steatotic hepatocytes are then harvested and processed by centrifugation and compaction to generate the texture and consistency of conventional foie gras. This foie gras product lacks texture of skeletal muscle meats. Moreover, the foie gras is substantially composed of only hepatocytes unlike skeletal muscle meats that include myocytes, endothelial cells, and adipose cells. The finished foie gras is firm, light-colored, and lacks any of the veins or blemishes that are often found in conventional foie gras. Accordingly, the foie gras is packaged for sale with a label indicating grade A quality and that it was made without force feeding.

### Example 21 - cultured foie gras produced using induced pluripotent stem cells

Avian dermal fibroblasts are isolated from a goose. An episomal reprogramming strategy is employed to create induced pluripotent stem cells from the isolated dermal fibroblasts without the use of classic viral reprogramming techniques. The induced pluripotent stem cells are cultured using optimized media substrates and media formulations to achieve persistent cellular proliferation and maintenance of the de-differentiated state. The media formulations utilize synthetic serum-free media. The cells are cultured in a pathogen-free cell culture system. Next, the iPS cells are induced to differentiate into hepatocytes and expanded to a desired quantity of cells. The culture media is supplemented with high concentrations of free fatty acids such as oleic acid, thereby inducing the hepatocytes to undergo steatosis by taking in and storing an excess amount of extracellular fatty acids. The steatotic hepatocytes are then harvested and processed by centrifugation and compaction to generate the texture and consistency of conventional foie gras.

### Example 22 - cultured foie gras produced using direct cell reprogramming

Avian dermal fibroblasts are isolated from a duck. A reprogramming strategy using the overexpression of transcription factors is employed to directly reprogram the dermal fibroblasts into hepatocytes without creating an intermediate pluripotent cell type. The hepatocytes are expanded to a desired quantity of cells and grown in culture media supplemented with high concentrations of free fatty acids such as oleic acid, which cause the hepatocytes to undergo steatosis by taking in and storing an excess amount of extracellular fatty acids. The steatotic hepatocytes are then harvested and processed by centrifugation and compaction to generate the texture and consistency of conventional foie gras.

### Example 23 - cultured foie gras produced using immortalized mature hepatocytes

Mature avian hepatocytes are isolated from duck liver. The hepatocytes are immortalized using classical techniques such as transformation with SV40 Large T Antigen or spontaneous hepatocyte immortalization by sequentially passaging the hepatocytes until spontaneous mutations arise that result in immortalization. The immortalized hepatocytes are expanded to a desired quantity of cells and grown in culture media supplemented with high concentrations of free fatty acids such as oleic acid, which cause the hepatocytes to undergo steatosis by taking in and storing an excess amount of extracellular fatty acids. The steatotic hepatocytes are then harvested and processed by centrifugation and compaction to generate the texture and consistency of conventional foie gras.

### Example 24 - cultured foie gras produced using nascent hepatic stem cells

Hepatic stem or progenitor cells are isolated from duck liver. The hepatic stem cells are cultured using optimized media substrates and media formulations to achieve persistent cellular proliferation and maintenance of the pluripotent state. The media formulations utilize synthetic serum-free media. The cells are cultured in a pathogen-free cell culture system. Next, the hepatic stem cells are induced to differentiate into mature hepatocytes and expanded to a desired quantity of cells. The culture media is supplemented with high concentrations of free fatty acids such as oleic acid, thereby inducing the hepatocytes to undergo steatosis by taking in and storing an excess amount of extracellular fatty acids. The steatotic hepatocytes are then harvested and processed by centrifugation and compaction to generate the texture and consistency of conventional foie gras.

### Example 25 - genetic modulation of cultured hepatocytes to induce steatosis for producing foie gras

Hepatocytes are obtained using any of the procedures described in examples 1-5, except the step of culturing the hepatocytes in a lipid enriched culture medium has been replaced with a step that genetically manipulates the metabolic pathway(s) responsible for lipid metabolism. In this case, the genetic manipulation results in the accumulation of lipid droplets within the cytoplasm of the hepatocytes, thereby resulting in steatosis.

### Example 26 - producing an edible composition using solution blow spinning

A solution of gelatin, collagen, and cellulose is prepared in an ethanol solvent. The solution is pumped through the central nozzle of an airbrush containing concentric nozzles with the outermost nozzle providing a high velocity air stream. When the monomers hit the high pressure stream of gas or air, the low pressure and shear from the inner nozzle causes the solvent to quickly evaporate, and entangled polymer fibers formed from the solutes from the solution (gelatin, collagen, cellulose) are collected as nano-microfibers on a spinning cylinder. The cylinder's rotation collects the fibers in alignment to create a first layer of fibers having a geometry similar to muscle fibrils. A nozzle then deposits a second layer of cultured fish myocytes on the surface of the first layer of fibers. A second airbrush then generates a third layer of fibers formed from lipophilic cellulose derivatives and fish oil. A second nozzle then deposits a fourth layer of cultured fish adipocytes on the surface of the third layer of fibers. These layers are collected by the rotating cylinder. The collected layers are then packaged and refrigerated for storage and later distributed for human consumption.

The core technologies described herein is applied to the production of various processed foods and meats such as trout, tuna, other seafood products, and avian meats based on similar goals of environmental, ethical, and nutritional benefits.

### Example 27 - producing an edible meat product using solution blow spinning

A solution of gelatin, collagen, and cellulose is prepared in an ethanol solvent. The solution is pumped through the central nozzle of an airbrush containing concentric nozzles with the outermost nozzle providing a high velocity air stream. When the monomers hit the high pressure stream of gas or air, the low pressure and shear from the inner nozzle causes the solvent to quickly evaporate, and entangled polymer fibers formed from the solutes from the solution (gelatin, collagen, cellulose) are collected as nano-microfibers on a spinning cylinder. The cylinder's rotation collects the fibers in parallel alignment to create a first layer of fibers having an aligned fibrous geometry similar to muscle fibrils. A nozzle then deposits a second layer of hydrogel comprising fats and oils approximating the fat content of salmon meat. A crosslinking agent is used to strengthen the connection between the fibers. These layers are collected by the rotating cylinder. The collected layers are then packaged and refrigerated for storage and later distributed for human consumption.

### Example 28 - producing an edible composition using solution blow spinning and cell culturing

A solution of gelatin, collagen, and cellulose is prepared in an ethanol solvent. The solution is pumped through the central nozzle of an airbrush containing concentric nozzles with the outermost nozzle providing a high velocity air stream. When the monomers hit the high pressure stream of gas or air, the low pressure and shear from the inner nozzle causes the solvent to quickly evaporate, and entangled polymer fibers formed from the solutes from the solution (gelatin, collagen, cellulose) are collected as nano-microfibers on a spinning cylinder. The cylinder's rotation collects the fibers in alignment to create a first layer of fibers having a geometry similar to muscle fibrils. The fibers are supplemented with growth factors and differentiation factors designed to induce growth and differentiation of undifferentiated or partially differentiated cell population into muscle cells. A nozzle then deposits a second layer of undifferentiated fish pre-myocytes on the surface of the first layer of fibers. A second airbrush then generates a third layer of fibers formed from lipophilic cellulose derivatives and fish oil. These fibers are supplement with growth factors and differentiation factors for inducing growth and differentiation into adipocytes. A second nozzle then deposits a fourth layer of undifferentiated fish pre-adipocytes on the surface of the third layer of fibers. These layers are collected by the rotating cylinder. The collected layers are then cultured to allow the cells to grow and differentiate. Finally, the finished synthetic meat composition is packaged and refrigerated for storage and later distributed for human consumption.

## Claims

1. An edible composition for human consumption, comprising:
a) a population of cells, wherein the population of cells comprises a cultured population of cells; and
b) a plurality of polymeric fibers or scaffolds,
wherein the polymeric fibers or scaffolds and the population of cells are arranged as alternating layers of fibers and cells.

2. The composition of claim 1, wherein the polymeric fibers or scaffolds comprise at least one edible ingredient.

3. The composition of claim 2, wherein the edible ingredient comprises a texturizer, bulking agent or thickener, preservative, flavor enhancer, antimicrobial agent, pH modulator, desiccant, sweetener, curing or pickling agent, coloring agent, or any combination thereof.

4. The composition of claim 2, wherein the edible ingredient comprises a protein, a carbohydrate, a fat, or any combination thereof.

5. The composition of claim 1, wherein the polymeric fiber or scaffold comprises a supplement.

6. The composition of claim 5, wherein the supplement comprises a vitamin, a mineral, or both.

7. The composition of claim 1, wherein the population of cells comprises myocytes.

8. The composition of claim 7, wherein the plurality of fibers comprises a layer of fibers incorporating the myocytes and aligned to mimic muscle fibers.

9. The composition of claim 1, wherein the population of cells further comprises adipocytes.

10. The composition of claim 9, wherein the plurality of fibers comprises a layer of fibers incorporating the adipocytes and configured to mimic adipose banding patterns.

11. The composition of claim 1, wherein the polymeric fibers or scaffolds are generated using a fiber spinning technique.

12. The composition of claim 11, wherein the fiber spinning technique is electrospinning, wet spinning, or solution blow spinning.

13. The composition of claim 1, wherein the composition comprises polymeric fibers generated using a fiber spinning technique and scaffolds generated using unidirectional freeze-drying, electro-spinning, solution blow spinning, wet spinning, casting, micromolding, 3D printing or extrusion, or any combination thereof.
